# EUROPEAN PATENT APPLICATION

(11) **EP 4 692 360 A1**
(43) Date of publication of application: **11.02.2026**
(21) Application number: 24778296.4
(22) Date of filing: 30.03.2024
(51) Int. Cl.: C12N 15/864, C07K 14/47, A61P 25/00

(54) **EXPRESSION VECTOR AND COMPOSITION FOR TREATING NERVOUS SYSTEM DISEASES**

(30) Priority: 30.03.2023 WO PCT/CN2023/085228; 12.01.2024 WO PCT/CN2024/072004
(71) Applicant: Shanghai Genemagic Biosciences Co., Ltd., Shanghai 201108 (CN)
(72) Inventor: ZHOU, Haibo, Shanghai 201108 (CN); HU, Xinde, Shanghai 201108 (CN); SU, Jinlin, Shanghai 201108 (CN)
(74) Representative: Lavoix
(86) International application number: PCT/CN2024/085103
(87) International publication number: WO 2024/199511

(57) **Abstract**

Provided is an expression vector, comprising: (a) a nucleic acid sequence encoding an artificial transcription factor capable of binding to RE1, the artificial transcription factor comprising an RZFD core domain and (b) a regulatory element for regulating the nucleic acid expression of the artificial transcription factor, wherein the amino acid sequence of the RZFD core domain contains any 5-8 sequences among SEQ ID NOs: 3-10 and does not contain sequences shown in SEQ ID NOs: 12 and 13; the amino acid sequence of the RZFD core domain contains any 6-8 sequences among SEQ ID NOs: 3-10.

## Description

### TECHNICAL FIELD

The present application relates to the field of molecular biology and therapeutic medicament for nervous system diseases, and particularly relates to the expression vectors and compositions comprising nucleic acid sequences encoding an artificial transcription factor capable of binding to RE1.

### CROSS-REFERENCE

This application claims the benefits of patent application No. PCT/CN2023/085228 filed on March 30, 2023, and patent application No. PCT/CN2024/072004 filed on January 12, 2024, the contents of which are incorporated herein by reference in their entirety.

### SEQUENCE LISTING SUBMITTED CONCURRENTLY

The entire contents of the following XML file are incorporated herein by reference in their entirety: Sequence Listing in Computer Readable Format (CRF) (File Name: TFH00974PCT-Sequence listing, Date: 20240329, Size: 131KB).

### BACKGROUND

The Repressor Element 1/Neuron-Restrictive Silencer Element (RE1/NRSE) is a negative regulatory element that binds to the RE1 Silencing Transcription Factor (REST), and can influence neuronal development and maturation.

In previous studies, scientists have successfully induced various specific types of neurons in in vitro culture systems by adding different culture media and small molecules, combined with the expression of multiple genes and transcription factors. However, these in vitro experiments are difficult to apply in vivo. The complex in vivo environment causes many factors screened in vitro can't function effectively in vivo as they do in in vitro experiments. Therefore, many factors screened in vitro for inducing neuronal transdifferentiation can't induce glial cells to transdifferentiate into neurons in vivo. How to achieve the regeneration of specific types of neurons in vivo is an extremely challenging problem.

### SUMMARY OF THE INVENTION

The present invention provides an expression vector. The present invention also provides a composition. The expression vector and composition provided by the present invention can promote the transdifferentiation of non-neuronal cells into functional neurons.

In a first aspect of the present invention, the provided expression vector comprises: (a) a nucleic acid sequence encoding an artificial transcription factor capable of binding to RE1, said artificial transcription factor comprises RZFD core domain; (b) a regulatory element regulating the expression of the nucleic acid of said artificial transcription factor; wherein the amino acid sequence of said RZFD core domain comprises any 5-8 sequences of SEQ ID NOs: 3-10, or is any 5-8 sequences of SEQ ID NOs: 3-10, and does not comprise the sequence shown in SEQ ID NO: 12 and 13. Preferably, the amino acid sequence of said RZFD core domain comprises any 6-8 sequences of SEQ ID NOs: 3-10, or is any 6-8 sequences of SEQ ID NOs: 3-10.

In some embodiments, the amino acid sequence of said RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 76; preferably, the amino acid sequence of said RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 74 or 75; more preferably, the amino acid sequence of said RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 2.

In some embodiments, the amino acid sequence of said RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 14.

In some embodiments, the nucleic acid sequence encoding said RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 15 or 64.

In some embodiments, said regulatory element comprises a promoter regulating the expression of the nucleic acid of the artificial transcription factor; preferably, said promoter comprises a glial cell-specific promoter.

Further, said glial cell-specific promoter is an astrocyte-specific promoter, or a Müller cell-specific promoter, or a cochlear glial cell-specific promoter. Further, said glial cell-specific promoter comprises GFAP promoter, ALDH1L1 promoter, EAAT1/GLAST promoter, glutamine synthetase promoter, S100β promoter, EAAT2/GLT-1 promoter, Plp1 promoter, or Rlbpl promoter.

Further, said cochlear glial cell-specific promoter comprises: GFAP promoter, ALDH1L1 promoter, EAAT1/GLAST promoter, or Plp1 promoter.

Further, said astrocyte-specific promoter or Müller cell-specific promoter comprises GFAP promoter, ALDH1L1 promoter, EAAT1/GLAST promoter, glutamine synthetase promoter, S100β promoter, EAAT2/GLT-1 promoter, Plp1 promoter, or Rlbp1 promoter.

In some embodiments, said regulatory element further comprises an enhancer that increases transcription of said artificial transcription factor; preferably, the enhancer of said expression vector comprises CMV enhancer, SV40 intronic enhancer, MVM intronic enhancer, β-globin intronic enhancer, or a synthetic intronic enhancer, or any combination of these enhancers (e.g., a combination of CMV enhancer and MVM enhancer). In some embodiments, the nucleic acid sequence of the CMV enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 16. In some embodiments, the nucleic acid sequence of the SV40 intron enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 17. In some embodiments, the nucleic acid sequence of the MVM intron enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 18. In some embodiments, the nucleic acid sequence of the β-globin intron enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 19. In some embodiments, the nucleic acid sequence of the synthetic intron enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any sequence shown in SEQ ID NOs: 20-22.

In some specific embodiments, the regulatory element may comprise one enhancer or multiple enhancers; and the multiple enhancers may be the same or different.

In some embodiments, the regulatory element further comprises a Woodchuck Hepatitis Virus Posttranscriptional Regulatory Element (WPRE). For example, the regulatory element comprises a promoter regulating the nucleic acid expression of the artificial transcription factor and a WPRE. Or the regulatory element comprises a promoter regulating the nucleic acid expression of the artificial transcription factor, a WPRE, and an enhancer that increases transcription of the artificial transcription factor.

In some embodiments, the artificial transcription factor further comprises at least one nuclear localization sequence (NLS). Preferably, said NLS is selected from the NLS of SV40, the NLS of nucleoplasmin, the NLS of c-myc, the NLS of hRNPA1M9, the NLS of Importin α, the NLS of p53, the NLS of influenza virus NS1, the NLS of hepatitis delta antigen, or the bpNLS.

In some embodiments, the NLS is located at the N-terminus or C-terminus of the RZFD core domain.

In some embodiments, the NLS is located at both the N-terminus and C-terminus of the RZFD core domain.

In some embodiments, the amino acid sequence of the NLS comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 24. In some embodiments, the nucleic acid sequence encoding the NLS comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 25.

In some embodiments, the artificial transcription factor further comprises a gene activation domain; preferably, said gene activation domain is located at the C-terminus and/or N-terminus of said RZFD core domain.

In some embodiments, the gene activation domain comprises the activation domain of VP64, P65, HSF1, VP16, RTA, Suntag, P300, or CBP, or any combination of these.

In some embodiments, the gene activation domain comprises the activation domain of VP64, the activation domain of P65, the activation domain of RTA, or the activation domain of HSF1, or any combination thereof.

In some embodiments, the activation domain is located at the C-terminus or N-terminus of the RZFD core domain. Further, the activation domain of VP64 is located at both the C-terminus and N-terminus of the RZFD core domain.

In some embodiments, the amino acid sequence of the VP64 activation domain comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 26. In some embodiments, the nucleic acid sequence encoding the VP64 activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 27. In some embodiments, the amino acid sequence of the P65 activation domain comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 69. In some embodiments, the amino acid sequence of the RTA activation domain comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 11. In some embodiments, the amino acid sequence of the HSF1 activation domain comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to the sequence shown in SEQ ID NO: 70.

In some embodiments, the gene activation domain comprises the activation domain of VP64 and the activation domain of P65; preferably, the gene activation domain further comprises the activation domain of HSF1 or the activation domain of RTA.

In some embodiments, the activation domain is located at the N-terminus or C-terminus of the RZFD core domain. Further, the activation domain is located at both the N-terminus and C-terminus of the RZFD core domain.

In some embodiments, the gene activation domain comprises the activation domains of P65 and HSF1. In some embodiments, the amino acid sequence of the activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:28. In some embodiments, the activation domain is encoded by a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:29.

In some embodiments, the gene activation domain comprises the activation domain of VP64, the activation domain of P65, and the activation domain of HSF1. Preferably, the sequence of the activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:73.

In some embodiments, the gene activation domain comprises the activation domain of VP64, the activation domain of P65, and the activation domain of RTA. Preferably, the sequence of the activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:72.

In some embodiments, the sequence of the gene activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:28, 69, 70, or 71.

In some embodiments, the artificial transcription factor further comprises nuclear localization sequence (NLS) and gene activation domain; preferably, the NLS and/or gene activation domain is located at the N-terminus and/or C-terminus of the RZFD core domain; more preferably, the artificial transcription factor is connected from the N-terminus to the C-terminus in one of the following types: RZFD core domain - NLS - gene activation domain; RZFD core domain - gene activation domain - NLS; NLS - gene activation domain - RZFD core domain; gene activation domain - NLS - RZFD core domain; NLS - RZFD core domain - gene activation domain; gene activation domain - RZFD core domain - NLS; gene activation domain - NLS - RZFD core domain - NLS - gene activation domain; or NLS - gene activation domain - RZFD core domain - gene activation domain - NLS.

In some embodiments, the gene activation domain comprises the activation domain of VP64, P65, HSF1, VP16, RTA, Suntag, P300, or CBP, or any combination thereof. Preferably, the gene activation domain comprises the activation domain of VP64, the activation domain of P65, the activation domain of RTA, or the activation domain of HSF1, or any combination thereof.

In some embodiments, the activation domain of VP64 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:26; the activation domain of P65 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:69; the activation domain of RTA comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:11; the activation domain of HSF1 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:70.

In some embodiments, the amino acid sequence of the NLS comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:24.

In some embodiments, the RZFD core domain is connected to the NLS via a linker; or the RZFD core domain is connected to the gene activation domain via a linker; or the NLS is connected to the gene activation domain via a linker.

In some embodiments, the artificial transcription factor comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs: 2, 14, 30, 32, 34, 57, 59, 61, 63, 67, 77, 78, 79, 80, 81, 82, 84, 86, 90, or 92.

In some embodiments, the nucleic acid sequence encoding the artificial transcription factor comprises or is a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs: 15, 31, 33, 35, 56, 58, 60, 64, 68, 85, 87, 91, or 93.

In some embodiments, the expression vector comprises or is a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs: 36, 37, or 62.

In a second aspect of the present invention, a composition is provided, comprising: (a) an artificial transcription factor capable of binding to RE1, which comprises RZFD core domain, a first NLS, and a first gene activation domain; or (b) a nucleic acid encoding the artificial transcription factor of (a); wherein the RZFD core domain comprises or is any 5-8 sequences of SEQ ID NOs: 3-10, and does not comprise the sequences shown in SEQ ID NO: 12 and 13. Preferably, the amino acid sequence of the RZFD core domain comprises or is any 6-8 sequences of SEQ ID NOs: 3-10.

In some embodiments, the amino acid sequence of the RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 76; preferably, the amino acid sequence of the RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 74 or 75; more preferably, the amino acid sequence of the RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 2.

In some embodiments, the amino acid sequence of the RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 14.

In some embodiments, the nucleic acid sequence encoding the RZFD core domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 15 or 64.

In some embodiments, from the N segment to the C terminus, the connection type of the RZFD core domain, the first NLS, and the first gene activation domain is selected from:
1) RZFD core domain -- first NLS -- first gene activation domain;
2) first gene activation domain -- first NLS -- RZFD core domain;
3) RZFD core domain -- first gene activation domain -- first NLS;
4) first NLS -- first gene activation domain -- RZFD core domain;
5) first NLS -- RZFD core domain -- first gene activation domain;
6) first gene activation domain -- RZFD core domain -- first NLS.

In some embodiments, the RZFD core domain, the first NLS, and the first gene activation domain may be connected via linkers or without linkers.

In some embodiments, the artificial transcription factor further comprises a second NLS. In specific embodiments, the first NLS and the second NLS may be the same or different.

In some embodiments, the first NLS is located at the N-terminus of the RZFD core domain, and the second NLS is located at the C-terminus of the RZFD core domain.

In some embodiments, the first gene activation domain is located at the N-terminus of the first NLS.

In some embodiments, the artificial transcription factor further comprises a second gene activation domain. In specific embodiments, the first NLS and the second NLS may be the same or different.

In some embodiments, the second gene activation domain and the first gene activation domain are located at opposite ends of the RZFD core domain.

In some embodiments, the second gene activation domain is located at the C-terminus of the second NLS.

In some embodiments, the first NLS comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 24. In some embodiments, the nucleic acid sequence encoding the first NLS comprises or is a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 25.

In some embodiments, the second NLS comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:24. In some embodiments, the nucleic acid sequence encoding the second NLS comprises or is a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 25.

In some embodiments, the first gene activation domain comprises the activation domain of VP64, P65, HSF1, VP16, RTA, Suntag, P300, or CBP, or any combination of these; preferably, the first gene activation domain comprises the activation domain of VP64, the activation domain of P65, the activation domain of RTA, or the activation domain of HSF1, or any combination thereof. In some embodiments, the activation domain of VP64 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO.: 26; the activation domain of P65 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO.: 69; the activation domain of RTA comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO.: 11; the activation domain of HSF1 comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO.: 70. In some embodiments, the nucleic acid sequence encoding the activation domain of VP64 has at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO.: 27.

In some embodiments, the first gene activation domain comprises the activation domain of VP64 and the activation domain of P65; preferably, the first gene activation domain further comprises the activation domain of HSF1 or the activation domain of RTA.

In some embodiments, the first gene activation domain comprises the activation domain of VP64, the activation domain of P65, and the activation domain of HSF1; preferably, the amino acid sequence of the first activation domain comprises or is the amino acid sequence having at least 70%, at least 80%, at least 85%, at least 90%, at least 91%, at least 92%, at least 93%, at least 94%, at least 95%, at least 96%, at least 97%, at least 98%, or at least 99% or 100% identical to SEQ ID NO:73.

In some embodiments, the first gene activation domain comprises the activation domain of VP64, the activation domain of P65, and the activation domain of RTA; preferably, the amino acid sequence of the first activation domain comprises or is the amino acid sequence having at least 70%, at least 80%, at least 85%, at least 90%, at least 91%, at least 92%, at least 93%, at least 94%, at least 95%, at least 96%, at least 97%, at least 98%, or at least 99% or 100% identical to SEQ ID NO:72.

In some embodiments, the amino acid sequence of the first gene activation domain comprises or is the amino acid sequence having at least 70%, at least 80%, at least 85%, at least 90%, at least 91%, at least 92%, at least 93%, at least 94%, at least 95%, at least 96%, at least 97%, at least 98%, or at least 99% or 100% identical to SEQ ID NO: 28, 69, 70, or 71.

In some embodiments, the nucleic acid sequence encoding the first gene activation domain comprises or is a nucleotide sequence having at least 70%, at least 80%, at least 85%, at least 90%, at least 91%, at least 92%, at least 93%, at least 94%, at least 95%, at least 96%, at least 97%, at least 98%, or at least 99% or 100% identical to SEQ ID NO: 29.

In some embodiments, the second gene activation domain comprises the activation domains of VP64, P65, HSF1, VP16, RTA, Suntag, P300, or CBP, or any combination thereof; preferably, the second gene activation domain comprises the activation domain of VP64; more preferably, the second gene activation domain comprises the activation domain of VP64 and the activation domain of P65; even more preferably, the second gene activation domain further comprises the activation domain of HSF1 or the activation domain of RTA. In some embodiments, the amino acid sequence of the activation domain of VP64 comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:26. In some embodiments, the nucleic acid sequence encoding the activation domain of VP64 comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:27. In some embodiments, the amino acid sequence of the activation domain of P65 comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:69; in some embodiments, the amino acid sequence of the activation domain of RTA comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 11; in some embodiments, the amino acid sequence of the activation domain of HSF1 comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:70.

In some embodiments, the second gene activation domain comprises the activation domain of VP64 and the activation domain of P65; preferably, the second gene activation domain further comprises the activation domain of HSF1 or the activation domain of RTA.

In some embodiments, the second gene activation domain comprises the activation domain of VP64, the activation domain of P65, and the activation domain of HSF1; preferably, the sequence of the second activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:73.

In some embodiments, the second gene activation domain comprises the activation domain of VP64, the activation domain of P65, and the activation domain of RTA; preferably, the sequence of the second activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:72.

In some embodiments, the sequence of the second gene activation domain comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:28, 69, 70, or 71.

In some embodiments, the artificial transcription factor of the composition comprises or is an amino acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs: 2, 14, 30, 32, 34, 57, 59, 61, 63, 67, 77, 78, 79, 80, 81, 82, 84, 86, 90, or 92. In some embodiments, the nucleic acid sequence encoding the artificial transcription factor comprises or is a nucleotide sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs: 15, 31, 33, 35, 56, 58, 60, 64, 68, 85, 87, 91, or 93.

In some embodiments, the composition further comprises a regulatory element for regulating the nucleic acid expression of the artificial transcription factor; preferably, the regulatory element includes a promoter to regulate the nucleic acid expression of the artificial transcription factor; more preferably, the promoter comprises a glial cell-specific promoter. In some embodiments, the glial cell-specific promoter includes astrocyte-specific promoter, Müller cell-specific promoter, or cochlear glial cell-specific promoter; preferably, the glial cell-specific promoter includes GFAP promoter, ALDH1L1 promoter, EAAT1/GLAST promoter, glutamine synthetase promoter, S100β promoter, EAAT2/GLT-1 promoter, Plp1 promoter, or Rlbp1 promoter.

In some embodiments, the regulatory element further comprises an enhancer that increases transcription of the artificial transcription factor; preferably, the enhancer comprises CMV enhancer, SV40 intronic enhancer, MVM intronic enhancer, β-globin intronic enhancer, or synthetic intronic enhancer, or any combination thereof. In some specific embodiments, the nucleic acid sequence of the CMV enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:16. The nucleic acid sequence of the SV40 intronic enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 17. The nucleic acid sequence of the MVM intronic enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:18. The nucleic acid sequence of the β-globin intronic enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to SEQ ID NO:19. The nucleic acid sequence of the synthetic intronic enhancer comprises or is a sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs:20-22.

In some specific embodiments, the regulatory element may comprise one enhancer or multiple enhancers, and the multiple enhancers may be the same or different.

In some embodiments, the regulatory element further comprises a woodchuck hepatitis virus post-transcriptional regulatory element (WPRE). For example, the regulatory element comprises a promoter regulating the nucleic acid expression of the artificial transcription factor and a WPRE, or the regulatory element comprises a promoter regulating the nucleic acid expression of the artificial transcription factor, a WPRE, and an enhancer that increases transcription of the artificial transcription factor.

In some embodiments, the composition comprises or is a nucleic acid sequence having at least 70%, at least 80%, at least 85%, at least 90%, at least 91%, at least 92%, at least 93%, at least 94%, at least 95%, at least 96%, at least 97%, at least 98%, at least 99%, or 100% identity to any one of SEQ ID NOs:36, 37, or 62.

In a third aspect of the present invention, a method is provided for transdifferentiating non-neuronal cells of a subject into functional neurons, which comprises contacting the expression vector according to any one of the first aspect of the present invention or the composition according to any one of the second aspect of the present invention with the non-neuronal cells. In some specific embodiments, the contact is performed in vitro. In some specific embodiments, the contact is performed in vivo.

In some embodiments, the functional neurons include dopamine neurons, retinal ganglion cells, photoreceptor cells, cochlear spiral ganglion neurons, GABA neurons, 5-HT neurons, glutamatergic neurons, ChAT neurons, NE neurons, motor neurons, spinal cord neurons, spinal motor neurons, spinal sensory neurons, bipolar cells, amacrine cells, pyramidal cells, interneurons, medium spiny neurons (MSNs), Purkinje cells, granule cells, olfactory sensory neurons, periglomerular cells, or any combination thereof. In some embodiments, the dopamine neurons express one or more of the following markers: NeuN, TH, FoxA2, Nurr1, Pitx3, Vmat2, or DAT. In some embodiments, the retinal ganglion neurons express one or more of the following markers: RBPMS, Pax6, Brn3a, Brn3b, Brn3c, and Map2. In some embodiments, the photoreceptor cells express one or more of the following markers: rhodopsin, mCAR, m-opsin, or s-opsin. In some embodiments, the cochlear spiral ganglion neurons express one or more of the following markers: NeuN, Prox1, Tuj-1, and Map2.

In some embodiments, the functional neurons have axons.

In some embodiments, the non-neuronal cells include glial cells, fibroblasts, stem cells, neural progenitor cells, or neural stem cells.

In some embodiments, the glial cells include astrocytes, oligodendrocytes, ependymal cells, Schwann cells, NG2 cells, satellite cells, Müller cells, or inner ear glial cells.

In some embodiments, the efficiency of transdifferentiating non-neuronal cells into functional neurons is at least 1%.

In some embodiments, the glial cells are located in the brain, spinal cord, eyes, or ears. In some embodiments, the glial cells are located in the striatum, substantia nigra, ventral tegmental area of the midbrain, medulla oblongata, hypothalamus, dorsal midbrain, or cerebral cortex of the brain.

In some embodiments, the glial cells comprise astrocytes, and the functional neurons comprise dopamine neurons. In some embodiments, the method provided in this aspect comprises a method for transdifferentiating astrocytes into dopamine neurons in a subject. In some embodiments, the astrocytes are located in the striatum and/or substantia nigra. In some embodiments, the method comprises administering the expression vector or composition provided herein, or a product containing the expression vector or composition provided herein, to the striatum and/or substantia nigra of the subject.

In some embodiments, the glial cells comprise Müller cells, and the functional neuronal cells comprise retinal ganglion cells (RGCs) and/or photoreceptor cells. In some embodiments, the method provided in this aspect involves transdifferentiating Miiller cells into retinal ganglion cells (RGCs) and/or photoreceptor cells in a subject. In some embodiments, the Müller cells are located under the retina or in the vitreous cavity. In some embodiments, the method comprises administering the expression vector or composition provided herein, or a product containing the expression vector or composition provided herein, to the subretinal space or vitreous cavity of the subject.

In some embodiments, the glial cells comprise cochlear glial cells, and the functional neuronal cells comprise cochlear spiral ganglion neurons. In some embodiments, the method provided herein involves transdifferentiating cochlear glial cells into cochlear spiral ganglion neurons in a subject. In some embodiments, the cochlear glial cells are located in the inner ear. In some embodiments, the method comprises administering the expression vector or composition provided herein, or a product containing the expression vector or composition provided herein, to the inner ear of the subject.

In a fourth aspect of the present invention, a method for treating a disease in a subject in need is provided, comprising administering a therapeutically effective amount of the expression vector according to any embodiment of the first aspect of the present invention, or administering a therapeutically effective amount of the composition according to any embodiment of the second aspect of the present invention.

In some embodiments, the disease comprises Parkinson's disease, Alzheimer's disease, stroke, schizophrenia, Huntington's disease, depression, motor neuron disease, ALS, spinal muscular atrophy, epilepsy, ataxia, visual impairment caused by RGC cell death, glaucoma, age-related RGC damage, optic nerve injury, retinal ischemia or hemorrhage, Leber's hereditary optic neuropathy, degeneration or death of photoreceptor cells due to trauma or degeneration, macular degeneration, retinitis pigmentosa, diabetes-related blindness, night blindness, color blindness, hereditary blindness, spiral ganglion cell death, or any combination thereof.

In some embodiments, the disease comprises Parkinson's disease. In some embodiments, the administration is performed in the brain of the subject in need. In some embodiments, the administration is performed in the striatum or substantia nigra of the subject in need thereof.

In some embodiments, the disease comprises an optic nerve disease. In some embodiments, the administration is performed in the eye of the subject in need. In some embodiments, the administration is performed in the vitreous cavity or subretinal space of the subject in need. In some embodiments, the optic nerve disease includes but is not limited to visual impairment caused by RGC cell death, glaucoma, or age-related RGC damage.

In the present application, when describing that a given domain is located at the N- or C-terminus of the RZFD core domain, it means that the given domain can be located close to the N terminus of the RZFD core domain or be located on the N terminus side of the RZFD core domain with other domains intervening, or it means that the given domain can be located close to the C-terminus of the RZFD core domain or be located on the C-terminal side with other domains intervening.

For example, an NLS is located at the N-terminus and/or C-terminus of the RZFD core domain, it may be understood as the NLS being directly adjacent to the N-terminus of the RZFD core domain or positioned on the N-terminal side with some amino acid sequences intervening, or it can be understood as the NLS being directly adjacent to the C-terminus of the RZFD core domain or positioned on the C-terminal side with some amino acid sequences intervening.

The present application also relates to the use of the aforementioned expression vector or composition for transdifferentiating non-neuronal cells into functional neurons, or for preparing a medicament for transdifferentiating non-neuronal cells into functional neurons.

All details described in the aforementioned methods for transdifferentiating non-neuronal cells into functional neurons are applicable to the use for transdifferentiating non-neuronal cells into functional neurons or for preparing a medicament for transdifferentiating non-neuronal cells into functional neurons.

The present application further relates to the use of the aforementioned expression vector or composition for treating diseases, or for preparing a medicament for treating diseases. The diseases include Parkinson's disease, Alzheimer's disease, stroke, schizophrenia, Huntington's disease, depression, motor neuron disease, ALS, spinal muscular atrophy, epilepsy, ataxia, visual impairment caused by RGC cell death, glaucoma, age-related RGC damage, optic nerve injury, retinal ischemia or hemorrhage, Leber's hereditary optic neuropathy, degeneration or death of photoreceptor cells due to trauma or degeneration, macular degeneration, retinitis pigmentosa, diabetes-related blindness, night blindness, color blindness, hereditary blindness, spiral ganglion cell death, or any combination thereof.

The present application also relates to the artificial transcription factor mentioned above, which comprises RZFD core domain, wherein, the amino acid sequence of the RZFD core domain comprises any 5-8 sequences of SEQ ID NOs: 3-10 and does not include the sequences shown in SEQ ID NO: 12 and 13. Preferably, the amino acid sequence of the RZFD core domain comprises any 6-8 sequences of SEQ ID NOs: 3-10. All artificial transcription factors described in other aspects above are applicable to the artificial transcription factor described in this aspect.

Through detailed description, additional aspects and advantages of the present invention will be apparent to those skilled in the art. The methods disclosed and described are only as exemplary embodiments. It should be understood that the present invention also includes other different embodiments, and some details may include various obvious modifications, all of which are within the scope of the present disclosure. Accordingly, the figures and description are to be regarded as illustrative rather than restrictive.

The present application further relates to the following:
**1.** An expression vector comprising: (a) a nucleic acid sequence encoding a RE1 silencing transcription factor (REST) variant, wherein the REST variant comprises a DNA binding domain of REST; (b) a promoter; (c) an enhancer. **2.** The expression vector according to item 1, wherein the enhancer comprises a CMV enhancer, a SV40 intronic enhancer, a MVM intronic enhancer, a β-globin intronic enhancer, or a synthetic intronic enhancer. **3.** The expression vector according to item 2, wherein the CMV enhancer comprises a nucleic acid sequence having at least 70% identity to SEQ ID NO:16. **4.** The expression vector according to item 2, wherein the SV40 intronic enhancer comprises a nucleic acid sequence having at least 70% identity to SEQ ID NO:17. **5.** The expression vector according to item 2, wherein the MVM intronic enhancer comprises a nucleotide sequence having at least 70% identity to SEQ ID NO:18. **6.** The expression vector according to item 2, wherein the β-globin intronic enhancer comprises a nucleotide sequence having at least 70% identity to SEQ ID NO:19. **7.** The expression vector according to item 2, wherein the synthetic enhancer comprises a nucleotide sequence having at least 70% identity to any one of SEQ ID NOs:20-22. **8.** The expression vector according to any one of items 1-7, wherein the promoter comprises a glial cell-specific promoter. **9.** The expression vector according to item 8, wherein the glial cell-specific promoter comprises an astrocyte-specific promoter or a Müller cell-specific promoter. **10.** The expression vector according to item 8, wherein the glial cell-specific promoter comprises a GFAP promoter, an ALDH1L1 promoter, an EAAT1/GLAST promoter, a glutamine synthetase promoter, an S100β promoter, an EAAT2/GLT-1 promoter, or an Rlbp1 promoter. **11.** The expression vector according to item 8, wherein the glial cell-specific promoter comprises a cochlear glial cell-specific promoter. **12.** The expression vector according to item 11, wherein the cochlear glial cell-specific promoter comprises a GFAP promoter, an ALDH1L1 promoter, an EAAT1/GLAST promoter, or a Plp1 promoter. **13.** The expression vector according to any one of items 1-12, wherein the expression vector further comprises a woodchuck hepatitis virus post-transcriptional regulatory element (WPRE). **14.** The expression vector according to any one of items 1-13, wherein the DNA-binding domain of REST comprises an RE1-binding domain. **15.** The expression vector according to item 14, wherein the RE1-binding domain comprises a sequence having at least 70% identity to SEQ ID NO:2 or 14. **16.** The expression vector according to item 15, wherein the RE1-binding domain is encoded by a sequence having at least 70% identity to SEQ ID NO:15. **17.** The expression vector according to any one of items 1-16, wherein the REST variant further comprises a nuclear localization sequence (NLS). **18.** The expression vector according to item 17, wherein the NLS is located at the N-terminus or C-terminus of the DNA-binding domain of REST. **19.** The expression vector according to item 18, wherein the NLS is located at both the N-terminus and C-terminus of the DNA-binding domain of REST. **20.** The expression vector according to item 17, wherein the NLS comprises an amino acid sequence having at least 70% identity to SEQ ID NO:24. **21.** The expression vector according to item 20, wherein the NLS is encoded by a nucleotide sequence having at least 70% identity to SEQ ID NO:25. **22.** The expression vector according to any one of items 1-21, wherein the REST variant further comprises a gene activation domain. **23.** The expression vector according to item 22, wherein the gene activation domain comprises VP64, P65-HSF1, VP16, RTA, Suntag, P300, CBP, or any combination thereof. **24.** The expression vector according to item 23, wherein the gene activation domain comprises VP64. **25.** The expression vector according to item 24, wherein VP64 is located at the C-terminus or N-terminus of the DNA-binding domain of REST. **26.** The expression vector according to item 25, wherein VP64 is located at both the C-terminus and N-terminus of the DNA-binding domain of REST. **27.** The expression vector according to any one of items 23-26, wherein VP64 comprises an amino acid sequence having at least 70% identity to SEQ ID NO:26. **28.** The expression vector according to item 27, wherein VP64 is encoded by a nucleotide sequence having at least 70% identity to SEQ ID NO:27. **29.** The expression vector according to item 23, wherein the gene activation domain comprises the P65-HSF1. **30.** The expression vector according to item 29, wherein P65-HSF1 is located at the N-terminus or C-terminus of the DNA-binding domain of REST. **31.** The expression vector according to item 30, wherein P65-HSF1 is located at both the N-terminus and C-terminus of the DNA-binding domain of REST. **32.** The expression vector according to any one of items 29-31, wherein P65-HSF1 comprises an amino acid sequence having at least 70% identity to SEQ ID NO:28. **33.** The expression vector according to item 32, wherein P65-HSF1 is encoded by a nucleotide sequence having at least 70% identity to SEQ ID NO:29. **34.** The expression vector according to any one of items 1-33, wherein the REST variant further comprises an NLS and a gene activation domain. **35.** The expression vector according to item 34, wherein the gene activation domain comprises VP64. **36.** The expression vector according to item 35, wherein the NLS and VP64 are located at the N-terminus and C-terminus of the DNA-binding domain of REST. **37.** The expression vector according to item 36, wherein the REST variant comprises an amino acid sequence having at least 70% identity to SEQ ID NO:32. **38.** The expression vector according to item 37, wherein the REST variant is encoded by a nucleotide sequence having at least 70% identity to SEQ ID NO:33. **39.** The expression vector according to item 34, wherein the gene activation domain comprises P65-HSF1. **40.** The expression vector according to item 39, wherein the NLS and P65-HSF1 are located at the N-terminus and C-terminus of the DNA-binding domain of REST. **41.** The expression vector according to any one of items 1-40, wherein the REST variant lacks the inhibitory domains of REST at the N-terminus and C-terminus. **42.** The expression vector according to item 40, wherein the REST variant comprises an amino acid sequence having at least 70% identity to any one of SEQ ID NOs:34, 67, 84, 86, 90, or 92. **43.** The expression vector according to item 40, wherein the REST variant is encoded by a nucleotide sequence having at least 70% identity to any one of SEQ ID NOs:35, 68, 85, 87, 91, or 93. **44.** A composition comprising: (a) a REST variant comprising a DNA-binding domain of REST, a first NLS, a second NLS, and a first gene activation domain; or (b) a nucleic acid encoding the REST variant thereof. **45.** The composition according to item 44, wherein the DNA-binding domain comprises an RE1 binding domain. **46.** The composition according to item 44, wherein the first NLS is located at the N-terminus of the DNA binding domain of REST. **47.** The composition according to item 46, wherein the second NLS is located at the C-terminus of the DNA binding domain of REST. **48.** The composition according to item 46, wherein the first gene activation domain is located at the N-terminus of the first NLS. **49.** The composition according to any one of items 44-47, wherein the REST variant further comprises a second gene activation domain. **50.** The composition according to item 49, wherein the second gene activation domain is located at the C-terminus of the second NLS. **51.** The composition according to any one of items 44-50, wherein the DNA binding domain of REST comprises an amino acid sequence having at least 70% identity to SEQ ID NO:2 or 14. **52.** The composition according to item 51, wherein the DNA binding domain is encoded by a nucleotide sequence having at least 70% identity to SEQ ID NO:15. **53.** The composition according to any one of items 44-50, wherein the first NLS comprises an amino acid sequence having at least 70% identity to SEQ ID NO:24. **54.** The composition according to item 53, wherein the first NLS is encoded by a nucleotide sequence having at least 70% identity to SEQ ID NO:25. **55.** The composition according to any one of items 44-54, wherein the second NLS comprises an amino acid sequence having at least 70% identity to SEQ ID NO:24. **56.** The composition according to item 55, wherein the second NLS is encoded by a nucleotide sequence having at least 70% identity to SEQ ID NO:25. **57.** The composition according to any one of items 44-56, wherein the first gene activation domain comprises VP64. **58.** The composition according to item 57, wherein VP64 has an amino acid sequence having at least 70% identity to SEQ ID NO:26. **59.** The composition according to item 58, wherein VP64 is encoded by a nucleotide sequence having at least 70% identity to SEQ ID NO:27. **60.** The composition according to any one of items 44-56, wherein the first gene activation domain comprises P65-HSF1. **61.** The composition according to item 60, wherein P65-HSF1 comprises an amino acid sequence having at least 70% identity to SEQ ID NO:28. **62.** The composition according to item 61, wherein P65-HSF1 is encoded by a nucleotide sequence having at least 70% identity to SEQ ID NO:29. **63.** The composition according to item 49, wherein the second gene activation domain comprises VP64. **64.** The composition according to item 63, wherein VP64 comprises an amino acid sequence having at least 70% identity to SEQ ID NO:26. **65.** The composition according to item 64, wherein VP64 is encoded by a nucleotide sequence having at least 70% identity to SEQ ID NO:27. **66.** The composition according to item 49, wherein the second gene activation domain comprises P65-HSF1. **67.** The composition according to item 66, wherein P65-HSF1 comprises an amino acid sequence having at least 70% identity to SEQ ID NO:28. **68.** The composition according to item 67, wherein P65-HSF1 is encoded by a nucleotide sequence having at least 70% identity to SEQ ID NO:29. **69.** The composition according to any one of items 44-68, wherein the REST variant comprises an amino acid sequence having at least 70% identity to any one of SEQ ID NOs:32, 34, 67, 84, 86, 90, or 92. **70.** The composition according to item 69, wherein the nucleic acid encoding the REST variant comprises a nucleotide sequence having at least 70% identity to any one of SEQ ID NOs:33, 35, 68, 85, 87, 91, or 93. **71.** A method for transdifferentiating non-neuronal cells into functional neurons in a subject, comprising administering to the subject the expression vector according to any one of items 1-43 or the composition according to any one of items 44-70. **72.** A method for transdifferentiating non-neuronal cells into functional neurons in vitro, comprising contacting cells with the expression vector according to any one of items 1-43 or the composition according to any one of items 44-70. **73.** The method according to item 71 or 72, wherein the functional neurons comprise dopamine neurons, retinal ganglion cells, photoreceptor cells, cochlear spiral ganglion neurons, GABA neurons, 5-HT neurons, glutamatergic neurons, ChAT neurons, NE neurons, motor neurons, spinal cord neurons, spinal motor neurons, spinal sensory neurons, bipolar cells, horizontal cell, amacrine cells, pyramidal cells, interneurons, medium spiny neurons (MSNs), Purkinje cells, granule cells, olfactory sensory neurons, periglomerular cells, or any combination thereof. **74.** The method according to item 73, wherein the dopamine neurons express one or more markers selected from the group consisting of NeuN, TH, FoxA2, Nurr1, Pitx3, Vmat2, or DAT. **75.** The method according to item 73, wherein the retinal ganglion neurons express one or more markers selected from the group consisting of RBPMS, Pax6, Brn3a, Brn3b, Brn3c, and Map2. **76.** The method according to item 73, wherein the photoreceptor cells express one or more markers selected from the group consisting of rhodopsin, mCAR, m-opsin, or s-opsin. **77.** The method according to item 73, wherein the cochlear spiral ganglion neurons express one or more markers selected from the group consisting of NeuN, Prox1, Tuj-1, and Map2. **78.** The method according to any one of items 71-77, wherein the functional neurons comprise axons. **79.** The method according to any one of items 71-77, wherein the non-neuronal cells comprise glial cells, fibroblasts, stem cells, neural progenitor cells, or neural stem cells. **80.** The method according to item 79, wherein the glial cells comprise astrocytes, oligodendrocytes, ependymal cells, Schwann cells, NG2 cells, satellite cells, Müller cells, or inner ear glial cells. **81.** The method according to any one of items 71-80, wherein the transdifferentiating efficiency of non-neuronal cells to functional neurons is at least 1%. **82.** A method for treating a disease in a subject in need thereof, comprising administering a therapeutically effective amount of the expression vector according to any one of items 1-43 or the composition according to any one of items 44-70. **83.**The method according to item 82, wherein the disease comprises Parkinson's disease, Alzheimer's disease, stroke, schizophrenia, Huntington's disease, depression, motor neuron disease, ALS, spinal muscular atrophy, Pick's disease, sleep disorders, epilepsy, ataxia, visual impairment caused by RGC cell death, glaucoma, age-related RGC damage, optic nerve injury, retinal ischemia or hemorrhage, Leber's hereditary optic neuropathy, degeneration or death of photoreceptor cells due to trauma or degeneration, macular degeneration, retinitis pigmentosa, diabetes-related blindness, night blindness, color blindness, hereditary blindness, spiral ganglion cell death, or any combination thereof.

### Incorporation by reference

All publications, patents, and patent applications mentioned in this specification are herein incorporated by reference to the same extent as if each individual publication, patent, or patent application is specifically and individually indicated to be incorporated by reference. To the extent publications and patents or patent applications incorporated by reference contradict the disclosure contained in the specification, the specification is intended to supersede and/or take precedence over any such contradictory material.

### Description of the drawings

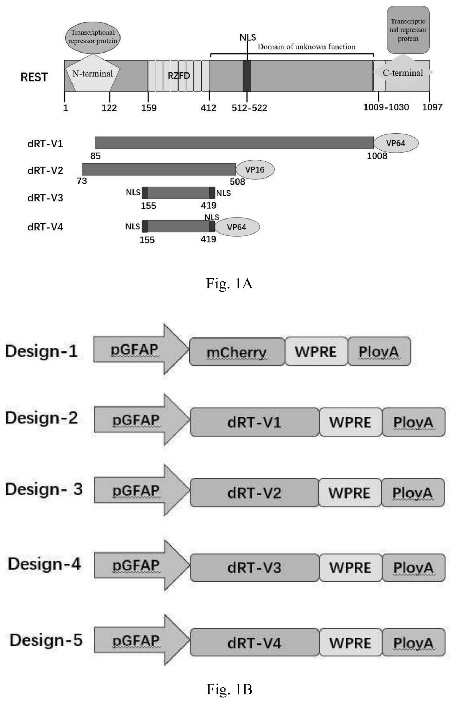

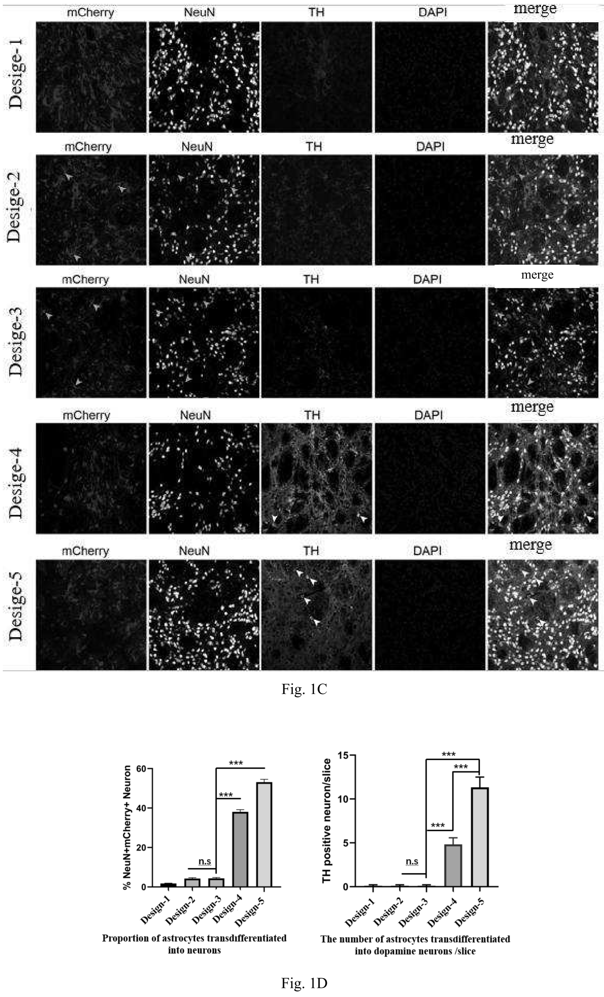

**Figure 1****:** In vivo transdifferentiation efficiency of different versions of artificial transcription factors. **(****Fig. 1A****)** Schematic of the functional domains of the REST protein and the designs of different versions of artificial transcription factors, wherein NLS, VP16, and VP64 are added sequences. **(****Fig. 1B****)** Schematic of various AAV expression vectors, using the GFAP promoter to achieve specific expression in astrocytes, wherein mCherry is the coding sequence of the red fluorescent protein, while dRT-V1, dRT-V2, dRT-V3, and dRT-V4 represent different versions of artificial transcription factors. **(****Fig. 1C****)** Results of in vivo transdifferentiation using different versions of artificial transcription factors. Wherein the design-1 is the control group, while design-2 to design-5 demonstrate the results of in vivo transdifferentiation performed by different artificial transcription factor, red fluorescence indicates mCherry expression, NeuN is a neuron-specific protein marker, TH is a dopamine neuron-specific protein marker, and DAPI stains cell nuclei. Scale bar is 50 µm. **(****Fig. 1D****)** The proportion of astrocytes transdifferentiated into neurons in each group. Design-1 serves as the control group, which shows less than 2% mCherry+NeuN double-positive cells. The group of design-2 and design-3 shows approximately 5% mCherry+NeuN double-positive cells, while the group of design-4 and design-5 show significantly higher proportions of mCherry+NeuN double-positive cells. Statistical results of TH staining, a specific protein marker for dopamine neurons, showed that no TH-positive cells are observed in design-1, design-2, and design-3 groups, while TH-positive cells are observed in design-4 and design-5 groups. The number of TH-positive cells in design-5 is approximately twice that of design-4. Statistical analysis is performed using t-test. Error bars represent SEM. * denotes P < 0.05, ** denotes P < 0.01, and *** denotes P < 0.001.

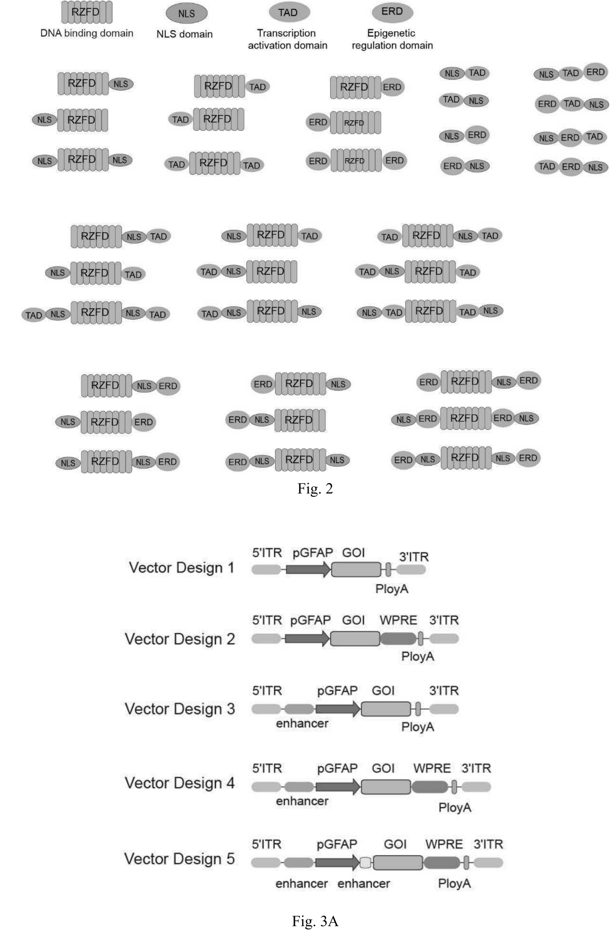

**Figure 2****:** Schematic diagram of an artificial transcription factor. RZFD is the DNA-binding sequence, NLS is the nuclear localization sequence, TAD is the transcription activation domain, and EPD is the epigenetic regulation domain. RZFD may be used alone or in any combination with one or more of NLS, TAD, or EPD. The NLS, TAD, EPD, or their combined elements may be positioned at either the N-terminus or C-terminus of RZFD.

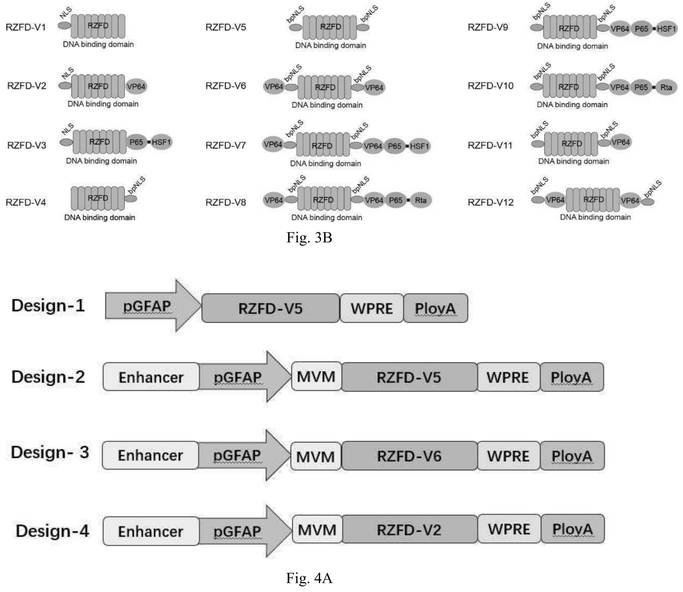

**Figure 3****:** Exemplary vector design schemes. **(****Fig. 3A****)** illustrates different vector design strategies, the astrocyte-specific GFAP promoter is used to drive expression of the artificial transcription factor (i.e., gene of interest(GOI) in the figure) (plasmid design 1); or the WPRE regulatory sequence is added to the artificial transcription factor (plasmid design 2); or an enhancer is added in front of the promoter (plasmid design 3); or both an enhancer and WPRE are added (plasmid design 4); or different enhancers are added (lasmid Design 5). **(****Fig. 3B****)** shows the design schemes of various artificial transcription factors (ATFs) used in the examples, and each ATF is named from RZFD-V1 to RZFD-V12.

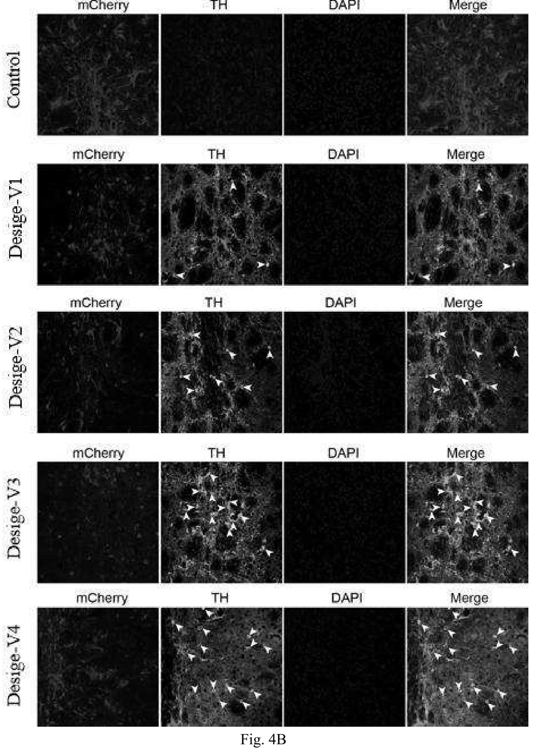

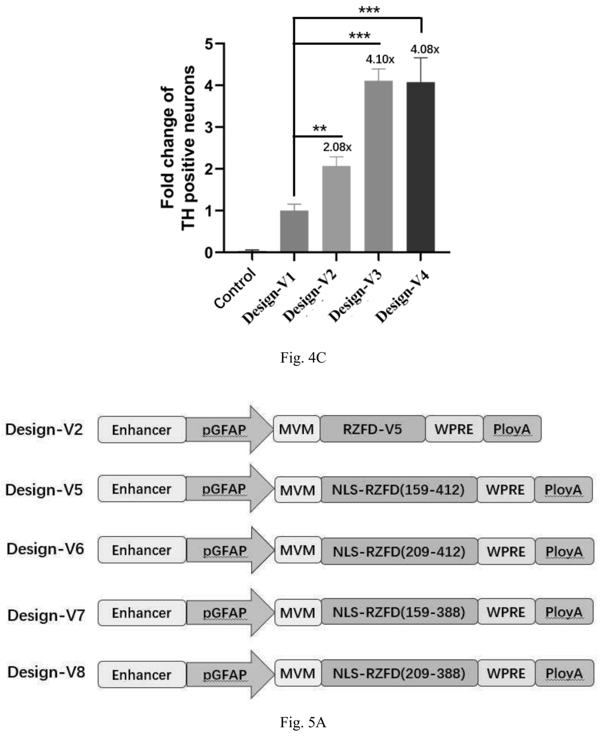

**Figure 4****:** Comparison of in vivo transdifferentiation into dopamine neurons with different vector designs. **Fig. 4A** shows the designs of the vectors used in Fig. 4B. Design-V1 uses the glial cell-specific promoter GFAP to drive RZFD-V5. Design-V2 adds two enhancers (including the CMV enhancer and MVM intronic enhancer) based on the framework of design-V1. Compared to design-V2, design-V3 expresses RZFD-V6, whereas design-V2 expresses RZFD-V5. RZFD-V6 is based on RZFD-V5 with the addition of one VP64 domain at each end. Design-V4 uses RZFD-V2 as the gene of interest (GOI), which, compared to RZFD-V6, has only one VP64 and one NLS located at either end of RZFD. **Fig. 4B** shows the results of in vivo transdifferentiation using the designs from Fig. 4A. The control group is injected with AAV-pGFAP-mCherry, while design-V1 to design-V4 are injected with AAV-pGFAP-dRT-V5, AAV-CMV enhancer-pGFAP-MVM-dRT-V5, AAV-CMV enhancer-pGFAP-MVM-dRT-V6, and AAV-CMV enhancer-pGFAP-MVM-dRT-V2, respectively. TH is a specific protein marker of dopamine neurons, DAPI stains nuclei, and white arrows indicate TH-positive neurons. The scale bar is 50 µm. **Fig. 4C** shows the statistical results of the number of TH-positive cells in each group, which is expressed as fold change relative to design-V1. The control group showed no TH-positive cells. The design-V2 group (with enhancers) produced approximately twice as many TH-positive cells as design-V1. The design-V3 group produced about 4 times as many TH-positive cells as design-V1 and twice as many as design-V2. The design-V4 group produced a similar number of TH-positive cells as design-V3, both approximately 4 times higher than design-V1 and twice higher than design-V2. Statistical analysis is performed using t-test. Error bars represent SEM. * denotes P < 0.05, ** denotes P < 0.01, and *** denotes P < 0.001.

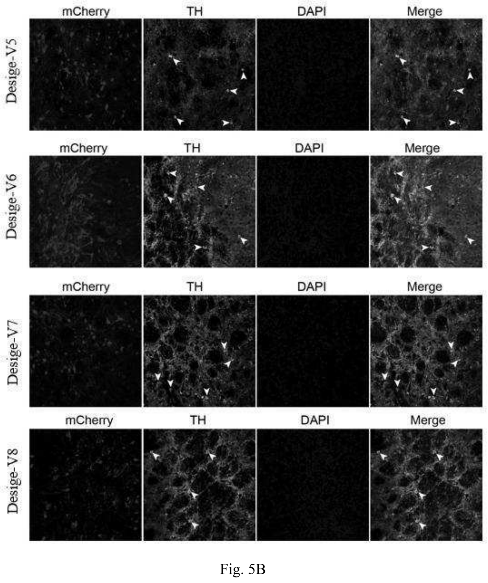

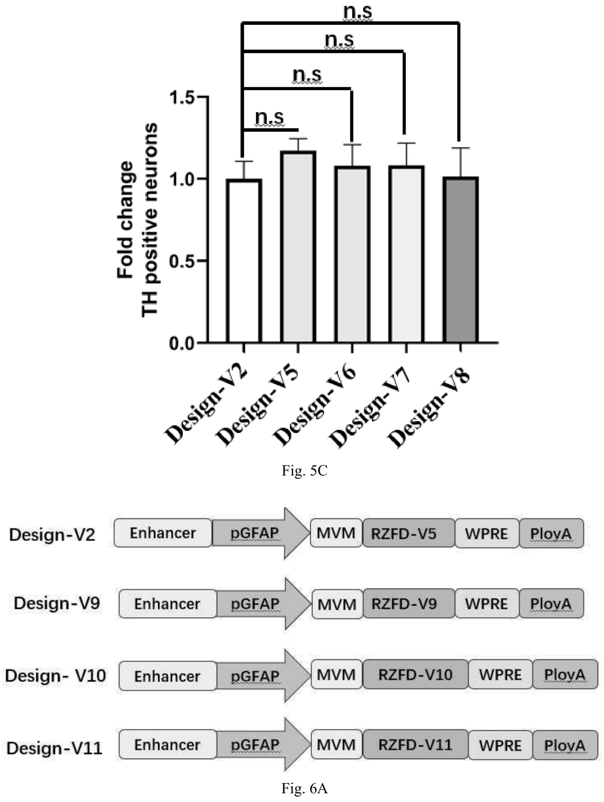

**Figure 5****:** Comparison of in vivo transdifferentiation efficiency using different truncated RZFD variants. **Fig. 5A** illustrates the schematic diagrams of the vector designs used in the experiment in Fig. 5B. The ATF region of design-V5 is NLS-RZFD (159-412) with 8 zinc finger domains. The ATF region of design-V6 is NLS-RZFD (209-412) with 7 zinc fingers (ZF2-8). The ATF region of design-V7 is NLS-RZFD (159-388) with 7 zinc fingers (ZF1-7). The ATF region of design-V8 is NLS-RZFD (209-388) with 6 zinc fingers (ZF2-7). The remaining parts of the vectors are same in design. **Fig. 5B** shows the in vivo transdifferentiation effects of the various ATF designs depicted in Fig. 5A. TH is a specific protein marker for dopaminergic neurons, and DAPI stains the cell nuclei. White arrows indicate TH-positive neurons. Designs V5 to V8 all successfully transdifferentiated astrocytes into dopamine neurons. Scale bar: 50 µm. **Fig. 5C** shows the statistical results of number of TH-positive cells in each group. Using design-V2 as the reference, the fold change in TH-positive cell numbers in other groups is calculated relative to that in design-V2. Designs V5-V8 produced similar numbers of TH-positive cells as design-V2, with no statistically significant differences. Statistical analysis is performed using t-test. Error bars represent SEM. * denotes P < 0.05, ** denotes P < 0.01, and *** denotes P < 0.001.

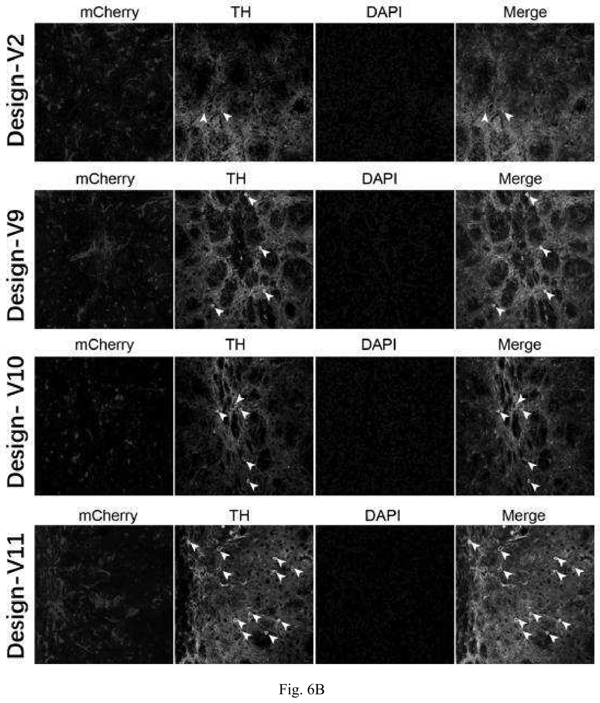

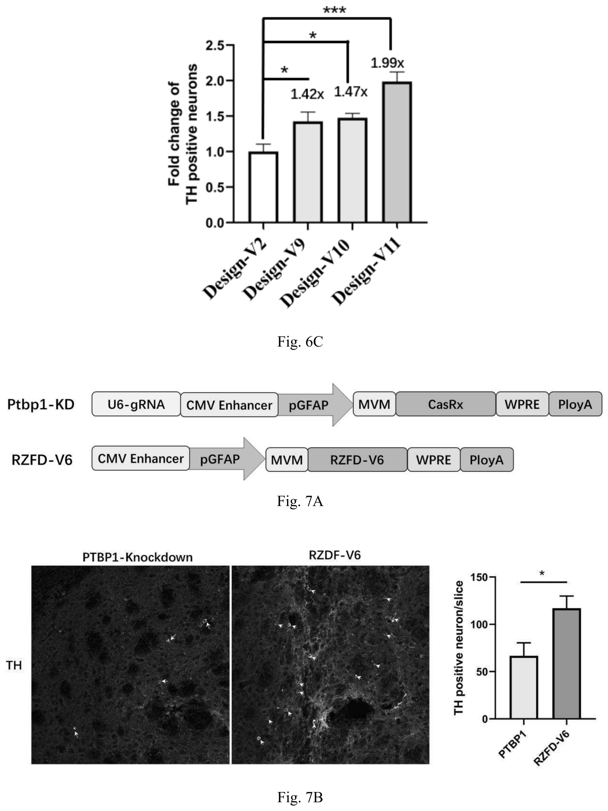

**Figure 6****:** Comparison of transdifferentiation efficiency using different activation domains. **Fig. 6A** shows the schematic diagrams of the vector designs used in this experiment of Fig. 6B. Like the design strategy of design-V2, the ATF of design-V9 is RZFD-V9 while the activation domain is VP64-p65-HSF1, the ATF of design-V10 is RZFD-V10 while the activation domain is VP64-p65-Rta, the ATF of design-V11 is RZFD-V11 while the activation domain is VP64. **Fig. 6B** shows the representative images of in vivo transdifferentiation results for the various ATF designs shown in Fig. 6A. TH is a specific protein marker for dopaminergic neurons, and DAPI stains nuclei. White arrows indicate TH-positive neurons. Designs V2-V11 all successfully generated dopamine neurons through transdifferentiation. Scale bar: 50 µm. **Fig. 6C** shows the statistical results of the number of TH-positive cells in each group. Using design-V2 as the reference, the fold change in TH-positive cell numbers in other groups is calculated relative to that in design-V2. Design-V9 and design-V10 produced similar numbers of TH-positive cells, which is 1.42-fold and 1.47-fold relative to Design-V2 respectively. The design-V10 group yielded the highest number of TH-positive cells, which is 1.99-fold relative to design-V2. Differences are analyzed using t-test. Error bars represent SEM. * denotes P < 0.05, ** denotes P < 0.01, and *** denotes P < 0.001.
Figure 7: Comparison of in vivo transdifferentiation effects between RZFD-V6 and knockdown of Ptbp1 expression. **Fig. 7A** shows schematic diagrams of the various vector designs used in the experiments of Fig. 7B. The Ptbp1-KD vector is designed with U6 as the gRNA promoter and GFAP as the promoter for CasRx expression. The GFAP and enhancer are consistent with the RZFD-V6 expression vector (design-V3 in this study). **Fig. 7B** shows representative images and statistical results of the in vivo transdifferentiation effects of knockdown of Ptbp1 or overexpression of RZFD-V6. TH is a dopamine neuron-specific protein marker. White arrows point to TH-positive neurons. The number of TH-positive neurons in the RZFD-V6 group is approximately twice that of the Ptbp1-KD group. Scale bar, 50 µm. Differences are analyzed using a t-test. Error bars represent SEM. * indicates P < 0.05, ** indicates P < 0.01, and *** indicates P < 0.001.

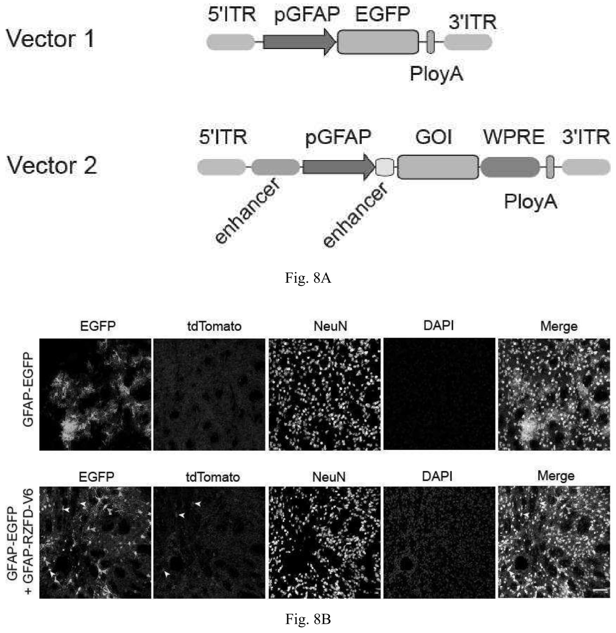

**Figure 8****:** Study of the transdifferentiation effect of RZFD-V6 in 6-OHDA-induced Dat-Cre::Ai9 lineage-tracing mice. **Fig. 8A** shows a schematic diagram of vector design. Vector 1 is an AAV for labeling, using the astrocyte-specific promoter GFAP to drive EGFP expression in astrocytes; vector 2 uses the astrocyte-specific promoter GFAP to drive expression of an artificial transcription factor (ATF: RZFD-V6). **Fig.8B** shows representative images of cells stained to show transdifferentiation of astrocytes into neurons or dopamine neurons after AAV injection in Dat-Cre::Ai9 mice induced by 6-OHDA. The red fluorescence indicates tdTomato expressed in mature dopamine neurons Dat-Cre::Ai9, while the white signal indicates staining for the neuron-specific protein marker NeuN. White arrows point to tdTomato-positive mature dopamine neurons. Scale bar: 50 µm.

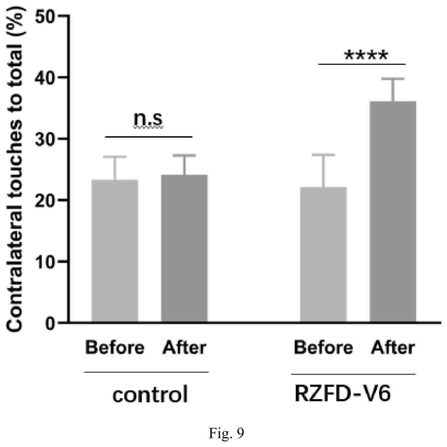

**Figure 9****:** Therapeutic effect of the artificial transcription factor RZFD-V6 in a PD mouse model. AAV containing RZFD-V6 is injected into a 6-OHDA-induced Parkinson's disease mouse model, and behavioral analysis is performed 1.5-3 months after treatment. The control group showed no difference between pre- and post-treatment, while the group injected with the artificial transcription factor RZFD-V6 showed significant behavioral improvements.

### Detailed Description

While various embodiments of the disclosure have been shown and described herein, it will be obvious to those skilled in the art that such embodiments are provided by way of example only. Numerous variations, changes, and substitutions may occur to those skilled in the art without departing from the disclosure. It should be understood that various alternatives to the embodiments of the disclosure described herein may be employed.

The present application provides expression vectors and compositions capable of regulating the repressor element 1/neuron-restrictive silencer element (RE1/NRSE). The application further discloses methods and uses of these expression vectors and compositions for transdifferentiating non-neuronal cells into functional neurons, as well as their therapeutic applications and methods for treating diseases, particularly neurological disorders.

The repressor element 1/neuron-restrictive silencer element (RE1/NRSE), which is also referred to as RE1 in this text, is a specific DNA sequence with a length of about 21 bp (ranging from 20-23 bp), mainly binding to REST (RE1-silencing transcription factor, also known as neuron-restrictive silencer factor (NRSF) to regulate the expression of genes related to neuron development and maturation. RE1 is a negative regulatory element involved in neuronal generation, and the first discovery is at the 5' end of promoters of NaV1.2 and SCG10, and which regulates the expression of genes related to neuronal development and maturation. In non-neuronal cells, the RE1 site is bound by a silencing complexe composed of histone deacetylases and methylases to inhibit the expression of neuron- related genes. However, there are more than 1,800 RE1 elements in mice and humans, making it difficult to regulate with existing technologies. For instance, CRISPR-mediated gene regulation and epigenetic modification techniques has very high precision and can precisely regulate the expression of specific genes, but it is difficult to regulate the expression of genes regulated by RE1 in this way.

Neurological disorders refer to diseases associated with neuronal death or degeneration, including Parkinson's disease, Alzheimer's disease, stroke, schizophrenia, Huntington's disease, depression, motor neuron diseases, ALS, spinal muscular atrophy, epilepsy, ataxia, visual impairment caused by RGC cell death, glaucoma, age-related RGC damage, optic nerve injury, retinal ischemia or hemorrhage, Leber's hereditary optic neuropathy, degeneration or death of photoreceptor cells due to trauma or degeneration, macular degeneration, retinitis pigmentosa, diabetes-related blindness, night blindness, color blindness, hereditary blindness, spiral ganglion cell death, and others.

Motor neuron diseases (MND) refer to progressive disorders caused by damage to motor neurons, including amyotrophic lateral sclerosis (ALS,), progressive bulbar palsy, primary lateral sclerosis, and progressive muscular atrophy.

This application provides expression vectors and compositions capable of modulating the binding of REST to RE1/NRSE, enabling the transdifferentiation of glial cells into neurons. In some embodiments, they can induce the transdifferentiation of astrocytes into dopamine neurons. In some embodiments, they can induce the transdifferentiation of Miiller cells into photoreceptor cells or retinal ganglion cells.

In some embodiments, the expression vectors and compositions provided herein are capable of transdifferentiating non-neuronal cells into functional neuronal cells in a subject. In some embodiments, the binding of REST to the RE1/NRSE element can be blocked by administering the expression vectors and compositions to a site of interest in vivo (e.g., a site affected by a disease), thereby achieving transdifferentiation of non-neuronal cells into functional neurons.

In some embodiments, the expression vector provided herein comprises:
(a) a nucleic acid sequence encoding an artificial transcription factor capable of binding to RE1, the artificial transcription factor comprising RZFD core domain; and
(b) a regulatory element for regulating expression of the artificial transcription factor nucleic acid; wherein the RZFD core domain comprises any 5-8 sequences of SEQ ID NOs: 3-10, and does not comprise the sequences shown in SEQ ID NOs: 12 and 13. In some embodiments, the amino acid sequence of the RZFD core domain comprises any 6-8 sequences of SEQ ID NOs: 3-10. In some embodiments, the amino acid sequence of the RZFD core domain may comprise the sequence shown in any one of SEQ ID NOs: 2, 14, 74, 76, and 75. In some embodiments, the amino acid sequence of the RZFD core domain may comprise a sequence having at least 70%, at least 80%, at least 85%, at least 90%, at least 91%, at least 92%, at least 93%, at least 94%, at least 95%, at least 96%, at least 97%, at least 98%, or at least 99% or 100% identity to the sequence shown in any one of SEQ ID NOs: 2, 14, 74, 76, and 75.

In some embodiments, the composition provided herein includes: (a) an artificial transcription factor comprising: RZFD core domain, a first NLS, and a first gene activation domain; or (b) a nucleic acid encoding the artificial transcription factor; wherein the RZFD core domain comprises any 5-8 sequences of SEQ ID NOs: 3-10, and does not comprise the sequences shown in SEQ ID NOs: 12 and 13. In some embodiments, the amino acid sequence of the RZFD core domain comprises any 6-8 sequences of SEQ ID NOs: 3-10. In some embodiments, the amino acid sequence of the RZFD core domain may comprise the sequence shown in any one of SEQ ID NOs: 2, 14, 74, 76, and 75. In some embodiments, the amino acid sequence of the RZFD core domain may comprises a sequence having at least 70%, at least 80%, at least 85%, at least 90%, at least 91%, at least 92%, at least 93%, at least 94%, at least 95%, at least 96%, at least 97%, at least 98%, or at least 99% or 100% identity to the sequence shown in any one of SEQ ID NOs: 2, 14, 74, 76, and 75.

The composition or expression vector provided herein further includes additional optimization of the artificial transcription factor, such as adding NLS, gene activation domain, etc., to achieve transdifferentiation from non-neuronal cells to functional neurons.

It should be understood that the numbering of specific positions or residues in the various sequences herein will depend on the specific protein and numbering scheme. Those skilled in the art will be able to identify corresponding residues in any homologous proteins and the corresponding encoding nucleic acids by methods well known in the art, such as by sequence alignment and determination of homologous residues.

### Definitions

Whenever the term "at least," "greater than," or "greater than or equal to" precedes the first numerical value in a series of two or more numerical values, the term "at least," "greater than," or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "not more than," "less than," or "less than or equal to" precedes first numerical value in a series of two or more numerical values, the term "not more than," "less than," or "less than or equal to" applies to each of the numerical values in the series of numerical values. For example, less than or equal to 3, 2, or 1 is equivalent to less than or equal to 3, less than or equal to 2, or less than or equal to 1.

As used herein, unless the context clearly indicates otherwise, the singular form shall also include the plural form. In addition, when the terms "comprising", "containing", "having", "with", "including", or other words with similar meanings are used in the detailed description and/or claims, these terms are intended to be inclusive in a manner similar to the term "comprising."

The terms "determine," "measure," "evaluate," "assess," and "analyze" are often used interchangeably herein to refer to forms of measurement. These terms include determining whether an element is present or not (e.g., detection). These terms may include quantitative, qualitative, or both quantitative and qualitative determinations. Assessing is relative or absolute. As used herein, "detecting" may include determining the amount of something present in addition to determining whether it is present or absent depending on the context.

The terms "about" or "approximately" refer to an acceptable error range for a particular value as determined by one of ordinary skill in the art, which will depend in part on how the value is measured or determined, e.g., the limitations of the measurement system. For example, "about" can mean within 1 or more than 1 standard deviation, per the practice in the given value. Where specific values are described in the application and claims, the term "about" should be assumed to mean an acceptable error range for the particular value unless otherwise stated.

As used herein, the phrases "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" each mean A alone, B alone, C alone, A and B together, A and C together, B and C together, or A, B, and C together.

As used herein, the term "nucleic acid" refers to a polymer containing at least two nucleotides (e.g., deoxyribonucleotides or ribonucleotides) in a single-stranded or double-stranded form, including DNA and RNA. A "nucleotide" comprises a deoxyribose (DNA) or ribose (RNA), a base, and a phosphate group. The nucleotides are linked together by the phosphate group. A "base" includes purines and pyrimidines, and also includes natural adenine, thymine, guanine, cytosine, uracil, inosine, and their natural analogs, as well as synthetic derivatives of purines and pyrimidines, including but not limited to substitutions with new reactive groups, such as, but not limited to, amines, alcohols, thiols, carboxylates, and alkyl halides. Nucleic acids include nucleic acids containing known nucleotide analogs or modified backbone residues or bonds, which are synthetic, naturally occurring, and non-naturally occurring, and exhibit similar binding properties to reference nucleic acids.. Examples of such analogs and/or modified residues include, but are not limited to, phosphorothioates, phosphoroamidates, methylphosphonates, chiral methylphosphonates, 2'-O-methyl ribonucleotides, and peptide nucleic acids (PNAs).

As used herein, the terms "protein," "polypeptide," and "peptide" are used interchangeably to refer to a polymer of amino acid residues linked by peptide bonds, which may be composed of two or more polypeptide chains. The terms "polypeptide," "protein," and "peptide" refer to a polymer of which at least two amino acid monomers are linked together by an amide bond. Amino acids can be L-optical isomers or D-optical isomers. More specifically, the terms "polypeptide," "protein," and "peptide" refer to a molecule composed of two or more amino acids in a specific order; for example, an order determined by the sequence of nucleotide in a gene or RNA encoding the protein. In some cases, a protein may be a fragment of the protein, for example, a domain, subdomain, or motif of the protein. In some cases, a protein may be a variant (or mutant) of the protein, having one or more amino acid residues inserted, deleted, and/or substituted into the amino acid sequence of a naturally occurring (or at least known) protein. A polypeptide may be a single linear polymer chain formed by linking the carboxyl and amino groups of adjacent amino acid residues via peptide bonds. Polypeptides can be modified, for example, by the addition of carbohydrates, phosphorylation, etc. A protein can include one or more polypeptides.

The protein or its variant can be naturally occurring or recombinant. Methods for detecting and/or measuring polypeptides in biological materials are well known in the art and include, but are not limited to, Western blotting, flow cytometry, ELISA, RIA, and various proteomics techniques. An exemplary method for measuring or detecting polypeptides is an immunoassay, such as ELISA. This type of protein quantification can be based on an antibody capable of capturing a specific antigen and a second antibody capable of detecting the captured antigen.

As used herein, the term "fragment" or equivalent terms may refer to a portion of a protein that is less than the full length of the protein and optionally retains the function of the protein. In addition, when the portion of the protein is aligned with the protein, the sequence of the portion of the protein can have, for example, at least 80% identity to the portion of the protein sequence.

Ranges provided herein are understood to be shorthand for all values within the range. For example, a range of 1 to 50 is understood to include any number, combination of numbers, or subrange within the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, and includes all intermediate decimal values between the above integers, such as 1.1, 1.2, 1.3, 1.4, 1.5, 1.6, 1.7, 1.8, and 1.9. Subranges are also included, as well as "nested subranges" extending from either endpoint of the range. For example, a nested subrange of 1 to 50 could include 1 to 10, 1 to 20, 1 to 30, and 1 to 40 in one direction, or 50 to 40, 50 to 30, 50 to 20, and 50 to 10 in the other direction.

Sequence identity can be measured using sequence analysis software (e.g., BLAST, BESTFIT, GAP, or PILEUP/PRITTYBOX programs). Such software matches identical or similar sequences by evaluating the identity of various substitutions, deletions, and/or other modifications. Conservative substitutions generally include substitutions within the following groups: glycine, alanine; valine, isoleucine, leucine; aspartic acid, glutamic acid, asparagine, glutamine; serine, threonine; lysine, arginine; and phenylalanine, tyrosine.

The term "nuclear localization sequence" (NLS), also known as nuclear localization signal (NLS), usually refers to a short amino acid sequence that, when fused to a protein, can guide the protein into the cell nucleus. Non-limiting examples of NLSs include NLS sequences derived from: the NLS of the SV40 virus large T antigen (e.g., the sequence shown in SEQ ID NO:38); the NLS of a nucleoplasmin (e.g., the sequence shown in SEQ ID NO:39); the NLS of c-myc (e.g., the sequence shown in SEQ ID NO:40 or 41); the NLS of hRNPA1 M9 (e.g., the sequence shown in SEQ ID NO:42); the sequence from the IBB domain of importin-α (e.g., the sequence shown in SEQ ID NO:43); the sequence of myoma T protein (e.g., the sequence shown in SEQ ID NO:44 or 45); the sequence of human p53 (e.g., the sequence shown in SEQ ID NO:46); the sequence of mouse c-abl IV (e.g., the sequence shown in SEQ ID NO:47); the sequence of influenza virus NS1 (e.g., the sequence shown in SEQ ID NO:48 or 49); the sequence of the hepatitis virus delta antigen (e.g., the sequence shown in SEQ ID NO:50); the sequence of the mouse Mx1 protein (e.g., the sequence shown in SEQ ID NO:51); sequence of human poly (ADP ribose) polymerase (e.g., sequence shown in SEQ ID NO: 52); and sequence of steroid hormone receptor (human) glucocorticoid (e.g., sequence shown in SEQ ID NO: 53).

In some embodiments, the RZFD core domain can be fused to one or more nuclear localization sequences (NLS), such as about or more than about 1, 2, 3, 4, 5, 6, 7, 8, 9, 10 or more NLS. In some embodiments, the area at or near the N-terminus and/or C-terminus of RZFD core domain comprises about 1, 2, 3, 4, 5, 6, 7, 8, 9, 10 or more NLS.

In one embodiment, an NLS is considered to be near the N-terminus or C-terminus of RZFD core domain when it is located within about 1, 2, 3, 4, 5, 10, 15, 20, 25, 30, 40, 50, or more amino acids from the N-terminus or C-terminus of the RZFD core domain along the polypeptide chain.

The term "gene activation domain" is a domain that can mediate transcription and activation, which can promote the binding of RNA polymerase and the promoter, thereby regulating the expression of genes (e.g., the RZFD core domain herein). Non-limiting examples of gene activation domains include gene activation domain sequences derived from VP64, P65, HSF1, VP16, MyoD1, RTA, SET7/9, Suntag, P300, CBP, or the activation domain of histone acetyltransferase, or combinations thereof, such as a combination of VP64 and P65, a combination of VP64, P65, and HSF1, a combination of MyoD1 and RTA, a combination of SET7/9, Suntag, and P300, and the like.

In some embodiments, the RZFD core domain can be fused to one or more gene activation domains, such as about or more than about 1, 2, 3, 4, 5, 6, 7, 8, 9, 10 or more gene activation domains. In some embodiments, the area at or near the N-terminus and/or C-terminus of RZFD core domain comprises about 1, 2, 3, 4, 5, 6, 7, 8, 9, 10 or more gene activation.

In one embodiment, a gene activation domain is considered to be near the N-terminus or C-terminus of RZFD core domain when it is located within about 1, 2, 3, 4, 5, 10, 15, 20, 25, 30, 40, 50, or more amino acids from the N-terminus or C-terminus of the RZFD core domain along the polypeptide chain.

In some embodiments, the NLS can be fused to one or more gene activation domains, such as about or more than about 1, 2, 3, 4, 5, 6, 7, 8, 9, 10 or more gene activation domains. In some embodiments, the area at or near the N-terminus and/or C-terminus of NLS comprises about 1, 2, 3, 4, 5, 6, 7, 8, 9, 10 or more gene activation domains.

In one embodiment, a gene activation domain is considered to be near the N-terminus or C-terminus of an NLS when it is located within about 1, 2, 3, 4, 5, 10, 15, 20, 25, 30, 40, 50, or more amino acids from the N-terminus or C-terminus of the NLS along the polypeptide chain.

The term "artificial transcription factor" refers to a protein that can bind to RE1 and promote neuron generation or regulate the expression of neuron-related genes. In some embodiments, the artificial transcription factor can compete with endogenous REST for binding to RE1/NRSE. The artificial transcription factor comprises RZFD core domain that can bind to RE1.

As used herein, the term "RZFD core domain", also referred to as "core domain", refers to a sequence comprising any 5-8 sequences of SEQ ID NOs: 3-10, and excluding the sequences of SEQ ID NOs: 12 and 13. In some embodiments, the amino acid sequence of the RZFD core domain comprises any 6-8 sequences of SEQ ID NOs: 3-10. In some embodiments, the amino acid sequence of the RZFD core domain may comprise the sequence of any one of SEQ ID NOs: 2, 14, 74, 76, and 75. In some embodiments, the amino acid sequence of the RZFD core domain may comprise a sequence having at least 70%, at least 80%, at least 85%, at least 90%, at least 91%, at least 92%, at least 93%, at least 94%, at least 95%, at least 96%, at least 97%, at least 98%, or at least 99%, or 100% identity to the sequence of any one of SEQ ID NOs: 2, 14, 74, 76, and 75.

As used herein, the terms "homology", "identity" or "similarity" of amino acid sequences or nucleotide sequences have the same meaning, and refer to the proportion of identical bases or amino acids in the entire sequence between one sequence and another compared sequence.

The term "enhancer" refers to a short region of DNA that can bind to proteins. After binding to proteins, it can increase the transcription of a target gene. Enhancers can be located upstream or downstream of the target gene. The sequence of an enhancer may be located close to the target gene or not.

The term "WPRE" stands for woodchuck hepatitis virus post-transcriptional regulatory element, which can increase the expression efficiency of exogenous gene fragments. For example, incorporating WPRE into the 3' UTR region of rAAV carrying an exogenous gene (e.g., nucleic acid encoding an artificial transcription factor) can increase mRNA expression levels and translation efficiency, thereby enhancing transgene expression.

The term "regulatory element" refers to a nucleic acid sequence that can regulate the transcription of a target gene, enhance transcript stability, or increase the protein expression level of a target gene. Exemplary regulatory elements can be promoters, enhancers, WPREs, etc.

### Artificial transcription factor and the RZFD core domain

RE1 can bind to REST (RE1 silencing transcription factor) to regulate the expression of genes related to neuronal development and maturation. REST is an endogenous protein that binds to RE1. The sequence of human wild-type REST protein is shown in SEQ ID NO: 1.

Through domain prediction and protein structure modeling, it is found that the amino acid sequence of the wild-type human REST protein contains eight zinc finger domains (RZFD) at positions 159-412. Residues 1-83 at the N-terminal amino acid sequence of REST contain an N-terminal repression domain, which can bind to proteins such as Sin3a and Sin3b. Residues 1008-1097 of the C-terminal amino acid sequence of REST include an inhibitory domain and one zinc finger domain, which can bind to proteins such as RCOR1.

The artificial transcription factor of the present application comprises RZFD core domain. This core domain is capable of binding to RE1 and promoting neuron generation or regulating the expression of neuron-related genes. In some embodiments, the artificial transcription factor can competitively bind to RE1 with endogenous REST.

In some embodiments, the artificial transcription factor may comprise a sequence as shown in SEQ ID NO: 2, 14, 30, 32, 34, 57, 59, 61, 63, 67, 77, 78, 79, 80, 81, 82, 84, 86, 90, or 92. In some embodiments, the artificial transcription factor may be a mutant sequence of the sequence as shown in SEQ ID NO: 2, 14, 30, 32, 34, 57, 59, 61, 63, 67, 77, 78, 79, 80, 81, 82, 84, 86, 90, or 92, and the mutant may include mutations such as, but not limited to, insertions, deletions, point mutations, InDel mutations, substitutions, or any combination thereof. In some embodiments, the artificial transcription factor can be a sequence that is at least 70%, at least 80%, at least 85%, at least 90%, at least 91%, at least 92%, at least 93%, at least 94%, at least 95%, at least 96%, at least 97%, at least 98%, or at least 99% identical to the sequence shown in SEQ ID NO: 2, 14, 30, 32, 34, 57, 59, 61, 63, 67, 77, 78, 79, 80, 81, 82, 84, 86, 90, or 92.

In some embodiments, the nucleic acid sequence of the mutant may comprise at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 25, at least about 30, at least about 35, at least about 40, at least about 45, at least about 50, or more mutations.

The nucleic acid sequence of the artificial transcription factor may comprise a codon-optimized form of REST.

In some embodiments, the coding sequence of the codon-optimized artificial transcription factor may comprise the nucleotide sequence of SEQ ID NO: 64. Optionally, the coding sequence of the codon-optimized artificial transcription factor may comprise a nucleotide sequence that has at least about 70%, at least about 80%, at least about 85%, at least about 90%, at least about 92%, at least about 94%, at least about 96%, at least about 97%, at least about 98%, or at least about 99% identical to SEQ ID NO: 64.

Codon optimization may increase expression compared to the coding sequence of a non-codon optimized artificial transcription factor. The coding sequence of a codon optimized artificial transcription factor can increase expression by at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, or more, compared to the coding sequence of a non-codon optimized artificial transcription factor.

Wild-type REST contains an N-terminal inhibitory domain, an intermediate DNA-binding domain that binds to DNA, and a C-terminal transcriptional repression domain.

In some embodiments, the artificial transcription factor comprises the RZFD core domain but does not comprise the sequences shown in SEQ ID NOs: 12 and 13. For example, the artificial transcription factor may comprise amino acid sequences 159-412 of REST but does not comprise the N-terminal and/or C-terminal repression domains of REST. The sequence shown in SEQ ID NO: 12 is the N-terminal amino acid sequence of REST, and the sequence shown in SEQ ID NO: 13 is the C-terminal amino acid sequence of REST.

The core domain may comprise any 5-8 sequences of SEQ ID NOs: 3-10. In some embodiments, the amino acid sequence of the RZFD core domain comprises any 6-8 sequences of SEQ ID NOs: 3-10. In some embodiments, the amino acid sequence of the RZFD core domain may comprise the sequence of any one of SEQ ID NOs: 2, 14, 74, 76, and 75. In some specific embodiments, the core domain may comprise an amino acid sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to the sequence shown in any one of SEQ ID NOs: 2, 14, 74, 76, and 75.

In some embodiments, the artificial transcription factor may comprise aRZFD core domain, an NLS, and/or an activation domain, or the artificial transcription factor may comprise a RZFD core domain, an NLS, and/or an epigenetic regulatory domain, or the artificial transcription factor may comprise a RZFD core domain, an NLS, an activation domain, and an epigenetic regulatory domain. In some specific embodiments, the artificial transcription factor may be designed as shown in Figure 1, wherein RZFD represents the RZFD core domain described herein, NLS represents an optional nuclear localization signal, TAD represents an optional transcription activation domain, and ERD represents an optional epigenetic regulatory domain.

In some embodiments, the RZFD core domain, NLS, TAD, or ERD can be connected using a suitable linker.

The artificial transcription factor can comprise the amino acid sequence of any one of SEQ ID NO: 2, 14, 30, 32, 34, 57, 59, 61, 63, 67, 77, 78, 79, 80, 81, 82, 84, 86, 90, or 92. The RZFD variant can comprise an amino acid sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to any one of SEQ ID NO: 2, 14, 30, 32, 34, 57, 59, 61, 63, 67, 77, 78, 79, 80, 81, 82, 84, 86, 90, or 92. The artificial transcription factor can be encoded by a nucleic acid sequence of any one of SEQ ID NOs: 15, 31, 33, 35, 56, 58, 60, 64, 68, 85, 87, 91, or 93. The artificial transcription factor can be encoded by a nucleic acid sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to any one of SEQ ID NOs: 15, 31, 33, 35, 56, 58, 60, 64, 68, 85, 87, 91, or 93.

### Nuclear Localization Signal

The artificial transcription factor may further include a nuclear localization signal (NLS). A nuclear localization signal is an amino acid sequence that can guide a protein into the nucleus via nuclear transport. The NLS may be located at the N-terminus of a protein (e.g., RZFD core domain). In addition, the NLS may be located at the C-terminus of a protein (e.g., RZFD core domain). The NLS may be located at the N-terminus of RZFD core domain. In addition, the NLS may be located at the C-terminus of RZFD core domain. In some embodiments, the NLS is located at both the N-terminus and the C-terminus of RZFD core domain.

The artificial transcription factor may comprise 1, 2, 3, 4, 5, 6, 7, 8, 9, 10 or more NLS sequences. The NLS may comprise the amino acid sequence of any one of SEQ ID NOs: 24 or 38-53. Optionally, the NLS may comprise an amino acid sequence that is at least about 70%, at least about 80%, at least about 85%, at least about 90%, at least about 92%, at least about 94%, at least about 96%, at least about 97%, at least about 98%, or at least about 99% identical to any one of SEQ ID NOs: 24 or 38-53. The NLS may be encoded by a nucleic acid sequence of SEQ ID NO: 25. Optionally, the NLS can be encoded by a nucleic acid sequence having at least about 70%, at least about 80%, at least about 85%, at least about 90%, at least about 92%, at least about 94%, at least about 96%, at least about 97%, at least about 98%, or at least about 99% identity to the sequence of SEQ ID NO:25.

The artificial transcription factor having NLS may comprise an amino acid sequence as shown in any one of SEQ ID NOs: 30, 32, 34, 57, 59, 67, or 61. Optionally, the artificial transcription factor having NLS may comprise an amino acid sequence that is at least about 70%, at least about 80%, at least about 85%, at least about 90%, at least about 92%, at least about 94%, at least about 96%, at least about 97%, at least about 98%, or at least about 99% identical to any one of SEQ ID NOs: 30, 32, 34, 57, 59, 67, or 61. The nucleic acid sequence encoding the artificial transcription factor having NLS may comprise a nucleic acid sequence as shown in any one of SEQ ID NOs: 31, 33, 35, 56, 58, 60, 68, or 64. Optionally, the nucleic acid sequence encoding the artificial transcription factor having NLS may comprise a nucleic acid sequence that is at least about 70%, at least about 80%, at least about 85%, at least about 90%, at least about 92%, at least about 94%, at least about 96%, at least about 97%, at least about 98% or at least about 99% identical to any one of SEQ ID NOs: 31, 33, 35, 56, 58, 60, 68, or 64.

The artificial transcription factor may further include a nuclear export signal (NES). A nuclear export signal is an amino acid sequence that marks a protein for export from the cell nucleus via nuclear transport. The NES may be fused to the N-terminus of a protein (e.g., a core domain). Optionally, the NES may be fused to the C-terminus of a protein (e.g., a core domain). A protein (e.g., an artificial transcription factor) may include 1, 2, 3, 4, 5, 6, 7, 8, 9, 10, or more NES sequences. The NES may include the amino acid sequence of any one of SEQ ID NOs: 54 or 55. Optionally, the NES may include an amino acid sequence that is at least about 70%, at least about 80%, at least about 85%, at least about 90%, at least about 92%, at least about 94%, at least about 96%, at least about 97%, at least about 98%, or at least about 99% identical to the sequence of any one of SEQ ID NOs: 54 or 55.

### Gene activation domain

In some embodiments, artificial transcription factor also includes a gene activation domain (in this application, also abbreviated as activation domain). Activation domain can include epigenetic modification protein or gene activation regulatory element, optionally, activation domain includes the activation domain of VP64, P65, HSF1, VP16, MyoD1, RTA, SET7/9, Suntag, P300, CBP or histone acetyltransferase or any combination thereof. In some embodiments, gene activation domain includes the activation domain of VP64, the activation domain of P65, the activation domain of RTA or the activation domain of HSF1, or any combination thereof.

In some embodiments, the activation domain may comprise multiple activation domains, for example, comprise the activation domain of VP64 and the activation domain of P65. In some embodiments, the activation domain further comprises the activation domain of VP64, the activation domain of P65, and the activation domain of HSF1. In some embodiments, the activation domain further comprises the activation domain of VP64, the activation domain of P65, and the activation domain of RTA.

The activation domain can be located at the N-terminus of the RZFD core domain. Alternatively, the activation domain can be located at the C-terminus of the RZFD core domain. Alternatively, the activation domain can be located at both the C-terminus and the N-terminus of the RZFD core domain.

In some embodiments, the NLS can be fused to one or more gene activation domains, such as about or more than about 1, 2, 3, 4, 5, 6, 7, 8, 9, 10 or more gene activation domains. In some embodiments, there are about 1, 2, 3, 4, 5, 6, 7, 8, 9, 10 or more gene activation domains at or near the N-terminus and/or C-terminus of the NLS.

In one embodiment, a gene activation domain is considered to be near the N-terminus or C-terminus of an NLS when it is located within about 1, 2, 3, 4, 5, 10, 15, 20, 25, 30, 40, 50, or more amino acids along the polypeptide chain from the N-terminus or C-terminus of the NLS.

The activation domain of VP64 can comprise the amino acid sequence of SEQ ID NO: 26. Alternatively, the activation domain of VP64 can comprise an amino acid sequence that is at least about 70%, at least about 80%, at least about 85%, at least about 90%, at least about 92%, at least about 94%, at least about 96%, at least about 97%, at least about 98%, or at least about 99% identical to the sequence of SEQ ID NO: 26. The activation domain of VP64 can be encoded by a nucleic acid sequence that comprises the sequence of SEQ ID NO: 27. The activation domain of VP64 can be encoded by a nucleic acid sequence that comprises a sequence that is at least about 70%, at least about 80%, at least about 85%, at least about 90%, at least about 92%, at least about 94%, at least about 96%, at least about 97%, at least about 98%, or at least about 99% identical to the sequence of SEQ ID NO: 27.

The activation domain of P65 comprises an amino acid sequence that is at least 70%, at least 80%, at least 85%, at least 90%, at least 90%, at least 91%, at least 92%, at least 93%, at least 94%, at least 95%, at least 96%, at least 97%, at least 98%, or at least 99% identical to SEQ ID NO: 69.

The activation domain of RTA comprises an amino acid sequence that is at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 11.

The activation domain of HSF1 comprises an amino acid sequence that is at least 70%, at least 80%, at least 85%, at least 90%, at least 90%, at least 91%, at least 92%, at least 93%, at least 94%, at least 95%, at least 96%, at least 97%, at least 98%, or at least 99% identical to SEQ ID NO:70.

The fusion form of several activation domains can include a fusion form of the activation domains of P65 and HSF1 (abbreviated herein as P65-HSF1 activation domain or P65-HSF1). The P65-HSF1 can comprise the amino acid sequence of SEQ ID NO: 28. Alternatively, the P65-HSF1 activation domain can comprise an amino acid sequence having at least about 70%, at least about 80%, at least about 85%, at least about 90%, at least about 92%, at least about 94%, at least about 96%, at least about 97%, at least about 98%, or at least about 99% identical to the sequence of SEQ ID NO: 28. The P65-HSF1 activation domain can be encoded by a nucleic acid sequence comprising the sequence of SEQ ID NO: 29. The P65-HSF1 activation domain can be encoded by a nucleic acid sequence comprising a sequence that is at least about 70%, at least about 80%, at least about 85%, at least about 90%, at least about 92%, at least about 94%, at least about 96%, at least about 97%, at least about 98%, or at least about 99% identical to the sequence of SEQ ID NO:29.

The fusion form of several activation domains can include a fusion form of the activation domain of VP64, the activation domain of P65, and the activation domain of RTA. In specific embodiments, the activation domain of the fusion form can comprise an amino acid sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 72.

The fusion form of several activation domains can include a fusion form of the activation domain of VP64, the activation domain of P65, and the activation domain of HSF1. In specific embodiments, the activation domain of the fusion form can comprise an amino acid sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 73.

The fusion form of several activation domains can include a fusion form of the activation domain of P65 and the activation domain of RTA. In specific embodiments, the activation domain of the fusion form can comprise an amino acid sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 71.

### Construction direction of artificial transcription factors

In some embodiments, the artificial transcription factor comprises RZFD core domain, NLS, and gene activation domain.

The connection of RZFD core domain, NLS, and gene activation domain from N-terminus to C-terminus can be optional.

In some specific embodiments, the artificial transcription factor may include an NLS and a gene activation domain located at the N-terminus of the RZFD core domain. Alternatively, the artificial transcription factor may include an NLS and a gene activation domain located at the C-terminus of the RZFD core domain. Alternatively, the artificial transcription factor may include an NLS located at the N-terminus of the RZFD core domain and a gene activation domain located at the C-terminus of the RZFD core domain. Alternatively, the artificial transcription factor may include an NLS located at the C-terminus of the RZFD core domain and a gene activation domain located at the N-terminus of the RZFD core domain.

In some specific embodiments, the NLS may be at the N-terminus of the gene activation domain. Alternatively, the NLS may be at the C-terminus of the gene activation domain. The NLS may be adjacent to the gene activation domain. Alternatively, the NLS may not be adjacent to the gene activation domain. The artificial transcription factor may comprise 1, 2, 3, 4, 5 or more NLS domains and 1, 2, 3, 4, 5 or more gene activation domains. In some embodiments, the artificial transcription factor may comprise at least two NLS domains and at least one gene activation domain. In some embodiments, the artificial transcription factor may comprise at least two NLS domains and at least two gene activation domains.

### Linker

In some embodiments of the present invention, when any two of the nuclear localization signal, core domain, gene activation domain, enhancer, or WPRE are fused to each other, they can be connected by a linker, also called a connector.

The term "linker" refers to a short peptide or its coding sequence that connects different polypeptide sequences or amino acid sequences. In some embodiments, the short peptide can be a short peptide containing2, 3, 4, 5, 6, 7, 8, 9 or 10 amino acids, or a short peptide containing more amino acids, such as 11-15. Typically, such short sequences do not have special biological activity except for connecting protein fragments or nucleic acid fragments or maintaining a certain minimal distances or other spatial relationships between protein fragments or nucleic acid fragments. However, in some embodiments, the linker can be selected to influence certain properties of the linker and/or fusion molecule, such as the folding, net charge or hydrophobicity of the linker.

Suitable linkers for use in the methods of the present invention are well known to those skilled in the art and include, but are not limited to, linear or branched carbon linkers, heterocyclic carbon linkers, or peptide linkers. The linker can also be a covalent bond (carbon-carbon bond or carbon-heteroatom bond).

### Expression vector

The nucleic acid encoding the artificial transcription factor can be located on an expression vector. The expression vector can comprise a nucleic acid sequence encoding the artificial transcription factor, wherein the artificial transcription factor comprises a core domain.

An expression vector, also referred to as an expression construct, can be a bacterial plasmid or a viral vector designed for genetic expression in a cell. In some embodiments, the viral vector can comprise a recombinant adeno-associated viral vector (rAAV), an adeno-associated viral (AAV) vector, an adenoviral vector, a lentiviral vector, a retroviral vector, a poxvirus vector, a herpes virus, an SV40 viral vector, or any combination thereof.

In some specific embodiments, the adeno-associated virus (AAV) vector can be selected from AAV1, AAV2, AAV5, AAV6, AAV8, AAV9, AAV10, AAVrh, AAV-DJ, AAV-PHP.B, AAV-PHP.S and AAV-PHP.eB, or a mutant thereof.

The expression vector may further comprise a regulatory element. The regulatory element is a nucleic acid region that regulates the transcription (e.g., expression or translation rate) of the associated gene. The regulatory element may be a protein transcription start site, a promoter, an enhancer, an inhibitor, or a post-transcriptional regulatory element.

The expression vector may further comprise a protein translation initiation site (e.g., a Kozak sequence). The Kozak sequence may comprise the sequence of SEQ ID NO: 23. The Kozak sequence may comprise a sequence having at least about 70%, at least about 80%, at least about 90%, at least about 91%, at least about 92%, at least about 93%, at least about 94%, at least about 95%, at least about 96%, at least about 97%, at least about 98%, or at least about 99% sequence identity to SEQ ID NO: 23. In SEQ ID NO: 23, R is a purine (A or G).

### Regulatory elements

Regulatory elements are nucleic acid regions that modulate the transcription (e.g., expression or translation rate) of an associated gene (e.g., an artificial transcription factor).

In some embodiments, the regulatory element includes a promoter that regulates the nucleic acid expression of the associated gene. Preferably, the promoter comprises a glial cell-specific promoter.

In some embodiments, the regulatory element comprises an enhancer that enhances gene expression. Preferably, the enhancer comprises a CMV enhancer, an SV40 intronic enhancer, an MVM intronic enhancer, a β-globin intronic enhancer, or a synthetic intronic enhancer, or any combination thereof.

In some embodiments, the regulatory element comprises a Woodchuck Hepatitis Virus Posttranscriptional Regulatory Element (WPRE).

In some embodiments, the regulatory element comprises an enhancer and a woodchuck hepatitis virus posttranscriptional regulatory element (WPRE).

### Promoter

Regulatory elements include promoters that regulate the nucleic acid expression of the associated genes.

A promoter is a cis-acting regulatory element, which is a nucleic acid region capable of initiating the transcription of a downstream associated nucleic acid sequence. A promoter can be tissue-specific (e.g., specific to a particular cell type). Non-limiting examples of promoters can be stemcell promoters, osteocyte promoters, blood cell promoters, and neuronal promoters, muscle cell promoters, or sperm cell promoters.

A promoter is one that helps to drive the expression of a specific gene. Non-limiting examples of promoters include photoreceptor promoters, cholinergic neuron promoters, adrenergic neuron promoters, peptidergic neuron promoters, glial cell promoters (e.g., astrocyte-specific promoters or Müller glial cell promoters), Schwann cell promoters, Purkinje cell promoters, and medium spiny neuron promoters.

In some embodiments, the glial cell-specific promoter may include an astrocyte-specific promoter. Non-limiting examples of glial cell-specific promoters may include GFAP promoter, ALDHI1L1 promoter, EAAT1/GLAST promoter, glutamine synthetase promoter, S100β promoter, EAAT2/GLT-1 promoter or Rlbp1 promoter. Glial cell-specific promoters may include a promoter specific for Müller glial (MG) cells. Glial cell-specific promoters may include cochlear glial cell-specific promoters. Non-limiting examples of cochlear glial cell-specific promoters may include GFAP promoter, ALDH1L1 promoter, EAAT1/GLAST promoter or Plp1 promoter.

In some embodiments, the glial cell-specific promoter may include the GFAP promoter. In some embodiments, the cochlear glial cell-specific promoter may include the GFAP promoter. The GFAP promoter may include the nucleotide sequence shown in SEQ ID NO: 65 or 66. The GFAP promoter may include a nucleotide sequence having at least about 70%, 80%, 85%, 90%, 95%, 99% or more sequence identity to SEQ ID NO: 65 or 66.

### Enhancer

The expression vector may further comprise an enhancer. The enhancer is a cis-regulatory element, which is a nucleic acid region capable of binding a protein to increase the likelihood of transcription of an associated nucleic acid sequence. Non-limiting examples of enhancers include the CMV enhancer, the SV40 intronic enhancer, the MVM intronic enhancer, the β-globin intronic enhancer, or a synthetic intronic enhancer, or any combination thereof.

In the technical solution of the present application, one enhancer may be included, or multiple enhancers may be included, for example, 2, 3, 4, 5, 6, 7, 8, 9, or 10 enhancers may be included.

The enhancer can be a CMV enhancer. For example, the CMV enhancer can comprise the sequence of SEQ ID NO: 16. The CMV enhancer can comprise a sequence that has at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to SEQ ID NO: 16.

The enhancer can be an SV40 intronic enhancer. For example, the SV40 intronic enhancer can comprise the sequence of SEQ ID NO: 17. The SV40 intronic enhancer can comprise a sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to SEQ ID NO: 17.

The enhancer can be an MVM intronic enhancer. For example, the MVM intronic enhancer can comprise the sequence of SEQ ID NO: 18. The MVM intronic enhancer can comprise a sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to SEQ ID NO: 18.

The enhancer can be a β-globin intronic enhancer. For example, the β-globin intronic enhancer can comprise the sequence of SEQ ID NO: 19. The β-globin intronic enhancer can comprise a sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to SEQ ID NO: 19.

The enhancer can be a synthetic intronic enhancer. For example, the synthetic intronic enhancer can comprise the sequence of any one of SEQ ID NOs: 20-22. The synthetic intronic enhancer can comprise a sequence having at least about 70%, at least about 80%, at least about 85%, at least about 90%, at least about 91%, at least about 92%, at least about 93%, at least about 94%, at least about 95%, at least about 96%, at least about 97%, at least about 98%, or at least about 99% sequence identity to any one of SEQ ID NOs: 20-22.

### Transdifferentiation

Described herein are methods for differentiating non-neuronal cells into functional neurons by administering an expression vector or composition described herein to modulate the binding of endogenous REST to RE1/NRSE.

In some embodiments, the non-neuronal cells can be terminally differentiated cells, progenitor cells, stem cells, and the like.

In some embodiments, the expression vectors or compositions described herein can be used to target the RE1 sequence, thereby blocking the binding of endogenous REST to RE1 by competitively binding to RE1 against endogenous REST.

In some embodiments, the expression vectors or compositions can be adeno-associated virus (AAV) vectors or lentiviral vectors carrying artificial transcription factor. Optional AAV vectors include, but are not limited to, AAV2, AAV3, AAV4, AAV5, AAV6, AAV7, AAV8, AAV9, AAV 10, or mutants thereof.

In non-neuronal cells (e.g., glial cells), the expression of the RZFD core domain can reduce the suppression of the REST silencing complex on the expression of neuron-related genes. In some embodiments, the activation domain such as activation domain of VP64, P65, HSF1, Rta, etc. is fused to the RZFD core domain, which can further promote the expression of neuron-related genes, and promote the transdifferentiation of glial cells to neurons. In some embodiments, NLS such as bpNLS is fused to the RZFD core domain, which can further promote the expression of neuron-related genes, and promote the transdifferentiation of glial cells to neurons. In some embodiments, NLS, activation domain, and RZFD core domain is fused, which can further promote the expression of neuron-related genes, and promote the transdifferentiation of glial cells to neurons.

In some embodiments, the NLS, activation domain, and RZFD core domain can be connected in the manner shown in Figure 1, and the RZFD core domain, NLS, TAD, or ERD can be connected using a linker.

In some embodiments, expression vectors or compositions described herein can transdifferentiate glial cells particularly astrocytes into dopamine neurons. In some embodiments, the designs of the artificial transcription factor of expression vectors or compositions described herein can be as shown in Fig. 2B. Expression vectors or compositions using RZFD-V1, RZFD-V2, RZFD-V3, RZFD-V4, RZFD-V5, RZFD-V6, RZFD-V7 or RZFD-V8 as the artificial transcription factors can transdifferentiate glial cells into dopamine neurons. In a specific embodiment, expression vectors or compositions can be AAV vectors. In some embodiments, the detection of dopamine neurons can be detected by immunofluorescence staining. In some embodiments, astrocytes can be transdifferentiated into dopamine neurons by injecting AAV vectors carrying artificial transcription factors into the brain (e.g., into the striatum or substantia nigra).

In some embodiments, the expression vectors or compositions described herein can transdifferentiate Miiller cells into retinal ganglion cells or photoreceptor cells. For example, injecting AAV vectors carrying the artificial transcription factors into the subretinal or vitreous cavity can transdifferentiate Miiller cells into retinal ganglion cells or photoreceptor cells. Retinal ganglion cells are the only cells in the visual pathway that transmit visual signals to the brain, and their loss or death can lead to permanent blindness.

Non-neuronal cells can include glial cells, fibroblasts, stem cells, neural precursor cells, or neural stem cells. In some embodiments, non-neuronal cells are glial cells. In some embodiments, glial cells are selected from astrocytes, oligodendrocytes, ependymal cells, Schwann cells, NG2 cells, satellite cells, Müller glial cells, inner ear glial cells, and any combination thereof.

In some embodiments, the glial cells are located in the brain, spinal cord, eye, or ear. In some embodiments, the glial cells are located in the striatum, substantia nigra, ventral tegmental area of the midbrain, medulla oblongata, hypothalamus, dorsal midbrain, or cerebral cortex of the brain.

In some embodiments, glial cells include astrocytes and functional neurons include dopamine neurons. In some embodiments, the methods provided herein relate to methods for transdifferentiating astrocytes into dopamine neuronal cells in a subject. In some embodiments, astrocytes are located in the striatum and/or substantia nigra. In some embodiments, the methods include administering an active substance provided herein to the striatum and/or substantia nigra of a subject.

In some embodiments, glial cells include Müller cells, and functional neuronal cells include retinal ganglion cells (RGCs) and/or photoreceptor cells. In some embodiments, provided herein are methods for transdifferentiating Müller cells into retinal ganglion cells (RGCs) and/or photoreceptor cells in a subject. In some embodiments, Müller cells are located below the retina or in the vitreous cavity. In some embodiments, the methods include administering an active substance provided herein to the subretinal or vitreous cavity of a subject.

In some embodiments, the glial cells include cochlear glial cells and the functional neuronal cells include cochlear spiral ganglion cells. In some embodiments, the methods provided herein relate to methods for transdifferentiating cochlear glial cells into cochlear spiral ganglion cells in a subject. In some embodiments, the cochlear glial cells are located in the inner ear. In some embodiments, the methods comprise administering an active agent provided herein to the inner ear of a subject.

Functional neurons can refer to neuronal cells with specific functions, such as dopamine neurons, retinal ganglion cells, photoreceptor cells, and other neurons with specific functions. In some embodiments, functional neurons have at least one morphological feature of neurons (e.g., synapses, axons). In some embodiments, functional neurons include axons. In some embodiments, functional neurons express at least one marker of mature neurons, such as NeuN gene expression products. In some embodiments, functional neurons have electrophysiological properties.

In some embodiments, the functional neurons express the NeuN gene. The NeuN gene is a known specific marker for mature neurons. Detection of a NeuN gene expression product (such as NeuN protein) in non-neuronal cells indicates that the non-neuronal cells have transdifferentiated into functional neurons.

Functional neurons can have different functions. In some embodiments, functional neurons include dopamine neurons, ganglion cells, retinal ganglion cells, photoreceptor cells and cochlear spiral ganglion cells, GABA neurons, 5-HT neurons, glutamatergic neurons, ChAT neurons, NE neurons, motor neurons, spinal neurons, spinal motor neurons, pyramidal neurons, interneurons, medium spiny neurons (MSN), Purkinje cells, granule cells, olfactory sensory neurons, periglomerular cells or any combination thereof.

In some embodiments, functional neurons include dopamine neurons. Dopamine neurons are neurons that contain and release dopamine (DA) as a neurotransmitter. Dopamine neurons are the main source of dopamine in the central nervous system. Dopamine is a catecholamine neurotransmitter that can influence neural functions such as emotions and rewards, and plays an important biological role in the central nervous system. The dopamine neurons in the brain are mainly concentrated in the substantia nigra pars compacta (SNc), ventral tegmental area (VTA), hypothalamus and periventricular area of the midbrain. Dopamine neurons may be related to various diseases of the human, the most typical of which is Parkinson's disease. The gradual loss of dopamine neurons can trigger many motor symptoms associated with Parkinson's disease.

In some embodiments, dopamine neurons may express one or more markers selected from NeuN, tyrosine hydroxylase (TH), FoxA2, Nurr1, Pitx3, Vmat2, and DAT. Marker may refer to gene expression product, such as mRNA or protein. Detection of the expression of one or more markers in functional neuronal cells indicate that the functional neurons are dopamine neurons. In some embodiments, dopamine neurons express TH. TH is an enzyme responsible for catalyzing the conversion of the amino acid L-tyrosine to dihydroxyphenylalanine (DOPA), and is also an enzyme participated in the synthesis and metabolism of dopamine in dopamine neurons. In some embodiments, dopamine neurons express NeuN, TH, and DAT.

In some embodiments, the functional neurons include retinal ganglion cells. Retinal ganglion cells are neurons located near the inner surface of the retina (ganglion cell layer) that receive visual information from photoreceptors through two types of interneurons (bipolar cells and amacrine cells). Their dendrites primarily establish synaptic connections with bipolar cells, and their axons extend to the optic disc to form the optic nerve, which then extends to the brain.

In some embodiments, retinal ganglion cells (RGCs) express one or more markers selected from RBPMS, Pax6, Brn3a, Brn3b, Brn3c, and Map2. RBPMS is a specific marker for RGCs. If the expression of RBPMS is detected in a functional neuronal cell, it indicates that the functional neuronal cell is an RGC. In some embodiments, retinal ganglion cells (RGCs) express NeuN and RBPMS.

In some embodiments, functional neurons include photoreceptor cells. Photoreceptor cells are specialized neuroepithelial cells found in the retina that have the function of perceiving light and perform phototransduction. They can be processed by bipolar cells and ganglion cells, converting light signals into electrical signals and transmitting to the brain. Photoreceptor cells include rods and cones.

In some embodiments, the photoreceptor cells express one or more markers selected from Rhodopsin, mCAR, M-opsin, and S-opsin. Rhodopsin, mCAR, M-opsin, and S-opsin are all specific markers for photoreceptor cells. If the expression of Rhodopsin, mCAR, M-opsin, and/or S-opsin is detected in functional neuronal cells, it indicates that the functional neurons are photoreceptor cells. In some embodiments, the photoreceptor cells express NeuN, Rhodopsin, and/or mCAR.

In some embodiments, the functional neurons include cochlear spiral ganglion cells. Cochlear spiral ganglion cells are bipolar ganglion cells and serve as primary neurons in the auditory pathway. Their peripheral processes connect to hair cells, while their intermediate processes contribute to the formation of the auditory nerve. Spiral ganglion cells play an important role in the transmission and encoding of sound signals.

In some embodiments, the cochlear spiral ganglion cells express one or more markers selected from NeuN, Prox1, Tuj-1, and Map2. Detecting the expression of Prox1 and Map2 in functional neuronal cells indicates that the functional neuronal cells are cochlear spiral ganglion cells. In some embodiments, the cochlear spiral ganglion cells express NeuN, Prox1, Tuj-1, and Map2.

In some embodiments, transdifferentiation is achieved by administering the fusion protein and/or the expression vector described herein to the glial cells. Administration to glial cells can occur locally at one or more of the following locations in a subject: 1) glial cells in the striatum; ii) in the substantia nigra of the brain, iii) glial cells in the retina; iv) glial cells in the inner ear; v) glial cells in the spinal cord; vi) glial cells in the prefrontal cortex; vii) glial cells in the motor cortex; viii) glial cells in the hypothalamus; and ix) glial cells in the ventral tegmental area (VTA).

In some embodiments, the efficiency of transdifferentiation from non-neuronal cells to functional neurons is at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, or more. In some embodiments, the efficiency of transdifferentiation from glial cells to functional neurons is at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100% or more.

The transdifferentiation efficiency can be detected and calculated by methods known to those skilled in the art. For example, fluorescent proteins can be used to lable the initial cells (such as glial cells) of transdifferentiation, such as GFAP-mCherry, GFAP-tdTomato, GFAP-EGFP etc. For example, Ai9 transgenic mice with fluorescently labeled glial cells can also be used. Since the transdifferentiated cells also carry fluorescence, the transdifferentiation efficiency can be calculated by calculating the percentage of the number of the transdifferentiated cells and the number of initially labeled cells. Alternatively, the transdifferentiation efficiency can also be calculated as the percentage of the number of cells produced by transdifferentiation compared to the number of cells of this type at the administration site, for example, in the substantia nigra, the percentage of newly generated dopamine neurons and dopamine neurons in the substantia nigra.

### Active substances

In this article, the active substance refers to a substance containing the expression vector or composition provided by the present application. In some embodiments, the active substance may further contain a pharmaceutically acceptable carrier.

**In** some specific embodiments, the expression vector described herein comprises: (a) a nucleic acid sequence encoding an artificial transcription factor capable of binding to RE1, the artificial transcription factor comprises RZFD core domain; and (b) a regulatory element for regulating expression of the artificial transcription factor nucleic acid; wherein the RZFD core domain comprises any 5-8 sequences of SEQ ID NOs: 3-10, and does not comprise the sequences shown in SEQ ID NOs: 12 and 13. In some embodiments, the amino acid sequence of the RZFD core domain comprises any 6-8 sequences of SEQ ID NOs: 3-10. In some embodiments, the amino acid sequence of the RZFD core domain may comprise the sequence shown in any one of SEQ ID NOs: 2, 14, 74, 76, and 75. In some embodiments, the RZFD core domain comprises a sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 2, 14, 74, 76, or 75. In some embodiments, the RZFD core domain comprises a sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 14. In some embodiments, the nucleic acid sequence encoding the RZFD core domain comprises a sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 15.

In some embodiments, the artificial transcription factor of the expression vector comprises RZFD core domain, NLS, and/or gene activation domain. In some embodiments, the NLS may be one or more. In some embodiments, the gene activation domain may be one or more.

In some embodiments, the regulatory element comprises a promoter that regulates the expression of nucleic acid encoding the artificial transcription factor. In some embodiments, the regulatory element of the expression vector comprises a promoter to regulate the expression of nucleic acid encoding the artificial transcription factor, as well as an enhancer to increase the transcription of the artificial transcription factor; or the regulatory element of the expression vector comprises a promoter to regulate the expression of nucleic acid encoding the artificial transcription factor and a woodchuck hepatitis virus post-transcriptional enhancer element (WPRE); or the regulatory element of the expression vector comprises a promoter to regulate the expression of nucleic acid encoding the artificial transcription factor nucleic acid, an enhancer to increase the transcription of the artificial transcription factor, and a woodchuck hepatitis virus post-transcriptional enhancer element (WPRE).

In some embodiments, the composition described herein comprises: (a) an artificial transcription factor capable of binding to RE1, comprising: RZFD core domain, a first NLS, and a first gene activation domain; or (b) a nucleic acid encoding the artificial transcription factor described in (a), wherein the RZFD core domain comprises any 5-8 sequences of SEQ ID NOs: 3-10 and does not comprise the sequences shown in SEQ ID NOs: 12 and 13. In some embodiments, the amino acid sequence of the RZFD core domain comprises any 6-8 sequences of SEQ ID NOs: 3-10. In some embodiments, the amino acid sequence of the RZFD core domain may comprise the sequence shown in any one of SEQ ID NOs: 2, 14, 74, 76, and 75. In some embodiments, the RZFD core domain comprises a sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 2, 14, 74, 76, or 75. In some embodiments, the nucleic acid sequence encoding the RZFD core domain comprises a sequence having at least 70%, at least 80%, at least 85%, at least 90%, at least 91%, at least 92%, at least 93%, at least 94%, at least 95%, at least 96%, at least 97%, at least 98%, or at least 99% identical to SEQ ID NO: 15.

In some embodiments, the artificial transcription factor of the composition further comprises a second NLS. In some embodiments, the artificial transcription factor of the composition further comprises a second gene activation domain. In some embodiments, the artificial transcription factor of the composition further comprises a second NLS and a second gene activation domain.

In some embodiments, the composition further comprises a regulatory element to regulate the expression of nucleic acid encoding the artificial transcription factor. In some embodiments, the regulatory element comprises a promoter for regulating the nucleic acid expression of the artificial transcription factor. In some embodiments, the regulatory element comprises a promoter for regulating the nucleic acid expression of the artificial transcription factor and an enhancer for increasing the transcription of the artificial transcription factor; or the regulatory element of the expression vector comprises a promoter for regulating the nucleic acid expression of the artificial transcription factor and a woodchuck hepatitis virus post-transcriptional enhancer element (WPRE); or the regulatory element of the expression vector comprises a promoter for regulating the nucleic acid expression of the artificial transcription factor, an enhancer for increasing the transcription of the artificial transcription factor, and a woodchuck hepatitis virus post-transcriptional enhancer element (WPRE).

### Disease treatment

In some embodiments, the administration of the expression vectors or compositions described herein can be used to prevent and/or treat diseases associated with loss of neuronal function or neuronal death in a subject in need. In some embodiments, the neurons obtained by transdifferentiation according to the present application can be used to prevent and/or treat diseases associated with loss of neuronal function or neuronal death in a subject in need.

The disease or disorder associated with loss of neuronal function or neuronal death can be a disease associated with loss of function or death of dopamine neurons, or a disease associated with visual impairment caused by loss or death of retinal ganglion or photoreceptor cells. In some embodiments, the disease associated with loss of neuronal function or neuronal death is selected from the group consisting of Parkinson's disease, Alzheimer's disease, stroke, schizophrenia, Huntington's disease, depression, motor neuron disease, amyotrophic lateral sclerosis, spinal muscular atrophy, Pick's disease, sleep disorders, epilepsy, ataxia, glaucoma, age-related RGC lesions, optic nerve damage, retinal ischemia or hemorrhage, Leber's hereditary optic neuropathy, photoreceptor cell degeneration or death due to injury or degeneration, macular degeneration, retinitis pigmentosa, diabetes-related blindness, night blindness, color blindness, hereditary blindness, congenital amaurosis, spiral neural deafness or hearing loss caused by ganglion cell death.

In some embodiments, the application provides a method for preventing and/or treating a disease associated with loss of function or death of neurons in a subject in need, including administering a therapeutically effective amount of the expression vector or composition described herein into the subretinal or vitreous body of the subject. The expression vector provided herein can transdifferentiate Miiller cells in the subretinal or vitreous cavity into retinal ganglion cells (RGC) and/or photoreceptor cells, wherein the diseases associated with loss of function or death of neurons are selected from: RGC cell death caused by optic nerve disorders, glaucoma, age-related RGC damage, optic nerve damage, retinal ischemia or hemorrhage, Leber's hereditary optic neuropathy, photoreceptor cell degeneration or death caused by damage or degeneration, macular degeneration, retinitis pigmentosa, diabetic related blindness, night blindness, color blindness, hereditary blindness and amaurosis.

The compositions described herein (also referred as pharmaceutical compositions) may comprise the expression vector described herein. The pharmaceutical composition may further comprise a carrier, an excipient, a binder, a filler, a suspending agent, a flavoring agent, a sweetener, a disintegrant, a dispersant, a surfactant, a lubricant, a colorant, a diluent, a solubilizer, a plasticizer, a stabilizer, a penetration enhancer, a wetting agent, a defoaming agent, an antioxidant, a preservative, or a combination thereof. The pharmaceutical composition may further comprise a carrier for delivering the polynucleotide, wherein the vector comprises viral vector, liposome, nanoparticle, exosome, or viral particle.

Administration of the pharmaceutical composition may include local, parenteral, intravenous, intradermal, intramuscular, or intraperitoneal administration. In some embodiments, the pharmaceutical composition is suitable for local administration into glial cells at one or more of the following sites: 1) glial cells in the striatum; ii) glial cells in the substantia nigra of the brain; iii) glial cells in the retina; iv) glial cells in the inner ear; v) glial cells in the spinal cord; vi) glial cells in the prefrontal cortex; vii) glial cells in the motor cortex; viii) glial cells in the hypothalamus; and ix) glial cells in the ventral tegmental area (VTA). In some embodiments, the pharmaceutical composition is suitable for intracranial or intraocular administration.

In some embodiments, the pharmaceutical composition further comprises i) one or more dopaminergic neuron-related factors, or ii) one or more retinal ganglion cell-related factors. The one or more dopaminergic neuron-related factors may be selected from FoxA2, Lmxla, Lmx1b, Nurrl, Pbx1a, Pitx3, Gata2, Gata3, FGF8, BMP, En1, En2, PET1, Pax family proteins (Pax3, Pax6, etc.), SHH, Wnt family proteins, TGF-β family proteins, or any combination thereof. The one or more retinal ganglion cell-related factors may be selected from β-catenin, Oct4, Sox2, Klf4, Crx, CamKII, Brn3a, Brn3b, Brn3C, Math5, Otx2, Ngn2, Ngn1, AscL1, miRNA9, miRNA-124, Nr2e3, Nrl, or any combination thereof.

In some embodiments, the present application provides a method for preventing and/or treating a disease associated with loss of function or death of neurons in a subject in need, which comprises administering a therapeutically effective amount of the active substance of the composition of the present application to the inner ear of the subject, for transdifferentiating the cochlear glial cells in inner ear into cochlear spiral ganglion cells, wherein the disease associated with loss of neuronal function or neuronal death is selected from deafness caused by spiral ganglion cell death or hearing loss.

In some embodiments, the present application provides a method for preventing and/or treating a disease associated with loss of function or death of neurons in a subject in need, which comprises administering a therapeutically effective amount of the active substance provided herein to the striatum and/or substantia nigra of the subject, for transdifferentiating astrocytes in the striatum and/or substantia nigra into dopamine neurons, wherein the disease associated with loss of neuronal function or neuronal death is selected from Parkinson's disease, depression, and Alzheimer's disease.

The methods for preventing and/or treating neuronal diseases described herein may comprise replacing non-neuronal cells, transdifferentiating the non-neuronal cells into functional neurons in vitro, and administering the functional neurons to a subject in need. Alternatively, the methods for preventing and/or treating neuronal diseases as described herein may comprise transdifferentiating non-neuronal cells into functional neurons in vivo. Alternatively, the methods for preventing and/or treating neuronal diseases as described herein may comprise administering therapeutically effective amount of nucleic acid, fusion protein, and/or expression vector as described herein that is capable of reducing the binding of REST to the RE1/NRSE element, or reducing the amount or activity of REST, thereby transdifferentiating non-neuronal cells into functional neurons at the site affected by the disease.

The subject (also referred to as an individual) can be a mammal, such as, but not limited to, non-human primates such as chimpanzees, and other apes and monkey species; farm animals such as cattle, horses, sheep, goats, pigs; domestic animals such as rabbits, dogs, and cats; laboratory animals, including rodents such as rats, mice, and guinea pigs. The mammal can be a human. The subject may be diagnosed or suspected of being at high risk for a disease. In some cases, the subject is not necessarily diagnosed or suspected of being at high risk for the disease. In some embodiments, the subject has one or more symptoms that indicate a disease or condition associated with a neurological disease or disorder.

Some experimental means and methods in this study can be implemented by conventional operations of those skilled in the art, for example:

Plasmid construction: The AAV backbone vector is digested with restriction endonucleases and recovered by agarose gel electrophoresis. PCR is performed using the cell cDNA as a template, and the PCR fragments are recovered after agarose gel electrophoresis. The backbone vector and fragment are ligated using the ClonExpress MultiS One-Step Cloning Kit (Vazyme, C113-02) from Vazyme Biotech. After ligation, the vector is transformed into DH5α Escherichia coli and plated. The next day, single colonies are identified and the positive clones are sequenced. Completely correct clones are expanded and plasmids extract.

AAV injection into mouse brain: Stereotaxic injection is performed using the RWD stereotaxic injection system (C57BL/6 or Dat-Cre:Ai9 mice, aged more than two months). The titer is approximately 5×10 ¹² vg/ml(1-3µl per injection). The AAVs are injected into the striatum (AP +0.8 mm, ML ±1.6 mm, and DV -2.8 mm) or substantia nigra (AP -3.0 mm, ML ±1.25 mm, and DV -4.5 mm).

Immunofluorescence staining of mouse tissues: After AAV injection, the tissues are collected, sliced and immunofluorescence staining. After perfusing with normal saline and 4% PFA, the brain of the mouse is removed and fixed with 4% paraformaldehyde (PFA) overnight, then dehydrated in 30% sucrose for at least 12 hours until the tissue sinks to the bottom of the solution. After embedding in OCT, frozen sections are made with a thickness of 30µm or 40µm. Before immunofluorescence staining, the brain slices are washed three times with 0.1M phosphate buffered saline (PBS) for 5-10 minutes each time. After incubation with the primary antibody at 4°C overnight, the slices are washed 3-4 times with PBS for 10-15 minutes each time. Then, the secondary antibody diluted in antibody diluent is added for incubation at room temperature for 2-3 hours. After the incubation, the slices are washed 3-4 times with PBS for 10-15 minutes each time. Finally, the slices are sealed and preserved with anti-fluorescence quenching mounting medium (Life Technology).

6-OHDA PD mouse model: For example, adult C57BL/6 mice (7-10 weeks) are used. Mice are intraperitoneally injected with 25 mg/kg desipramine hydrochloride (D3900, Sigma-Aldrich) 30 minutes before anesthesia. After anesthesia, 3 µg of 6-OHDA (H116, Sigma-Aldrich) or normal saline is injected into the right medial forebrain bundle of the mice: anteroposterior (A/P) = -1.2 mm, mediolateral (M/L) = -1.1 mm, and dorsoventral (D/V) = -5 mm. One hour after surgery, mice are subcutaneously injected with 1 ml of 4% glucose-saline solution.

### Examples

The following examples are only used to illustrate the technical solutions of the present invention and are not intended to limit the scope of the present invention.

### Example 1: Comparison of in vivo transdifferentiation effects of different versions of artificial transcription factors (ATF)

Due to the significant differences between in vivo and in vitro environments, many findings from in vitro studies are ineffective in vivo. To investigate whether different artificial transcription factors can achieve transdifferentiation of glial cells into neurons in vivo, this study constructed various versions of artificial transcription factors (Fig. 1A). Among them, dRT-V1, dRT-V2, and dRT-V3 have different transcriptional activation domains at their termini to enhance their transcriptional activity, with dRT-V3 lacking VP64. dRT-V1 utilizes amino acids at the site of 85-1008 of REST (amino acid sequence shown in SEQ ID NO:88, nucleotide sequence shown in SEQ ID NO:94), with the activation domain of VP64 fused to its the C-terminus. dRT-V2 utilizes amino acids at the site of 73-508 of REST (amino acid sequence shown in SEQ ID NO:89, nucleotide sequence shown in SEQ ID NO:96), with the activation domain of VP64 fused to its the C-terminus. In this study, the astrocyte-specific promoter GFAP is used to drive the expression of the gene of interest (GOI). The GOI is an artificial transcription factor (ATF). AAV-GFAP-mCherry fluorescent protein (design-1) is used in the control group (Fig. 1B). AAVs from each group are injected into the striatum of 6-OHDA-induced C57 mice. Samples are collected and analyzed 1.5-2 months after AAV injection. The results show that the red fluorescent-labeled cells in the AAV-GFAP-mCherry control group still maintain typical astrocyte morphology with small cell bodies and numerous thick cellular processes and branches (Fig. 1C). In the groups injected with design-2 (AAV-GFAP-dRT-V1) and design-3 (AAV-GFAP-dRT-V2), most red fluorescent-labeled cells still maintain typical astrocyte morphology with small cell bodies, some of the cells appear round in shape wih their cellular processes becoming slender and elongated. When stained with the neuron-specific protein marker NeuN, these cells are NeuN-negative, and only about 5% of the red fluorescent-labeled cells expresses NeuN. These results indicate that AAV-GFAP-dRT-V1 and AAV-GFAP-dRT-V2 have very weak ability to convert astrocytes into neurons in vivo. In the groups injected with design-4 (AAV-GFAP-dRT-V3) and design-5 (AAV-GFAP-dRT-V4), a large number of positive cells co-labeled with mCherry and NeuN are observed, indicating that design-4 (AAV-GFAP-dRT-V3) and design-5 (AAV-GFAP-dRT-V4) can efficiently transdifferentiate astrocytes into neurons.

To investigate whether dRT-V1, dRT-V2, dRT-V3, and dRT-V4 could transdifferentiate glial cells into dopamine neurons, we further stain sections from each group with dopamine neuron-specific protein markers. We find no TH-positive cells in the AAV-GFAP-mCherry control group, AAV-GFAP-dRT-V1, and AAV-GFAP-dRT-V2. However, TH-positive cells are present in the cells injected with design-4 (AAV-GFAP-dRT-V3) and design-5 (AAV-GFAP-dRT-V4). Furthermore, the number of TH-positive cells in the design-5 (AAV-GFAP-dRT-V4) group is approximately twice that of the design-4 (AAV-GFAP-dRT-V3) group. These results indicate that: (1) in vitro research results are likely ineffective in the complex environment of the body; (2) NLS may be an important factor in improving neuronal transdifferentiation; (3) the VP64 activation domain can significantly improve the efficiency of glial cell transdifferentiation into neurons and also significantly improve the production efficiency of dopamine neuron.

### Example 2: Designs of artificial transcription factor and expression vector based on RZFD

The results in Example 1 show that the RZFD domain can be designed as an artificial transcription factor, which can achieve efficient transdifferentiation of astrocytes into neurons or dopamine neurons in vivo. In order to further improve and optimize the design of artificial transcription factors (ATFs) based on RZFD, RZFD domain is fused to one or more of the following elements: nuclear localization sequences (NLS), transcription activation domains (TADs), and/or epigenetic regulatory domains (EPDs). The ATF needs to include a DNA binding domain. The DNA binding domain in this study is RZFD, which can be used alone or in combination with one or more of the following: nuclear localization sequence (NLS), transcription activation domain (TAD) and/or epigenetic regulatory domain (EPD). Furthermore, the NLS, TAD and/or EPD can be located at either the N-terminus or the C-terminus of RZFD domain (Figure 2).

Due to the high complexity of the in vivo environment, the efficiency of in vivo transdifferentiation depends on multiple factors. Not only does the design of the artificial transcription factor influence the result, but also the designs of the expression vector will also influence the result. As shown in Fig. 3A, this study utilizes the astrocyte-specific promoter GFAP to drive the expression of the gene of interest (GOI, which is the artificial transcription factor (ATF)). Some vectors are designed with the GFAP promoter driving the expression of GOI (e.g., Vector Design 1). Some vectors are designed to add the WPRE regulatory sequence following the GOI to enhance the mRNA stability (e.g., Vector Design 2). Some vectors are designed to further include an enhancer in front of the promoter (e.g., Vector Design 3). Some vectors are designed to add both enhancer and WPRE regulatory sequences (e.g., Vector Design 4 and Vector Design 5).

As shown in Fig. 3B, the specific ATF designs used in this study are: the RZFD core domain comprises the amino acid sequence shown in SEQ ID NO: 14. RZFD-V1 adds NLS at the N-terminus of the RZFD core domain (containing 8 zinc finger structures). The nucleic acid sequence is shown in SEQ ID NO: 56 and the amino acid sequence is shown in SEQ ID NO: 57. RZFD-V2 adds the activation domain of VP64 as the transcriptional regulatory element at the C-terminus and add the NLS at the N-terminus of the core domain. The nucleic acid sequence is shown in SEQ ID NO: 58 and the amino acid sequence is shown in SEQ ID NO: 59. RZFD-V3 adds the activation domain of P65-HSF1 at the C-terminus of and add the NLS at the N-terminus of the core domain. The nucleic acid sequence is shown in SEQ ID NO: 60 and the amino acid sequence is shown in SEQ ID NO: 61. RZFD-V4 adds bpNLS at the C-terminus of the core domain. RZFD-V5 adds bpNLS at both the N-terminus and the C-terminus of the core domain. The nucleic acid sequence is shown in SEQ ID NO:31, and the amino acid sequence is shown in SEQ ID NO:30. RZFD-V6 is based on RZFD-V5, with the activation domain of VP64 added to both the N-terminus and the C-terminus of RZFD-V5. The nucleic acid sequence is shown in SEQ ID NO:33, and the amino acid sequence is shown in SEQ ID NO:32. RZFD-V7 is based on RZFD-V6, with the activation domains of P65 and HSF1 further added at the C-terminus of RZFD-V6, while the N-terminus remains the same as RZFD-V6. The nucleic acid sequence is shown in SEQ ID NO:35, and the amino acid sequence is shown in SEQ ID NO:34. RZFD-V8 is based on RZFD-V6, with the activation domains of P65 and Rta added at the C-terminus of RZFD-V6, while the N-terminus remains the same as RZFD-V6. The amino acid sequence is shown in SEQ ID NO:67. RZFD-V9 is based on RZFD-V5, with the activation domain of VP64-P65-HSF1 further added at the C-terminus of RZFD-V5. The amino acid sequence is shown in SEQ ID NO:73. RZFD-V10 is based on RZFD-V5, with the activation domain of VP64-P65-Rta further added at the C-terminus of RZFD-V5. Its amino acid sequence is shown in SEQ ID NO:72. RZFD-V11 is based on RZFD-V5, with the activation domain of VP64 further added to the C-terminus of RZFD-V5. The nucleic acid sequence is shown in SEQ ID NO:91, and the amino acid sequence is shown in SEQ ID NO:90. RZFD-V12 is based on RZFD-V6, with the order of NLS and V64 adjusted. The nucleic acid sequence is shown in SEQ ID NO:93, and the amino acid sequence is shown in SEQ ID NO:92.

Among them, VP64, P65, HSF1, and Vta are all activation domains. In a specific embodiment, the NLS is bpNLS, having the amino acid sequence shown in SEQ ID NO: 24 and the nucleic acid sequence shown in SEQ ID NO: 25. NLS is a nuclear localization signal that can improve the efficiency of entry into the cell nucleus. The activation domain of VP64 has the amino acid sequence shown in SEQ ID NO: 26 and the nucleic acid sequence shown in SEQ ID NO: 27. The fusion form of the activation domains of P65 and HSF1 has the amino acid sequence shown in SEQ ID NO: 28 and the nucleic acid sequence shown in SEQ ID NO: 29. The activation domain of Vta has the amino acid sequence shown in SEQ ID NO: 11.

### Example 3: Adding enhancers and/or adding transcriptional activation domains can increase the production of dopamine neurons

To investigate whether adding enhancer sequences to the expression vector could increase the efficiency of in vivo transdifferentiation and the yield of dopamine neurons, this study designed design-V1 and design-V2 as shown in Fig. 4A, based on RZFD-V5. As shown in Fig. 4A, the sequence of the RZFD core domain is derived from positions 155 to 419 of REST. The astrocyte-specific promoter GFAP is used to drive the expression of the gene of interest, with the ATF (RZFD-V5) in the middle, followed downstream by WPRE and PolyA. Design-V2 is based on design-V1 but incorporates the CMV enhancer upstream of the GFAP promoter and the MVM enhancer downstream of the GFAP promoter. Design-V3 is based on design-V2 but replaces the RZFD-V5 region with RZFD-V6. Design-V4 is based on design-V3 but replaces the RZFD-V6 region with RZFD-V2.

The vectors designed above are packaged into AAV. The control group AAV and experimental AAV are injected into the striatum of 6-OHDA-induced C57 mice, respectively. Samples are collected and analyzed 1.5-3 months after AAV injection, the transdifferentiation of astrocytes into dopamine neurons is assessed. The dopamine neuron-specific marker TH is used to stain, and the results are shown in Fig. 4B. No TH-positive neurons are generated in the control group, while there are numerous of TH-positive dopamine neurons found in the striatum of mice injected with design-V1, design-V2, design-V3, or design-V4. TH-positive cells are counted in each group of design-V1, design-V2, design-V3, and design-V4. The statistical results are shown in Fig. 4C. Design-V1, which uses GFAP to drive ATF (RZFD-V5), generates the fewest dopamine neurons, while design-V2, which includes the enhancer sequence, generates approximately two-fold more dopamine neurons than design-V1. The number of dopamine neurons produced in the design-V3 group is about 2 times that of design-V2 and approximately 4.2 times that of design-V1. These results indicate that not only does adding enhancer sequences increase the number of TH-positive dopamine neurons, but adding the activation domain further enhances the yield of dopamine neurons. The number of dopamine neurons produced by design-V4 group is quantified and find to be similar to that in the design-V3 group. This indicates that having one NLS and one VP64 domain positioned at opposite ends of the RZFD core domain is sufficient to achieve a similar effect comparable to using two NLS-VP64 units.

### Example 4: Partial deletion of the zinc finger domain in RZFD does not affect its transdifferentiation effect in vivo

Previous results show that RZDF-V5 (NLS-RZFD (155-419) -NLS) is sufficient to transdifferentiate astrocytes into dopamine neurons in vivo. To further investigate whether the RZFD (155-419) core domain can be further shortened, different RZFD truncation sequences are designed in this study (Fig. 5A). The sequences of the ATF region is the truncated sequence of REST at positions 159-412 (amino acid sequence is shown in SEQ ID NO: 2), the truncated sequence of REST at positions 209-412 (amino acid sequence is shown in SEQ ID NO: 74), the truncated sequence of REST at positions 159-388 (amino acid sequence shown in SEQ ID NO: 75), and the truncated sequence of REST at positions 209-388 (amino acid sequence shown in SEQ ID NO: 76).

As shown in Figure 5A, different AAV expression vectors are constructed. Among them, the artificial transcription factor in design-V2 utilizes the RZFD core domain corresponding to amino acids 155-419 of REST, with bpNLS positioned at both the N-terminus and C-terminus of the RZFD core domain. The artificial transcription factor in design-V5 utilizes the RZFD core domain corresponding to amino acids 159-412 of REST (having zinc finger domains 1-8), with bpNLS positioned at the N-terminus of the RZFD core domain. The artificial transcription factor in design-V6 utilizes the RZFD core domain corresponding to amino acids 209-412 of REST (having zinc finger domains 1-7), with bpNLS positioned at the N-terminus of the RZFD core domain. The artificial transcription factor in design-V7 utilizes the RZFD core domain corresponding to amino acids 159-388 of REST (having zinc finger domains 2-8), with bpNLS positioned at the N-terminus of the RZFD core domain. The artificial transcription factor in design-V8 utilizes the RZFD core domain corresponding to amino acids 209-388 of REST (having zinc finger domains 2-7), with bpNLS positioned at the N-terminus of the RZFD core domain. During plasmid construction, the astrocyte-specific promoter GFAP is used to drive the expression of the artificial transcription factor. The CMV enhancer is added upstream of the GFAP promoter, the MVM enhancer is added downstream of the GFAP promoter, and a WPRE element is located downstream of the artificial transcription factor.

The above vectors are packaged into AAV, and the AAVs of each group are injected into the striatum of 6-OHDA-induced C57 mice respectively. Samples are collected and analyzed 1.5-2 months after AAV injection, the transdifferentiation of astrocytes into dopamine neurons is assessed. The dopamine neuron-specific marker TH is used for staining, and the results are shown in Fig. 5C. Compared with design-V2, design-V5utilizes the core sequence (159-412) that shows minimal difference from design-V1. The number of TH-positive neurons produced through transdifferentiation by design-V5 is also close to that of design-V1, with no significant difference. Compared with design-V5, design-V6 only contains 7 out of the 8 zinc finger domains (ZFD1-7), but the number of TH-positive neurons produced by transdifferentiation is also close to that of design-V1, with no significant difference. This indicates that the 7 zinc finger domains (ZFD1-7) are sufficient for function. Compared with design-V5, design-V7 only contained 7 out of the 8 zinc finger domains (ZFD2-8), but the number of TH-positive neurons generated by transdifferentiation is close to that of design-V1, with no significant difference. This indicates that 7 zinc finger domains (ZFD2-8) are also sufficient for function. Compared with design-V5, design-V8 only contained 6 out of the 8 zinc finger domains (ZFD2-7), but the number of TH-positive neurons generated by transdifferentiation is also close to that of design-V1, with no significant difference. This indicates that 6 zinc finger domains (ZFD2-7) are also sufficient for function. The number of TH-positive cells in each group is counted, and the results are shown in Fig. 5C. The results show that the in vivo transdifferentiation effects of these designs are equivalent, they can generate similar numbers of dopamine neurons and exhibit similar transdifferentiation efficiency.

### Example 5: Different transcriptional activation domains all can function

To further investigate the effects of different activation domains linked to the RZFD core domain, this study used design-V2 as a benchmark and further designed design-V9, design-V10, and design-V11. In this example, the plasmid designs for design-V2, design-V9, design-V10, and design-V11 are shown in Fig. 6A. The sequence of the RZFD core domain is derived from positions 155 to 419 of REST. The astrocyte-specific promoter GFAP is used to drive expression of the gene of interest. CMV is added upstream of the GFAP promoter as an enhancer, MVM is added downstream of the GFAP promoter as an enhancer, and WPRE is added downstream of the gene of interest. Among them, the ATF of design-V9 is RZFD-V9, the activation domain is VP64-p65-HSF1, the amino acid sequence of RZFD-V9 is shown in SEQ ID NO: 84, and the nucleic acid sequence is shown in SEQ ID NO: 85. The ATF of design-V10 is RZFD-V10, the activation domain is VP64-p65-Rta, the amino acid sequence of RZFD-V10 is shown in SEQ ID NO: 86, and the nucleic acid sequence is shown in SEQ ID NO: 87. The ATF of design-V11 is RZFD-V11, the activation domain is VP64, the amino acid sequence of RZFD-V11 is shown in SEQ ID NO: 90, and the nucleic acid sequence is shown in SEQ ID NO: 91.

The above expression vectors are packaged into AAV, and the control AAV and experimental AAV are injected into the striatum of 6-OHDA-induced C57 mice, respectively. Samples are collected and analyzed 1.5-3 months after AAV injection. The transdifferentiation of astrocytes into dopamine neurons is assessed. The dopamine neuron-specific marker TH is used for staining, and the results are shown in Fig. 6B. A large number of dopamine neurons are generated in the striatum of mice injected with design-V2, design-V9, design-V10, and design-V11 (TH-positive), while there are almost no dopamine neurons in the control group. The TH-positive cells in each group of design-V2, design-V9, design-V10, and design-V11 are counted, and the statistical graph is shown in Fig. 6C. Using Design-V2 (without the activation domain) as a benchmark, the number of dopamine neurons generated in the design-V9 and design-V10 group is approximately 1.42-fold and 1.47-fold that of design-V2, respectively. The number of dopamine neurons generated in the design-V11 group is approximately 1.99-fold that of the design-V2 group. These results demonstrate that the transcriptional activation elements in each group significantly enhance the efficiency of transdifferentiating astrocytes into dopaminergic neurons.

### Example 6: Comparison of transdifferentiation into dopamine neurons

Prior art has disclosed that inhibiting PTBP1 can induce the transdifferentiation of astrocytes into dopamine neurons. In order to compare the transdifferentiation efficiency, CRISPR/CasRx is used to knock down PTBP1, and the transdifferentiation efficiency is compared with that of the AAV of RZFD-V6 in this example. The astrocyte-specific promoter GFAP is used to drive the expression of the target gene (Fig. 7A). The nucleic acid containing CasRx and gRNA targeting PTBP1 (the nucleic acid sequence is shown in SEQ ID NO: 83) is packaged into an AAV vector as the PTBP1-KD group. The packaging of the AAV virus can refer to conventional experimental operations in the art. The AAVs of PTBP1-KD group and RZFD-V6 group are injected into the striatum of mice with the 6-OHDA model, respectively. Samples are collected and analyzed 1.5-2 months after AAV injection, the transdifferentiation of astrocytes into dopamine neurons is assessed. The results are shown in Fig. 7B. Compared with the PTBP1-KD group, the RZFD-V6 group produced more dopamine neurons, approximately twice the amount of the PTBP1-KD group. This indicates that overexpression of RZFD-V6 can more efficiently transdifferentiate astrocytes into dopamine neurons.

### Example 7: Analysis the transdifferentiation of astrocytes into dopamine neurons in Dat-Cre::Ai9 lineage-traced mice

The Dat-Cre::Ai9 mouse is a lineage-tracing mouse that can be used to label mature dopamine neurons. In this mouse, only mature Dat-expressing dopamine neurons are labeled with red fluorescent protein (the details of the mice can refer to technical documents in this field). To label astrocytes in Dat-Cre::Ai9 mice, this study constructed AAV expression vectors as shown in Fig. 8A. The control group used plasmid 1, and the experimental group used plasmid 2. RZFD-V6 is selected as the artificial transcription factor (gene of interest, GOI). The amino acid sequence of RZFD-V6 is shown in SEQ ID NO: 32, and the nucleic acid sequence is shown in SEQ ID NO: 64. The control group (plasmid 1) uses the astrocyte-specific promoter GFAP to drive the expression of EGFP in astrocytes for labeling. The experimental group (plasmid 2) uses the astrocyte-specific promoter GFAP to drive the expression of RZFD-V6. The CMV enhancer is added upstream of the GFAP promoter, and the MVM enhancer is added downstream of the GFAP promoter. The nucleic acid sequence of the CMV enhancer is shown in SEQ ID NO: 16, and the nucleic acid sequence of the MVM enhancer is shown in SEQ ID NO: 18.

The above vectors are packaged into AAV (AAV8) and injected into the striatum of 6-OHDA-modeled Dat-Cre::Ai9 mice. Samples are collected and analyzed 1.5-3 months after injection. The results are shown in Fig. 8B, where green fluorescence indicates cells labeled by AAV-GFAP-EGFP, red fluorescence indicates tdTomato fluorescence expressed by mature dopamine neurons, white fluorescence is immunofluorescence staining for the neuron-specific protein marker NeuN, and blue is DAPI staining for cell nuclei. In the control group injected with AAV-GFAP-EGFP, a large amount of green fluorescent protein is observed in astrocytes. These glial cells still maintain typical astrocyte morphology, don't co-label with NeuN, and have no tdTomato positive signal. In the group injected with GFAP-RZFD-V6, cells expressing green fluorescent protein show typical neuronal morphology, and the green fluorescent protein signal is co-labeled with NeuN (such as the cells indicated by yellow arrows in the GFAP-RZFD-V6 group in Figure 4), indicating that these cells have been reprogrammed from astrocytes into neurons. In the brains of DAT-Cre::Ai9 mice, only mature dopamine neurons can be labeled with tdTomato red fluorescent signals. In the striatum of mice injected with GFAP-RZFD-V6, many cells expressed tdTomato red fluorescence, and these cells are co-labeled with EGFP (such as the cells indicated by white arrows in Figure 4), indicating that RZFD-V6 can reprogram astrocytes into mature dopamine neurons.

### Example 8: Behavioral testing of PD model mice treated with RZFD-V6

To investigate the therapeutic effect of RZFD-V6 for the treatment of Parkinson's disease through dopamine neurons transdifferentiation, this study uses 6-OHDA to establish a Parkinson's disease mouse model in C57 mice with almost identical age. The mouse model undergoes a baseline behavioral test (cylinder test), and mice meeting the inclusion criteria are randomly divided into two groups, with 12 mice in each group. One group serves as a control group, and the other as a treatment group. The plasmid in control group uses GFAP to drive mCherry expression (GFAP-mCherry). In this embodiment, the expression vector design for the treatment group, as shown in Fig. 7A, uses RZFD-V6 as the gene of interest, driven by the astrocyte-specific promoter GFAP. The CMV enhancer is added upstream of the GFAP promoter, and the MVM enhancer (abbreviated as eGFAP) is added downstream of the GFAP promoter. WPRE is added downstream of the gene of interest. The AAVs of the control GFAP-mCherry and the experimental group AAV-eGFAP-RZFD-V6 are injected into the striatum region of the two groups of mice, respectively. Behavioral tests (cylinder tests) are performed 1.5 months after injection. As shown in Figure 9, the control group showed no behavioral differences before and after administration, while the treatment group showed significant behavioral improvements after administration compared to before administration. These results indicate that dopamine neurons generated from astrocyte reprogramming can function as dopamine neurons and significantly improve Parkinson's disease symptoms, potentially enabling the development of medicament to treat Parkinson's disease.

### Example 9: Reprogramming of Astrocytes into Dopamine Neurons by RZFD-V7 and RZFD-V8

To further investigate whether different activation domains fused to the RZFD core domain can function to reprogramm astrocytes into neurons or dopamine neurons, RZFD-V7 and RZFD-V8 are constructed as artificial transcription factors as shown in Fig. 2B. The amino acid sequence of RZFD-V7 is shown in SEQ ID NO: 34, and the nucleic acid sequence is shown in SEQ ID NO: 35. The amino acid sequence of RZFD-V8 is shown in SEQ ID NO: 67, and the nucleic acid sequence is shown in SEQ ID NO: 68. These expression vectors are packaged into AAV, and GFAP-EGFP or GFAP-mCherry is used as a control.

AAVs from the control and experimental groups are injected into the striatum of 6-OHDA-induced Parkinson's disease mice. Samples are collected and analyzed 1.5-3 months after AAV injection to examine the transdifferentiation of astrocytes into neurons or dopamine neurons. Staining with an antibody against the neuron-specific marker NeuN reveals that the red fluorescent signal in the control group does not co-label with NeuN, and these cells retain typical astrocyte morphology. In contrast, in the groups injected with RZFD-V7 and RZFD-V8 AAVs, cells expressing red fluorescent protein exhibit typical neuronal morphology and co-labeled with NeuN. This suggests that overexpressing RZFD-V7 and RZFD-V8 in astrocytes can reprogram astrocytes into neurons. To further investigate whether RZFD-V7 and RZFD-V8 could reprogram astrocytes into dopamine neurons, staining with the dopamine neuron-specific marker TH reveal that a large number of dopamine neurons (TH-positive) are generated in the striatum of mice injected with RZFD-V7 and RZFD-V8, while there are almost no dopamine neurons in the control group. These results suggest that different activation domains linked to the RZFD core domain can reprogram astrocytes into neurons or dopamine neurons.

### Example 10: Transdifferentiation of Miiller glial cells into retinal ganglion cells.

Although neurons are present in retinal tissue, their structure and cell types differ significantly from those in the brain striatum. The retina contains a large number of Müller glia and a small number of astrocytes, and these two cell types are substantially different. To further investigate whether Müller glia can be transdifferentiated into retinal ganglion cells, this example uses the specific promoter GFAP to drive overexpression of the target gene RZFD-V6 in retinal Müller glia.

First, Ai9 mice are modeled using NMDA to eliminate endogenous retinal ganglion cells. 2-3 weeks after the injection of NMDA, control AAV (GFAP-Cre) and experimental group AAV (GFAP-Cre+GFAP-RZFD-V6) are injected respectively. , The samples are collected and analyzed 1.5-2 months after the injection, and the number of red axons in the optic nerve is photographed and quantified. In the control AAV (GFAP-Cre) injection group, there is almost no red fluorescent signal (tdTomato-positive axons) in the optic nerve, while many tdTomato-positive axons are observed in the experimental group injected with GFAP-Cre+GFAP-RZFD-V6. Staining with RGC-specific markers Brn3a and RBPMS, obvious tdTomato-positive RGC cells are observed in the retina of the experimental group. These results indicate that the design scheme of the present application can transdifferentiate Müller glial cells into RGCs.

Although preferred embodiments of the present application are described herein, these embodiments are provided by way of example only for those skilled in the art. Without departing from the technical solutions of the present disclosure, those skilled in the art may make various changes and substitutions with reference to the disclosure of the embodiments. It should be understood that various alternatives to the embodiments of the present disclosure described herein may be adopted when practicing the technical solutions of the present disclosure.

The following are the sequence numbers and sequence information of the sequences involved in this application:

| SEQ ID NO | Description | Sequence |
|---|---|---|
| 1 | human REST | MATQVMGQSSGGGGLFTSSGNIGMALPNDMYDLHDLSKAELAAPQLIMLANVALTGEVNGSCCDY LVGEERQMAELMPVGDNNFSDSEEGEGLEESADIKGEPHGLENMELRSLELSVVEPQPVFEASGAPDI YSSNKDLPPETPGAEDKGKSSKTKPFRCKPCQYEAESEEQFVHHIRVHSAKKFFVEESAEKQAKARES GSSTAEEGDFSKGPIRCDRCGYNTNRYDHYTAHLKHHTRAGDNERVYKCIICTYTTVSEYHWRKHL RNHFPRKVYTCGKCNYFSDRKNNYVQHVRTHTGERPYKCELCPYSSSQKTHLTRHMRTHSGEKPFK CDQCSYVASNQHEVTRHARQVHNGPKPLNCPHCDYKTADRSNFKKHVELHVNPRQFNCPVCDYAA SKKCNLQYHFKSKHPTCPNKTMDVSKVKLKKTKKREADLPDNITNEKTEIEQTKIKGDVAGKKNEK SVKAEKRDVSKEKKPSNNVSVIQVTTRTRKSVTEVKEMDVHTGSNSEKFSKTKKSKRKLEVDSHSLH GPVNDEESSTKKKKKVESKSKNNSQEVPKGDSKVEENKKQNTCMKKSTKKKTLKNKSSKKSSKPPQ KEPVEKGSAQMDPPQMGPAPTEAVQKGPVQVEPPPPMEHAQMEGAQIRPAPDEPVQMEVVQEGPA QKELLPPVEPAQMVGAQIVLAHMELPPPMETAQTEVAQMGPAPMEPAQMEVAQVESAPMQVVQKE PVQMELSPPMEVVQKEPVQIELSPPMEVVQKEPVKIELSPPIEVVQKEPVQMELSPPMGVVQKEPAQR EPPPPREPPLHMEPISKKPPLRKDKKEKSNMQSERARKEQVLIEVGLVPVKDSWLLKESVSTEDLSPPS PPLPKENLREEASGDQKLLNTGEGNKEAPLQKVGAEEADESLPGLAANINESTHISSSGQNLNTPEGE TLNGKHQTDSIVCEMKMDTDQNTRENLTGINSTVEEPVSPMLPPSAVEEREAVSKTALASPPATMAA NESQEIDEDEGIHSHEGSDLSDNMSEGSDDSGLHGARPVPQESSRKNAKEALAVKAAKGDFVCIFCD RSFRKGKDYSKHLNRHLVNVYYLEEAAQGQE |
| 2 | RZFD Core domain | FRCKPCQYEAESEEQFVHHIRVHSAKKFFVEESAEKQAKARESGSSTAEEGDFSKGPIRCDRCGYNTN RYDHYTAHLKHHTRAGDNERVYKCIICTYTTVSEYHWRKHLRNHFPRKVYTCGKCNYFSDRKNNY VQHVRTHTGERPYKCELCPYSSSQKTHLTRHMRTHSGEKPFKCDQCSYVASNQHEVTRHARQVHNG PKPLNCPHCDYKTADRSNFKKHVELHVNPRQFNCPVCDYAASKKCNLQYHFKSKH |
| 3 | ZF-1 | FRCKPCQYEAESEEQFVHHIRVH |
| 4 | ZF-2 | IRCDRCGYNTNRYDHYTAHLKHHTRAGDNERV |
| 5 | ZF-3 | YKCIICTYTTVSEYHWRKH |
| 6 | ZF-4 | YTCGKCNYFSDRKNNYVQHVRTH |
| 7 | ZF-5 | YKCELCPYSSSQKTHLTRHMRTHSGEKP |
| 8 | ZF-6 | FKCDQCSYVASNQHEVTRHARQVHNGPKP |
| 9 | ZF-7 | LNCPHCDYKTADRSNFKKHVELHVNPRQ |
| 10 | ZF-8 | FNCPVCDYAASKKCNLQYHFKSKH |
| 11 | RTA amino acid sequence | DLSHPPPRGHLDELTTTLESMTEDLNLDSPLTPELNEILDTFLNDECLLHAMHISTGLSIFDTSLF |
| 12 | REST N-terminal amino acid | MATQVMGQSSGGGGLFTSSGNIGMALPNDMYDLHDLSKAELAAPQLIMLANVALTGEVNGSCCDY LVGEERQMAELMPVGDNNFSDSEEGEGLEESADIKGEPHGLENMELR |
| 13 | REST C-terminal amino acid | DEGIHSHEGSDLSDNMSEGSDDSGLHGARPVPQESSRKNAKEALAVKAAKGDFVCIFCDRSFRKGKD YSKHLNRHLVNVYYLEEAAQGQE |
| 14 | RZFD amino acid sequence | KTKPFRCKPCQYEAESEEQFVHHIRVHSAKKFFVEESAEKQAKARESGSSTAEEGDFSKGPIRCDRCG YNTNRYDHYTAHLKHHTRAGDNERVYKCIICTYTTVSEYHWRKHLRNHFPRKVYTCGKCNYFSDR KNNYVQHVRTHTGERPYKCELCPYSSSQKTHLTRHMRTHSGEKPFKCDQCSYVASNQHEVTRHARQ VHNGPKPLNCPHCDYKTADRSNFKKHVELHVNPRQFNCPVCDYAASKKCNLQYHFKSKHPTCPNKT |
| 15 | RZFDnucleic acid sequence | AAGACCAAACCCTTTCGCTGTAAGCCATGCCAATATGAAGCAGAATCTGAAGAACAGTTTGTGC ATCACATCAGAGTTCACAGTGCTAAGAAATTTTTTGTGGAAGAGAGTGCAGAGAAGCAGGCAAA AGCCAGGGAATCTGGCTCTTCCACTGCAGAAGAGGGAGATTTCTCCAAGGGCCCCATTCGCTGT GACCGCTGCGGCTACAATACTAATCGATATGATCACTATACAGCACACCTGAAACACCACACCA GAGCTGGGGATAATGAGCGAGTCTACAAGTGTATCATTTGCACATACACAACAGTGAGCGAGTA TCACTGGAGGAAACATTTAAGAAACCATTTTCCAAGGAAAGTATACACATGTGGAAAATGCAAC TATTTTTCAGACAGAAAAAACAATTATGTTCAGCATGTTAGAACTCATACAGGAGAACGCCCAT ATAAATGTGAACTTTGTCCTTACTCAAGTTCTCAGAAGACTCATCTAACTAGACATATGCGTACT CATTCAGGTGAGAAGCCATTTAAATGTGATCAGTGCAGTTATGTGGCCTCTAATCAACATGAAGT AACCCGCCATGCAAGACAGGTTCACAATGGGCCTAAACCTCTTAATTGCCCACACTGTGATTACA AAACAGCAGATAGAAGCAACTTCAAAAAACATGTAGAGCTACATGTGAACCCACGGCAGTTCA ATTGCCCTGTATGTGACTATGCAGCTTCCAAGAAGTGTAATCTACAGTATCACTTCAAATCTAAG CATCCTACTTGTCCTAATAAAACA |
| 16 | CMV enhancer | ATAACTTACGGTAAATGGCCCGCCTGGCTGACCGCCCAACGACCCCCGCCCATTGACGTCAATA ATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACG TCAATGACGGTAAATGGCCCGCCTGGCATTATGCCCAGTACATGACCTTATGGGACTTTCCTACT TGGCAGTACATCTACGTATTAGTCATCGCTATTACCATG |
| 17 | SV40 intronic enhancer | GTAAGTTTAGTCTTTTTGTCTTTTATTTCAGGTCCCGGATCCGGTGGTGGTGCAAATCAAAGAACT GCTCCTCAGTGGATGTTGCCTTTACTTCTAG |
| 18 | MVM intronic enhancer | AAGAGGTAAGGGTTTAAGGGATGGTTGGTTGGTGGGGTATTAATGTTTAATTACCTGGAGCACC TGCCTGAAATCACTTTTTTTCAGGTTGG |
| 19 | Beta-globin intronic enhancer | GTGAGTTTGGGGACCCTTGATTGTTCTTTCTTTTTCGCTATTGTAAAATTCATGTTATATGGAGGG GGCAAAGTTTTCAGGGTGTTGTTTAGAATGGGAAGATGTCCCTTGTATCACCATGGACCCTCATG ATAATTTTGTTTCTTTCACTTTCTACTCTGTTGACAACCATTGTCTCCTCTTATTTTCTTTTCATTTT CTGTAACTTTTTCGTTAAACTTTAGCTTGCATTTGTAACGAATTTTTAAATTCACTTTTGTTTATTT GTCAGATTGTAAGTACTTTCTCTAATCACTTTTTTTTCAAGGCAATCAGGGTATATTATATTGTAC TTCAGCACAGTTTTAGAGAACAATTGTTATAATTAAATGATAAGGTAGAATATTTCTGCATATAA ATTCTGGCTGGCGTGGAAATATTCTTATTGGTAGAAACAACTACACCCTGGTCATCATCCTGCCT TTCTCTTTATGGTTACAATGATATACACTGTTTGAGATGAGGATAAAATACTCTGAGTCCAAACC GGGCCCCTCTGCTAACCATGTTCATGCCTTCTTCTCTTTCCTACAG |
| 20 | Synthetic Intronic Enhancer 1 | GTAAGTGAGTCATATACTCCTAGCAGTCTTCAATGTGAACGACTTTGGAACAAGCGTTCGCACGG CACATTACTAACCAGGCTCTTTCCTCTTCTTCAG |
| 21 | Synthetic Intronic Enhancer 2 | GTAAGTGGATCAACAGCATCTAGTTGTCTTCTGCAACCATCACTCCTTACTGAACTCACGCTCGC GGCTCTACTAACGCGACCTTCCTTCTCCCTCCAG |
| 22 | Synthetic Intronic Enhancer 3 | GTAAGTATCACTCAACTATCTAGCCGTCTTCGGGACGTCATCACATCCAGTCAACCGTGGGCTAC GCATCTACTAACAGCGTCCTTCCTTCTTTCTCAG |
| 23 | Kozak Sequence | GCCRCCATGG |
| 24 | bpNLS amino acid sequence | KRTADGSEFESPKKKRKV |
| 25 | bpNLS nucleic acid sequence | AAACGGACAGCCGATGGCAGCGAGTTCGAGAGCCCCAAGAAGAAGAGAAAGGTG |
| 26 | VP64 amino acid sequence | RADALDDFDLDMLGSDALDDFDLDMLGSDALDDFDLDMLGSDALDDFDLDMLYID |
| 27 | VP64 nucleic acid sequence | AGAGCCGACGCTCTGGACGACTTCGACCTGGATATGCTGGGCTCCGATGCTCTGGATGACTTCGA CCTGGACATGCTGGGCAGCGACGCCCTGGACGACTTCGACCTGGACATGCTGGGAAGCGACGCC CTGGATGATTTTGATCTGGACATGCTCTACATCGAC |
| 28 | P65-HSF1 amino acid sequence | PSGQISNQALALAPSSAPVLAQTMVPSSAMVPLAQPPAPAPVLTPGPPQSLSAPVPKSTQAGEGTLSE ALLHLQFDADEDLGALLGNSTDPGVFTDLASVDNSEFQQLLNQGVSMSHSTAEPMLMEYPEAITRLV TGSQRPPDPAPTPLGTSGLPNGLSGDEDFSSIADMDFSALLSQISSSGQGGGGSGFSVDTSALLDLFSPS VTVPDMSLPDLDSSLASIQELLSPQEPPRPPEAENSSPDSGKQLVHYTAQPLFLLDPGSVDTGSNDLPV LFELGEGSYFSEGDGFAEDPTISLLTGSEPPKAKDPTVS |
| 29 | P65-HSF1 nucleic acid sequence | CCTTCAGGGCAGATCAGCAACCAGGCCCTGGCTCTGGCCCCTAGCTCCGCTCCAGTGCTGGCCCA GACTATGGTGCCCTCTAGTGCTATGGTGCCTCTGGCCCAGCCACCTGCTCCAGCCCCTGTGCTGA CCCCAGGACCACCCCAGTCACTGAGCGCTCCAGTGCCCAAGTCTACACAGGCCGGCGAGGGGAC TCTGAGTGAAGCTCTGCTGCACCTGCAGTTCGACGCTGATGAGGACCTGGGAGCTCTGCTGGGG AACAGCACCGATCCCGGAGTGTTCACAGATCTGGCCTCCGTGGACAACTCTGAGTTTCAGCAGCT GCTGAATCAGGGCGTGTCCATGTCTCATAGTACAGCCGAACCAATGCTGATGGAGTACCCCGAA GCCATTACCCGGCTGGTGACCGGCAGCCAGCGGCCCCCCGACCCCGCTCCAACTCCCCTGGGAA CCAGCGGCCTGCCTAATGGGCTGTCCGGAGATGAAGACTTCTCAAGCATCGCTGATATGGACTTT AGTGCCCTGCTGTCACAGATTTCCTCTAGTGGGCAGGGAGGAGGTGGAAGCGGCTTCAGCGTGG ACACCAGTGCCCTGCTGGACCTGTTCAGCCCCTCGGTGACCGTGCCCGACATGAGCCTGCCTGAC CTTGACAGCAGCCTGGCCAGTATCCAAGAGCTCCTGTCTCCCCAGGAGCCCCCCAGGCCTCCCGA GGCAGAGAACAGCAGCCCGGATTCAGGGAAGCAGCTGGTGCACTACACAGCGCAGCCGCTGTTC CTGCTGGACCCCGGCTCCGTGGACACCGGGAGCAACGACCTGCCGGTGCTGTTTGAGCTGGGAG AGGGCTCCTACTTCTCCGAAGGGGACGGCTTCGCCGAGGACCCCACCATCTCCCTGCTGACAGG CTCGGAGCCTCCCAAAGCCAAGGACCCCACTGTCTCC |
| 30 | RZFD-V5 amino acid sequence | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA |
| | | ASKKCNLQYHFKSKHPTCPNKTMGSKRTADGSEFESPKKKRKV |
| 31 | RZFD-V5 nucleic acid sequence | ATGAAACGGACAGCCGATGGCAGCGAGTTCGAGAGCCCCAAGAAGAAGAGAAAGGTGGGCAGC GGCAAGACCAAGCCCTTCAGGTGTAAACCCTGCCAGTACGAGGCCGAGTCTGAGGAACAGTTTG TCCACCACATCAGAGTGCACAGCGCTAAGAAATTCTTCGTTGAGGAATCTGCCGAGAAGCAGGC CAAGGCCCGGGAAAGCGGCTCCAGTACAGCCGAAGAGGGCGACTTCAGCAAGGGCCCTATCCG GTGCGACAGATGTGGCTATAATACCAACAGATACGACCACTACACAGCGCACCTGAAGCACCAC ACCCGGGCCGGCGACAACGAGAGAGTGTACAAGTGCATCATCTGCACCTACACCACAGTGTCCG AGTACCACTGGCGGAAACATCTCAGAAACCACTTTCCAAGAAAGGTGTACACCTGCGGCAAGTG CAACTACTTCAGCGACCGGAAGAACAACTACGTGCAGCACGTGCGGACACACACCGGCGAGAG ACCTTATAAGTGTGAACTGTGCCCTTACAGCAGCAGCCAGAAAACTCACCTGACCCGCCACATG AGAACCCATAGCGGAGAAAAGCCATTTAAGTGCGACCAGTGCAGCTACGTGGCCAGCAATCAAC ACGAGGTGACCAGACACGCCAGACAGGTGCACAACGGCCCTAAGCCTCTGAACTGTCCCCACTG CGACTACAAAACCGCCGATCGGAGCAACTTCAAGAAACACGTGGAACTGCACGTGAATCCTAGA CAGTTCAACTGCCCCGTGTGCGATTACGCCGCCTCCAAGAAGTGTAACCTGCAATACCACTTCAA GAGCAAGCATCCTACATGCCCTAACAAGACCATGGGCAGCAAGAGAACAGCTGATGGCAGCGA ATTCGAGTCCCCTAAGAAAAAAAGAAAGGTC |
| 32 | RZFD-V6 amino acid sequence | MRADALDDFDLDMLGSDALDDFDLDMLGSDALDDFDLDMLGSDALDDFDLDMLYIDGSGSGGSGS GSGGSGSGGSGSGKRTADGSEFESPKKKRKVGSGKTKPFRCKPCQYEAESEEQFVHHIRVHSAKKFF VEESAEKQAKARESGSSTAEEGDFSKGPIRCDRCGYNTNRYDHYTAHLKHHTRAGDNERVYKCIICT YTTVSEYHWRKHLRNHFPRKVYTCGKCNYFSDRKNNYVQHVRTHTGERPYKCELCPYSSSQKTHLT RHMRTHSGEKPFKCDQCSYVASNQHEVTRHARQVHNGPKPLNCPHCDYKTADRSNFKKHVELHVN PRQFNCPVCDYAASKKCNLQYHFKSKHPTCPNKTMGSKRTADGSEFESPKKKRKVSGGSSGGSSGSE TPGTSESATPESSGGSSGGSSRADALDDFDLDMLGSDALDDFDLDMLGSDALDDFDLDMLGSDALD DFDLDMLYIDGS |
| 33 | RZFD-V6 nucleic acid sequence | ATGAGAGCCGACGCCCTGGATGACTTCGACCTGGACATGCTCGGCAGCGACGCCCTGGACGACT TCGACCTGGATATGCTGGGCTCCGATGCTCTGGACGACTTCGACCTGGACATGCTGGGCTCTGAT GCCCTGGATGATTTTGATCTGGACATGCTGTACATCGACGGCAGCGGCAGCGGCGGCAGCGGCA GCGGCAGCGGCGGAAGCGGATCTGGAGGCAGCGGAAGCGGCAAACGGACAGCCGATGGCAGCG AGTTCGAGAGCCCCAAGAAGAAGAGAAAGGTGGGCAGCGGCAAGACCAAGCCCTTCAGGTGTA AACCCTGCCAGTACGAGGCCGAGTCTGAGGAACAGTTTGTCCACCACATCAGAGTGCACAGCGC TAAGAAATTCTTCGTTGAGGAATCTGCCGAGAAGCAGGCCAAGGCCCGGGAAAGCGGCTCCAGT ACAGCCGAAGAGGGCGACTTCAGCAAGGGCCCTATCCGGTGCGACAGATGTGGCTATAATACCA ACAGATACGACCACTACACAGCGCACCTGAAGCACCACACCCGGGCCGGCGACAACGAGAGAG TGTACAAGTGCATCATCTGCACCTACACCACAGTGTCCGAGTACCACTGGCGGAAACATCTCAG AAACCACTTTCCAAGAAAGGTGTACACCTGCGGCAAGTGCAACTACTTCAGCGACCGGAAGAAC AACTACGTGCAGCACGTGCGGACACACACCGGCGAGAGACCTTATAAGTGTGAACTGTGCCCTT ACAGCAGCAGCCAGAAAACTCACCTGACCCGCCACATGAGAACCCATAGCGGAGAAAAGCCAT TTAAGTGCGACCAGTGCAGCTACGTGGCCAGCAATCAACACGAGGTGACCAGACACGCCAGACA GGTGCACAACGGCCCTAAGCCTCTGAACTGTCCCCACTGCGACTACAAAACCGCCGATCGGAGC AACTTCAAGAAACACGTGGAACTGCACGTGAATCCTAGACAGTTCAACTGCCCCGTGTGCGATT ACGCCGCCTCCAAGAAGTGTAACCTGCAATACCACTTCAAGAGCAAGCATCCTACATGCCCTAA CAAGACCATGGGCAGCAAGCGGACCGCCGATGGCTCTGAGTTCGAGAGCCCCAAGAAGAAAAG AAAGGTGTCCGGCGGCAGCAGCGGAGGCAGCAGCGGCAGCGAGACACCTGGCACCAGCGAATC CGCCACCCCTGAGAGCAGCGGCGGCTCTTCTGGCGGAAGCTCTAGAGCCGACGCTCTGGACGAC TTCGACCTGGATATGCTGGGCTCCGATGCTCTGGATGACTTCGACCTGGACATGCTGGGCAGCGA CGCCCTGGACGACTTCGACCTGGACATGCTGGGAAGCGACGCCCTGGATGATTTTGATCTGGAC ATGCTCTACATCGACGGCAGC |
| 34 | RZFD-V7 amino acid sequence | MRADALDDFDLDMLGSDALDDFDLDMLGSDALDDFDLDMLGSDALDDFDLDMLYIDGSGSGGSGS GSGGSGSGGSGSGKRTADGSEFESPKKKRKVGSGKTKPFRCKPCQYEAESEEQFVHHIRVHSAKKFF VEESAEKQAKARESGSSTAEEGDFSKGPIRCDRCGYNTNRYDHYTAHLKHHTRAGDNERVYKCIICT YTTVSEYHWRKHLRNHFPRKVYTCGKCNYFSDRKNNYVQHVRTHTGERPYKCELCPYSSSQKTHLT RHMRTHSGEKPFKCDQCSYVASNQHEVTRHARQVHNGPKPLNCPHCDYKTADRSNFKKHVELHVN PRQFNCPVCDYAASKKCNLQYHFKSKHPTCPNKTMGSKRTADGSEFESPKKKRKVSGGSSGGSSGSE TPGTSESATPESSGGSSGGSSRADALDDFDLDMLGSDALDDFDLDMLGSDALDDFDLDMLGSDALD DFDLDMLYIDGSDGSGGGGSGGGGSPSGQISNQALALAPSSAPVLAQTMVPSSAMVPLAQPPAPAPV LTPGPPQSLSAPVPKSTQAGEGTLSEALLHLQFDADEDLGALLGNSTDPGVFTDLASVDNSEFQQLLN QGVSMSHSTAEPMLMEYPEAITRLVTGSQRPPDPAPTPLGTSGLPNGLSGDEDFSSIADMDFSALLSQI SSSGQGGGGSGFSVDTSALLDLFSPSVTVPDMSLPDLDSSLASIQELLSPQEPPRPPEAENSSPDSGKQL VHYTAQPLFLLDPGSVDTGSNDLPVLFELGEGSYFSEGDGFAEDPTISLLTGSEPPKAKDPTVS |
| 35 | RZFD-V7 nucleic acid sequence | ATGAGAGCCGACGCCCTGGATGACTTCGACCTGGACATGCTCGGCAGCGACGCCCTGGACGACT TCGACCTGGATATGCTGGGCTCCGATGCTCTGGACGACTTCGACCTGGACATGCTGGGCTCTGAT GCCCTGGATGATTTTGATCTGGACATGCTGTACATCGACGGCAGCGGCAGCGGCGGCAGCGGCA GCGGCAGCGGCGGAAGCGGATCTGGAGGCAGCGGAAGCGGCAAACGGACAGCCGATGGCAGCG AGTTCGAGAGCCCCAAGAAGAAGAGAAAGGTGGGCAGCGGCAAGACCAAGCCCTTCAGGTGTA AACCCTGCCAGTACGAGGCCGAGTCTGAGGAACAGTTTGTCCACCACATCAGAGTGCACAGCGC TAAGAAATTCTTCGTTGAGGAATCTGCCGAGAAGCAGGCCAAGGCCCGGGAAAGCGGCTCCAGT ACAGCCGAAGAGGGCGACTTCAGCAAGGGCCCTATCCGGTGCGACAGATGTGGCTATAATACCA ACAGATACGACCACTACACAGCGCACCTGAAGCACCACACCCGGGCCGGCGACAACGAGAGAG TGTACAAGTGCATCATCTGCACCTACACCACAGTGTCCGAGTACCACTGGCGGAAACATCTCAG AAACCACTTTCCAAGAAAGGTGTACACCTGCGGCAAGTGCAACTACTTCAGCGACCGGAAGAAC AACTACGTGCAGCACGTGCGGACACACACCGGCGAGAGACCTTATAAGTGTGAACTGTGCCCTT ACAGCAGCAGCCAGAAAACTCACCTGACCCGCCACATGAGAACCCATAGCGGAGAAAAGCCAT TTAAGTGCGACCAGTGCAGCTACGTGGCCAGCAATCAACACGAGGTGACCAGACACGCCAGACA GGTGCACAACGGCCCTAAGCCTCTGAACTGTCCCCACTGCGACTACAAAACCGCCGATCGGAGC AACTTCAAGAAACACGTGGAACTGCACGTGAATCCTAGACAGTTCAACTGCCCCGTGTGCGATT ACGCCGCCTCCAAGAAGTGTAACCTGCAATACCACTTCAAGAGCAAGCATCCTACATGCCCTAA CAAGACCATGGGCAGCAAGCGGACCGCCGATGGCTCTGAGTTCGAGAGCCCCAAGAAGAAAAG |
| | | AAAGGTGTCCGGCGGCAGCAGCGGAGGCAGCAGCGGCAGCGAGACACCTGGCACCAGCGAATC CGCCACCCCTGAGAGCAGCGGCGGCTCTTCTGGCGGAAGCTCTAGAGCCGACGCTCTGGACGAC TTCGACCTGGATATGCTGGGCTCCGATGCTCTGGATGACTTCGACCTGGACATGCTGGGCAGCGA CGCCCTGGACGACTTCGACCTGGACATGCTGGGAAGCGACGCCCTGGATGATTTTGATCTGGAC ATGCTCTACATCGACGGCAGCGATGGTAGTGGCGGTGGTGGTTCAGGAGGCGGCGGAAGCCCTT CAGGGCAGATCAGCAACCAGGCCCTGGCTCTGGCCCCTAGCTCCGCTCCAGTGCTGGCCCAGAC TATGGTGCCCTCTAGTGCTATGGTGCCTCTGGCCCAGCCACCTGCTCCAGCCCCTGTGCTGACCC CAGGACCACCCCAGTCACTGAGCGCTCCAGTGCCCAAGTCTACACAGGCCGGCGAGGGGACTCT GAGTGAAGCTCTGCTGCACCTGCAGTTCGACGCTGATGAGGACCTGGGAGCTCTGCTGGGGAAC AGCACCGATCCCGGAGTGTTCACAGATCTGGCCTCCGTGGACAACTCTGAGTTTCAGCAGCTGCT GAATCAGGGCGTGTCCATGTCTCATAGTACAGCCGAACCAATGCTGATGGAGTACCCCGAAGCC ATTACCCGGCTGGTGACCGGCAGCCAGCGGCCCCCCGACCCCGCTCCAACTCCCCTGGGAACCA GCGGCCTGCCTAATGGGCTGTCCGGAGATGAAGACTTCTCAAGCATCGCTGATATGGACTTTAGT GCCCTGCTGTCACAGATTTCCTCTAGTGGGCAGGGAGGAGGTGGAAGCGGCTTCAGCGTGGACA CCAGTGCCCTGCTGGACCTGTTCAGCCCCTCGGTGACCGTGCCCGACATGAGCCTGCCTGACCTT GACAGCAGCCTGGCCAGTATCCAAGAGCTCCTGTCTCCCCAGGAGCCCCCCAGGCCTCCCGAGG CAGAGAACAGCAGCCCGGATTCAGGGAAGCAGCTGGTGCACTACACAGCGCAGCCGCTGTTCCT GCTGGACCCCGGCTCCGTGGACACCGGGAGCAACGACCTGCCGGTGCTGTTTGAGCTGGGAGAG GGCTCCTACTTCTCCGAAGGGGACGGCTTCGCCGAGGACCCCACCATCTCCCTGCTGACAGGCTC GGAGCCTCCCAAAGCCAAGGACCCCACTGTCTCC |
| 36 | CMV enhancer-GFAP promoter-RZFD-V6-WPRE-ployA | ATAACTTACGGTAAATGGCCCGCCTGGCTGACCGCCCAACGACCCCCGCCCATTGACGTCAATA ATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACG TCAATGACGGTAAATGGCCCGCCTGGCATTATGCCCAGTACATGACCTTATGGGACTTTCCTACT TGGCAGTACATCTACGTATTAGTCATCGCTATTACCATGCTCGAGTACTAgAACATATCCTGGTGT GGAGTAGGGGACGCTGCTCTGACAGAGGCTCGGGGGCCTGAGCTGGCTCTGTGAGCTGGGGAGG AGGCAGACAGCCAGGCCTTGTCTGCAAGCAGACCTGGCAGCATTGGGCTGGCCGCCCCCCAGGG CCTCCTCTTCATGCCCAGTGAATGACTCACCTTGGCACAGACACAATGTTCGGGGTGGGCACAGT GCCTGCTTCCCGCCGCACCCCAGCCCCCCTCAAATGCCTTCCGAGAAGCCCATTGAGCAGGGGG CTTGCATTGCACCCCAGCCTGACAGCCTGGCATCTTGGGATAAAAGCAGCACAGCCCCCTAGGG GCTGCCCTTGCTGTGTGGCGCCACCGGCGGTGGAGAACAAGGCTCTATTCAGCCTGTGCCCAGG AAAGGGGATCAGGGGATGCCCAGGCATGGACAGTGGGTGGCAGGGGGGGAGAGGAGGGCTGTC TGCTTCCCAGAAGTCCAAGGACACAAATGGGTGAGGGGAGAGCTCTCCCCATAGCTGGGCTGCG GCCCAaccccaccccctcaggctatgccagggggtgttgccaggggcacccgggcatcgccagtctagcccactccttcataaagccctcgcatcccag gagcgagcagagccagagcaggttggagaggagacgcatcacctccgctgctcgcGAATTGGATCAAGAGGTAAGGGTTTAAG GGATGGTTGGTTGGTGGGGTATTAATGTTTAATTACCTGGAGCACCTGCCTGAAATCACTTTTTTT CAGGTTGGGGATCCGCCACCATGAGAGCCGACGCCCTGGATGACTTCGACCTGGACATGCTCGG CAGCGACGCCCTGGACGACTTCGACCTGGATATGCTGGGCTCCGATGCTCTGGACGACTTCGACC TGGACATGCTGGGCTCTGATGCCCTGGATGATTTTGATCTGGACATGCTGTACATCGACGGCAGC GGCAGCGGCGGCAGCGGCAGCGGCAGCGGCGGAAGCGGATCTGGAGGCAGCGGAAGCGGCAA ACGGACAGCCGATGGCAGCGAGTTCGAGAGCCCCAAGAAGAAGAGAAAGGTGGGCAGCGGCAA GACCAAGCCCTTCAGGTGTAAACCCTGCCAGTACGAGGCCGAGTCTGAGGAACAGTTTGTCCAC CACATCAGAGTGCACAGCGCTAAGAAATTCTTCGTTGAGGAATCTGCCGAGAAGCAGGCCAAGG CCCGGGAAAGCGGCTCCAGTACAGCCGAAGAGGGCGACTTCAGCAAGGGCCCTATCCGGTGCG ACAGATGTGGCTATAATACCAACAGATACGACCACTACACAGCGCACCTGAAGCACCACACCCG GGCCGGCGACAACGAGAGAGTGTACAAGTGCATCATCTGCACCTACACCACAGTGTCCGAGTAC CACTGGCGGAAACATCTCAGAAACCACTTTCCAAGAAAGGTGTACACCTGCGGCAAGTGCAACT ACTTCAGCGACCGGAAGAACAACTACGTGCAGCACGTGCGGACACACACCGGCGAGAGACCTT ATAAGTGTGAACTGTGCCCTTACAGCAGCAGCCAGAAAACTCACCTGACCCGCCACATGAGAAC CCATAGCGGAGAAAAGCCATTTAAGTGCGACCAGTGCAGCTACGTGGCCAGCAATCAACACGAG GTGACCAGACACGCCAGACAGGTGCACAACGGCCCTAAGCCTCTGAACTGTCCCCACTGCGACT ACAAAACCGCCGATCGGAGCAACTTCAAGAAACACGTGGAACTGCACGTGAATCCTAGACAGTT CAACTGCCCCGTGTGCGATTACGCCGCCTCCAAGAAGTGTAACCTGCAATACCACTTCAAGAGC AAGCATCCTACATGCCCTAACAAGACCATGGGCAGCAAGCGGACCGCCGATGGCTCTGAGTTCG AGAGCCCCAAGAAGAAAAGAAAGGTGTCCGGCGGCAGCAGCGGAGGCAGCAGCGGCAGCGAG ACACCTGGCACCAGCGAATCCGCCACCCCTGAGAGCAGCGGCGGCTCTTCTGGCGGAAGCTCTA GAGCCGACGCTCTGGACGACTTCGACCTGGATATGCTGGGCTCCGATGCTCTGGATGACTTCGAC CTGGACATGCTGGGCAGCGACGCCCTGGACGACTTCGACCTGGACATGCTGGGAAGCGACGCCC TGGATGATTTTGATCTGGACATGCTCTACATCGACGGCAGCGATATCGACTACAAGGATGACGA CGACAAGTAAAATTCataatcaacctctggattacaaaatttgtgaaagattgactggtattcttaactatgttgctccttttacgctatgtggatacgct gctttaatgcctttgtatcatgctattgcttcccgtatggctttcattttctcctccttgtataaatcctggttagttcttgccacggcggaactcatcgccgcctgccttgc ccgctgctggacaggggctcggctgttgggcactgacaattccgtggtgtttatttgtgaaatttgtgatgctattgctttatttgtaaccatctagctttatttgtgaaat ttgtgatgctattgctttatttgtaaccattataagctgcaataaacaagttaacaacaacaattgcattcattttatgtttcaggttcagggggagatgtgggaggtttttt aaa |
| 37 | CMV enhancer-GFAP promoter-RZFD-V7-WPRE-ployA | ATAACTTACGGTAAATGGCCCGCCTGGCTGACCGCCCAACGACCCCCGCCCATTGACGTCAATA ATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACG TCAATGACGGTAAATGGCCCGCCTGGCATTATGCCCAGTACATGACCTTATGGGACTTTCCTACT TGGCAGTACATCTACGTATTAGTCATCGCTATTACCATGCTCGAGTACTAgAACATATCCTGGTGT GGAGTAGGGGACGCTGCTCTGACAGAGGCTCGGGGGCCTGAGCTGGCTCTGTGAGCTGGGGAGG AGGCAGACAGCCAGGCCTTGTCTGCAAGCAGACCTGGCAGCATTGGGCTGGCCGCCCCCCAGGG CCTCCTCTTCATGCCCAGTGAATGACTCACCTTGGCACAGACACAATGTTCGGGGTGGGCACAGT GCCTGCTTCCCGCCGCACCCCAGCCCCCCTCAAATGCCTTCCGAGAAGCCCATTGAGCAGGGGG CTTGCATTGCACCCCAGCCTGACAGCCTGGCATCTTGGGATAAAAGCAGCACAGCCCCCTAGGG GCTGCCCTTGCTGTGTGGCGCCACCGGCGGTGGAGAACAAGGCTCTATTCAGCCTGTGCCCAGG AAAGGGGATCAGGGGATGCCCAGGCATGGACAGTGGGTGGCAGGGGGGGAGAGGAGGGCTGTC TGCTTCCCAGAAGTCCAAGGACACAAATGGGTGAGGGGAGAGCTCTCCCCATAGCTGGGCTGCG GCCCAaccccaccccctcaggctatgccagggggtgttgccaggggcacccgggcatcgccagtctagcccactccttcataaagccctcgcatcccag gagcgagcagagccagagcaggttggagaggagacgcatcacctccgctgctcgcGAATTGGATCAAGAGGTAAGGGTTTAAG GGATGGTTGGTTGGTGGGGTATTAATGTTTAATTACCTGGAGCACCTGCCTGAAATCACTTTTTTT |
| | | CAGGTTGGGGATCCGCCACCATGAGAGCCGACGCCCTGGATGACTTCGACCTGGACATGCTCGG CAGCGACGCCCTGGACGACTTCGACCTGGATATGCTGGGCTCCGATGCTCTGGACGACTTCGACC TGGACATGCTGGGCTCTGATGCCCTGGATGATTTTGATCTGGACATGCTGTACATCGACGGCAGC GGCAGCGGCGGCAGCGGCAGCGGCAGCGGCGGAAGCGGATCTGGAGGCAGCGGAAGCGGCAA ACGGACAGCCGATGGCAGCGAGTTCGAGAGCCCCAAGAAGAAGAGAAAGGTGGGCAGCGGCAA GACCAAGCCCTTCAGGTGTAAACCCTGCCAGTACGAGGCCGAGTCTGAGGAACAGTTTGTCCAC CACATCAGAGTGCACAGCGCTAAGAAATTCTTCGTTGAGGAATCTGCCGAGAAGCAGGCCAAGG CCCGGGAAAGCGGCTCCAGTACAGCCGAAGAGGGCGACTTCAGCAAGGGCCCTATCCGGTGCG ACAGATGTGGCTATAATACCAACAGATACGACCACTACACAGCGCACCTGAAGCACCACACCCG GGCCGGCGACAACGAGAGAGTGTACAAGTGCATCATCTGCACCTACACCACAGTGTCCGAGTAC CACTGGCGGAAACATCTCAGAAACCACTTTCCAAGAAAGGTGTACACCTGCGGCAAGTGCAACT ACTTCAGCGACCGGAAGAACAACTACGTGCAGCACGTGCGGACACACACCGGCGAGAGACCTT ATAAGTGTGAACTGTGCCCTTACAGCAGCAGCCAGAAAACTCACCTGACCCGCCACATGAGAAC CCATAGCGGAGAAAAGCCATTTAAGTGCGACCAGTGCAGCTACGTGGCCAGCAATCAACACGAG GTGACCAGACACGCCAGACAGGTGCACAACGGCCCTAAGCCTCTGAACTGTCCCCACTGCGACT ACAAAACCGCCGATCGGAGCAACTTCAAGAAACACGTGGAACTGCACGTGAATCCTAGACAGTT CAACTGCCCCGTGTGCGATTACGCCGCCTCCAAGAAGTGTAACCTGCAATACCACTTCAAGAGC AAGCATCCTACATGCCCTAACAAGACCATGGGCAGCAAGCGGACCGCCGATGGCTCTGAGTTCG AGAGCCCCAAGAAGAAAAGAAAGGTGTCCGGCGGCAGCAGCGGAGGCAGCAGCGGCAGCGAG ACACCTGGCACCAGCGAATCCGCCACCCCTGAGAGCAGCGGCGGCTCTTCTGGCGGAAGCTCTA GAGCCGACGCTCTGGACGACTTCGACCTGGATATGCTGGGCTCCGATGCTCTGGATGACTTCGAC CTGGACATGCTGGGCAGCGACGCCCTGGACGACTTCGACCTGGACATGCTGGGAAGCGACGCCC TGGATGATTTTGATCTGGACATGCTCTACATCGACGGCAGCGATGGTAGTGGCGGTGGTGGTTCA GGAGGCGGCGGAAGCCCTTCAGGGCAGATCAGCAACCAGGCCCTGGCTCTGGCCCCTAGCTCCG CTCCAGTGCTGGCCCAGACTATGGTGCCCTCTAGTGCTATGGTGCCTCTGGCCCAGCCACCTGCT CCAGCCCCTGTGCTGACCCCAGGACCACCCCAGTCACTGAGCGCTCCAGTGCCCAAGTCTACAC AGGCCGGCGAGGGGACTCTGAGTGAAGCTCTGCTGCACCTGCAGTTCGACGCTGATGAGGACCT GGGAGCTCTGCTGGGGAACAGCACCGATCCCGGAGTGTTCACAGATCTGGCCTCCGTGGACAAC TCTGAGTTTCAGCAGCTGCTGAATCAGGGCGTGTCCATGTCTCATAGTACAGCCGAACCAATGCT GATGGAGTACCCCGAAGCCATTACCCGGCTGGTGACCGGCAGCCAGCGGCCCCCCGACCCCGCT CCAACTCCCCTGGGAACCAGCGGCCTGCCTAATGGGCTGTCCGGAGATGAAGACTTCTCAAGCA TCGCTGATATGGACTTTAGTGCCCTGCTGTCACAGATTTCCTCTAGTGGGCAGGGAGGAGGTGGA AGCGGCTTCAGCGTGGACACCAGTGCCCTGCTGGACCTGTTCAGCCCCTCGGTGACCGTGCCCGA CATGAGCCTGCCTGACCTTGACAGCAGCCTGGCCAGTATCCAAGAGCTCCTGTCTCCCCAGGAGC CCCCCAGGCCTCCCGAGGCAGAGAACAGCAGCCCGGATTCAGGGAAGCAGCTGGTGCACTACAC AGCGCAGCCGCTGTTCCTGCTGGACCCCGGCTCCGTGGACACCGGGAGCAACGACCTGCCGGTG CTGTTTGAGCTGGGAGAGGGCTCCTACTTCTCCGAAGGGGACGGCTTCGCCGAGGACCCCACCA TCTCCCTGCTGACAGGCTCGGAGCCTCCCAAAGCCAAGGACCCCACTGTCTCCATCGACTACAAG GATGACGACGACAAGTAAAATTCataatcaacctctggattacaaaatttgtgaaagattgactggtattcttaactatgttgctccttttacgct atgtggatacgctgctttaatgcctttgtatcatgctattgcttcccgtatggctttcattttctcctccttgtataaatcctggttagttcttgccacggcggaactcatcg ccgcctgccttgcccgctgctggacaggggctcggctgttgggcactgacaattccgtggtgtttatttgtgaaatttgtgatgctattgctttatttgtaaccatctag ctttatttgtgaaatttgtgatgctattgctttatttgtaaccattataagctgcaataaacaagttaacaacaacaattgcattcattttatgtttcaggttcagggggaga tgtgggaggttttttaaa |
| 38 | NLS of SV40 viral large T antigen | PKKKRKV |
| 39 | Nucleoplasmic protein NLS | KRPAATKKAGQAKKKK |
| 40 | c-myc NLS | PAAKRVKLD |
| 41 | c-myc NLS | RQRRNELKRSP |
| 42 | hRNPA1 M9 NLS | NQSSNFGPMKGGNFGGRSSGPYGGGGQYFAKPRNQGGY |
| 43 | NLS from the IBB domain of importin-alpha | RMRIZFKNKGKDTAELRRRRVEVSVELRKAKKDEQILKRRNV |
| 44 | Myoma T protein NLS | VSRKRPRP |
| 45 | Myoma T protein NLS | PPKKARED |
| 46 | NLS of human p53 | POPKKKPL |
| 47 | NLS of mouse c-abl IV | SALIKKKKKMAP |
| 48 | NLS of influenza virus NS1 | DRLRR |
| 49 | NLS of influenza virus NS1 | PKQKKRK |
| 50 | NLS of hepatitis delta antigen | RKLKKKIKKL |
| 51 | NLS of mouse Mx1 | REKKKFLKRR |
| 52 | NLS of human poly(ADP-ribose) polymerase | KRKGDEVDGVDEVAKKKSKK |
| 53 | NLS of steroid hormone receptor (human) glucocorticoid | RKCLQAGMNLEARKTKK |
| 54 | NES-1 | LYPERLRRILT |
| 55 | NES-2 | PKKKRKV |
| 56 | NLS-RZFD-V1 nucleic acid sequence | ATGCCTAAAAAGAAAAGAAAGGTGGGTTCTGGTAAGACCAAACCCTTTCGCTGTAAGCCATGCC AATATGAAGCAGAATCTGAAGAACAGTTTGTGCATCACATCAGAGTTCACAGTGCTAAGAAATT TTTTGTGGAAGAGAGTGCAGAGAAGCAGGCAAAAGCCAGGGAATCTGGCTCTTCCACTGCAGAA GAGGGAGATTTCTCCAAGGGCCCCATTCGCTGTGACCGCTGCGGCTACAATACTAATCGATATG ATCACTATACAGCACACCTGAAACACCACACCAGAGCTGGGGATAATGAGCGAGTCTACAAGTG TATCATTTGCACATACACAACAGTGAGCGAGTATCACTGGAGGAAACATTTAAGAAACCATTTT CCAAGGAAAGTATACACATGTGGAAAATGCAACTATTTTTCAGACAGAAAAAACAATTATGTTC AGCATGTTAGAACTCATACAGGAGAACGCCCATATAAATGTGAACTTTGTCCTTACTCAAGTTCT CAGAAGACTCATCTAACTAGACATATGCGTACTCATTCAGGTGAGAAGCCATTTAAATGTGATC AGTGCAGTTATGTGGCCTCTAATCAACATGAAGTAACCCGCCATGCAAGACAGGTTCACAATGG GCCTAAACCTCTTAATTGCCCACACTGTGATTACAAAACAGCAGATAGAAGCAACTTCAAAAAA CATGTAGAGCTACATGTGAACCCACGGCAGTTCAATTGCCCTGTATGTGACTATGCAGCTTCCAA GAAGTGTAATCTACAGTATCACTTCAAATCTAAGCATCCTACTTGTCCTAATAAAACA |
| 57 | NLS-RZFD-V1 amino acid sequence | MPKKKRKVGSGKTKPFRCKPCQYEAESEEQFVHHIRVHSAKKFFVEESAEKQAKARESGSSTAEEGD FSKGPIRCDRCGYNTNRYDHYTAHLKHHTRAGDNERVYKCIICTYTTVSEYHWRKHLRNHFPRKVY TCGKCNYFSDRKNNYVQHVRTHTGERPYKCELCPYSSSQKTHLTRHMRTHSGEKPFKCDQCSYVAS NQHEVTRHARQVHNGPKPLNCPHCDYKTADRSNFKKHVELHVNPRQFNCPVCDYAASKKCNLQYH FKSKHPTCPNKT |
| 58 | NLS-RZFD-V2 nucleic acid sequence | ATGAAACGGACAGCCGATGGCAGCGAGTTCGAGAGCCCCAAGAAGAAGAGAAAGGTGGAATTC GGCAGCGGCTTCAGGTGTAAACCCTGCCAGTACGAGGCCGAGTCTGAGGAACAGTTTGTCCACC ACATCAGAGTGCACAGCGCTAAGAAATTCTTCGTTGAGGAATCTGCCGAGAAGCAGGCCAAGGC CCGGGAAAGCGGCTCCAGTACAGCCGAAGAGGGCGACTTCAGCAAGGGCCCTATCCGGTGCGA CAGATGTGGCTATAATACCAACAGATACGACCACTACACAGCGCACCTGAAGCACCACACCCGG GCCGGCGACAACGAGAGAGTGTACAAGTGCATCATCTGCACCTACACCACAGTGTCCGAGTACC ACTGGCGGAAACATCTCAGAAACCACTTTCCAAGAAAGGTGTACACCTGCGGCAAGTGCAACTA CTTCAGCGACCGGAAGAACAACTACGTGCAGCACGTGCGGACACACACCGGCGAGAGACCTTAT AAGTGTGAACTGTGCCCTTACAGCAGCAGCCAGAAAACTCACCTGACCCGCCACATGAGAACCC ATAGCGGAGAAAAGCCATTTAAGTGCGACCAGTGCAGCTACGTGGCCAGCAATCAACACGAGGT GACCAGACACGCCAGACAGGTGCACAACGGCCCTAAGCCTCTGAACTGTCCCCACTGCGACTAC AAAACCGCCGATCGGAGCAACTTCAAGAAACACGTGGAACTGCACGTGAATCCTAGACAGTTCA ACTGCCCCGTGTGCGATTACGCCGCCTCCAAGAAGTGTAACCTGCAATACCACTTCAAGAGCAA GCATTCCGGCGGCAGCAGCGGAGGCAGCAGCGGCAGCGAGACACCTGGCACCAGCGAATCCGC CACCCCTGAGAGCAGCGGCGGCTCTTCTGGCGGAAGCTCTAGAGCCGACGCTCTGGACGACTTC GACCTGGATATGCTGGGCTCCGATGCTCTGGATGACTTCGACCTGGACATGCTGGGCAGCGACG CCCTGGACGACTTCGACCTGGACATGCTGGGAAGCGACGCCCTGGATGATTTTGATCTGGACAT GCTCTACATCGACGGCAGC |
| 59 | NLS-RZFD-V2 amino acid sequence | MKRTADGSEFESPKKKRKVEFGSGFRCKPCQYEAESEEQFVHHIRVHSAKKFFVEESAEKQAKARES GSSTAEEGDFSKGPIRCDRCGYNTNRYDHYTAHLKHHTRAGDNERVYKCIICTYTTVSEYHWRKHL RNHFPRKVYTCGKCNYFSDRKNNYVQHVRTHTGERPYKCELCPYSSSQKTHLTRHMRTHSGEKPFK CDQCSYVASNQHEVTRHARQVHNGPKPLNCPHCDYKTADRSNFKKHVELHVNPRQFNCPVCDYAA SKKCNLQYHFKSKHSGGSSGGSSGSETPGTSESATPESSGGSSGGSSRADALDDFDLDMLGSDALDDF DLDMLGSDALDDFDLDMLGSDALDDFDLDMLYIDGS |
| 60 | NLS-RZFD-V3 nucleic acid sequence | atgCCTAAAAAGAAAAGAAAGGTGGGTTCTGGTaagaccaaaccctttcgctgtaagccatgccaatatgaagcagaatctgaag aacagtttgtgcatcacatcagagttcacagtgctaagaaattttttgtggaagagagtgcagagaagcaggcaaaagccagggaatctggctcttccactgcag aagagggagatttctccaagggccccattcgctgtgaccgctgcggctacaatactaatcgatatgatcactatacagcacacctgaaacaccacaccagagct ggggataatgagcgagtctacaagtgtatcatttgcacatacacaacagtgagcgagtatcactggaggaaacatttaagaaaccattttccaaggaaagtatac acatgtggaaaatgcaactatttttcagacagaaaaaacaattatgttcagcatgttagaactcatacaggagaacgcccatataaatgtgaactttgtccttactca agttctcagaagactcatctaactagacatatgcgtactcattcaggtgagaagccatttaaatgtgatcagtgcagttatgtggcctctaatcaacatgaagtaacc cgccatgcaagacaggttcacaatgggcctaaacctcttaattgcccacactgtgattacaaaacagcagatagaagcaacttcaaaaaacatgtagagctacat gtgaacccacggcagttcaattgccctgtatgtgactatgcagcttccaagaagtgtaatctacagtatcacttcaaatctaagcatcctacttgtcctaataaaaca atgGGTAGTGGCGGTGGTGGTTCAGGAGGCGGCGGAAGCCCTTCAGGGCAGATCAGCAACCAGGC CCTGGCTCTGGCCCCTAGCTCCGCTCCAGTGCTGGCCCAGACTATGGTGCCCTCTAGTGCTATGG TGCCTCTGGCCCAGCCACCTGCTCCAGCCCCTGTGCTGACCCCAGGACCACCCCAGTCACTGAGC GCTCCAGTGCCCAAGTCTACACAGGCCGGCGAGGGGACTCTGAGTGAAGCTCTGCTGCACCTGC AGTTCGACGCTGATGAGGACCTGGGAGCTCTGCTGGGGAACAGCACCGATCCCGGAGTGTTCAC AGATCTGGCCTCCGTGGACAACTCTGAGTTTCAGCAGCTGCTGAATCAGGGCGTGTCCATGTCTC ATAGTACAGCCGAACCAATGCTGATGGAGTACCCCGAAGCCATTACCCGGCTGGTGACCGGCAG CCAGCGGCCCCCCGACCCCGCTCCAACTCCCCTGGGAACCAGCGGCCTGCCTAATGGGCTGTCC GGAGATGAAGACTTCTCAAGCATCGCTGATATGGACTTTAGTGCCCTGCTGTCACAGATTTCCTC TAGTGGGCAGGGAGGAGGTGGAAGCGGCTTCAGCGTGGACACCAGTGCCCTGCTGGACCTGTTC AGCCCCTCGGTGACCGTGCCCGACATGAGCCTGCCTGACCTTGACAGCAGCCTGGCCAGTATCC AAGAGCTCCTGTCTCCCCAGGAGCCCCCCAGGCCTCCCGAGGCAGAGAACAGCAGCCCGGATTC AGGGAAGCAGCTGGTGCACTACACAGCGCAGCCGCTGTTCCTGCTGGACCCCGGCTCCGTGGAC ACCGGGAGCAACGACCTGCCGGTGCTGTTTGAGCTGGGAGAGGGCTCCTACTTCTCCGAAGGGG ACGGCTTCGCCGAGGACCCCACCATCTCCCTGCTGACAGGCTCGGAGCCTCCCAAAGCCAAGGA |
| | | CCCCACTGTCTCCCCCAAGAAGAAGCGCAAGGTG |
| 61 | NLS-RZFD-V3 amino acid sequence | MPKKKRKVGSGKTKPFRCKPCQYEAESEEQFVHHIRVHSAKKFFVEESAEKQAKARESGSSTAEEGD FSKGPIRCDRCGYNTNRYDHYTAHLKHHTRAGDNERVYKCIICTYTTVSEYHWRKHLRNHFPRKVY TCGKCNYFSDRKNNYVQHVRTHTGERPYKCELCPYSSSQKTHLTRHMRTHSGEKPFKCDQCSYVAS NQHEVTRHARQVHNGPKPLNCPHCDYKTADRSNFKKHVELHVNPRQFNCPVCDYAASKKCNLQYH FKSKHPTCPNKTMGSGGGGSGGGGSPSGQISNQALALAPSSAPVLAQTMVPSSAMVPLAQPPAPAPV LTPGPPQSLSAPVPKSTQAGEGTLSEALLHLQFDADEDLGALLGNSTDPGVFTDLASVDNSEFQQLLN QGVSMSHSTAEPMLMEYPEAITRLVTGSQRPPDPAPTPLGTSGLPNGLSGDEDFSSIADMDFSALLSQI SSSGQGGGGSGFSVDTSALLDLFSPSVTVPDMSLPDLDSSLASIQELLSPQEPPRPPEAENSSPDSGKQL VHYTAQPLFLLDPGSVDTGSNDLPVLFELGEGSYFSEGDGFAEDPTISLLTGSEPPKAKDPTVSPKKKR KV |
| 62 | enhancer-pGFAP-RZFD-V5-WPRE nucleic acid | ATAACTTACGGTAAATGGCCCGCCTGGCTGACCGCCCAACGACCCCCGCCCATTGACGTCAATA ATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACG TCAATGACGGTAAATGGCCCGCCTGGCATTATGCCCAGTACATGACCTTATGGGACTTTCCTACT TGGCAGTACATCTACGTATTAGTCATCGCTATTACCATGCTCGAGTACTAgAACATATCCTGGTGT GGAGTAGGGGACGCTGCTCTGACAGAGGCTCGGGGGCCTGAGCTGGCTCTGTGAGCTGGGGAGG AGGCAGACAGCCAGGCCTTGTCTGCAAGCAGACCTGGCAGCATTGGGCTGGCCGCCCCCCAGGG CCTCCTCTTCATGCCCAGTGAATGACTCACCTTGGCACAGACACAATGTTCGGGGTGGGCACAGT GCCTGCTTCCCGCCGCACCCCAGCCCCCCTCAAATGCCTTCCGAGAAGCCCATTGAGCAGGGGG CTTGCATTGCACCCCAGCCTGACAGCCTGGCATCTTGGGATAAAAGCAGCACAGCCCCCTAGGG GCTGCCCTTGCTGTGTGGCGCCACCGGCGGTGGAGAACAAGGCTCTATTCAGCCTGTGCCCAGG AAAGGGGATCAGGGGATGCCCAGGCATGGACAGTGGGTGGCAGGGGGGGAGAGGAGGGCTGTC TGCTTCCCAGAAGTCCAAGGACACAAATGGGTGAGGGGAGAGCTCTCCCCATAGCTGGGCTGCG GCCCAaccccaccccctcaggctatgccagggggtgttgccaggggcacccgggcatcgccagtctagcccactccttcataaagccctcgcatcccag gagcgagcagagccagagcaggttggagaggagacgcatcacctccgctgctcgcGAATTGGATCAAGAGGTAAGGGTTTAAG GGATGGTTGGTTGGTGGGGTATTAATGTTTAATTACCTGGAGCACCTGCCTGAAATCACTTTTTTT CAGGTTGGGGATCCGCCACCATGAAACGGACAGCCGATGGCAGCGAGTTCGAGAGCCCCAAGA AGAAGAGAAAGGTGGGCAGCGGCAAGACCAAGCCCTTCAGGTGTAAACCCTGCCAGTACGAGG CCGAGTCTGAGGAACAGTTTGTCCACCACATCAGAGTGCACAGCGCTAAGAAATTCTTCGTTGA GGAATCTGCCGAGAAGCAGGCCAAGGCCCGGGAAAGCGGCTCCAGTACAGCCGAAGAGGGCGA CTTCAGCAAGGGCCCTATCCGGTGCGACAGATGTGGCTATAATACCAACAGATACGACCACTAC ACAGCGCACCTGAAGCACCACACCCGGGCCGGCGACAACGAGAGAGTGTACAAGTGCATCATCT GCACCTACACCACAGTGTCCGAGTACCACTGGCGGAAACATCTCAGAAACCACTTTCCAAGAAA GGTGTACACCTGCGGCAAGTGCAACTACTTCAGCGACCGGAAGAACAACTACGTGCAGCACGTG CGGACACACACCGGCGAGAGACCTTATAAGTGTGAACTGTGCCCTTACAGCAGCAGCCAGAAAA CTCACCTGACCCGCCACATGAGAACCCATAGCGGAGAAAAGCCATTTAAGTGCGACCAGTGCAG CTACGTGGCCAGCAATCAACACGAGGTGACCAGACACGCCAGACAGGTGCACAACGGCCCTAA GCCTCTGAACTGTCCCCACTGCGACTACAAAACCGCCGATCGGAGCAACTTCAAGAAACACGTG GAACTGCACGTGAATCCTAGACAGTTCAACTGCCCCGTGTGCGATTACGCCGCCTCCAAGAAGT GTAACCTGCAATACCACTTCAAGAGCAAGCATCCTACATGCCCTAACAAGACCATGGGCAGCAA GAGAACAGCTGATGGCAGCGAATTCGAGTCCCCTAAGAAAAAAAGAAAGGTCTAAataatcaacctctg gattacaaaatttgtgaaagattgactggtattcttaactatgttgctccttttacgctatgtggatacgctgctttaatgcctttgtatcatgctattgcttcccgtatggct ttcattttctcctccttgtataaatcctggttagttcttgccacggcggaactcatcgccgcctgccttgcccgctgctggacaggggctcggctgttgggcactgac aattccgtggtgtttatttgtgaaatttgtgatgctattgctttatttgtaaccatctagctttatttgtgaaatttgtgatgctattgctttatttgtaaccattataagctgcaa taaacaagttaacaacaacaattgcattcattttatgtttcaggttcagggggagatgtgggaggttttttaaa |
| 63 | RZFD-V4 amino acid | MFRCKPCQYEAESEEQFVHHIRVHSAKKFFVEESAEKQAKARESGSSTAEEGDFSKGPIRCDRCGYN TNRYDHYTAHLKHHTRAGDNERVYKCIICTYTTVSEYHWRKHLRNHFPRKVYTCGKCNYFSDRKN NYVQHVRTHTGERPYKCELCPYSSSQKTHLTRHMRTHSGEKPFKCDQCSYVASNQHEVTRHARQVH NGPKPLNCPHCDYKTADRSNFKKHVELHVNPRQFNCPVCDYAASKKCNLQYHFKSKHGSGKRTAD GSEFESPKKKRKV |
| 64 | Codon-optimized RZFD | AAGACCAAGCCCTTCAGGTGTAAACCCTGCCAGTACGAGGCCGAGTCTGAGGAACAGTTTGTCC ACCACATCAGAGTGCACAGCGCTAAGAAATTCTTCGTTGAGGAATCTGCCGAGAAGCAGGCCAA GGCCCGGGAAAGCGGCTCCAGTACAGCCGAAGAGGGCGACTTCAGCAAGGGCCCTATCCGGTG CGACAGATGTGGCTATAATACCAACAGATACGACCACTACACAGCGCACCTGAAGCACCACACC CGGGCCGGCGACAACGAGAGAGTGTACAAGTGCATCATCTGCACCTACACCACAGTGTCCGAGT ACCACTGGCGGAAACATCTCAGAAACCACTTTCCAAGAAAGGTGTACACCTGCGGCAAGTGCAA CTACTTCAGCGACCGGAAGAACAACTACGTGCAGCACGTGCGGACACACACCGGCGAGAGACCT TATAAGTGTGAACTGTGCCCTTACAGCAGCAGCCAGAAAACTCACCTGACCCGCCACATGAGAA CCCATAGCGGAGAAAAGCCATTTAAGTGCGACCAGTGCAGCTACGTGGCCAGCAATCAACACGA GGTGACCAGACACGCCAGACAGGTGCACAACGGCCCTAAGCCTCTGAACTGTCCCCACTGCGAC TACAAAACCGCCGATCGGAGCAACTTCAAGAAACACGTGGAACTGCACGTGAATCCTAGACAGT TCAACTGCCCCGTGTGCGATTACGCCGCCTCCAAGAAGTGTAACCTGCAATACCACTTCAAGAGC AAGCATCCTACATGCCCTAACAAGACC |
| 65 | GFAP Promoter 1 | TAATCCCACCTCCCTCTCTGTGCTGGGACTCACAGAGGGAGACCTCAGGAGGCAGTCTGTCCATC ACATGTCCAAATGCAGAGCATACCCTGGGCTGGGCGCAGTGGCGCACAACTGTAATTCCAGCAC TTTGGGAGGCTGATGTGGAAGGATCACTTGAGCCCAGAAGTTCTAGACCAGCCTGGGCAACATG GCAAGACCCTATCTCTACAAAAAAAGTTAAAAAATCAGCCACGTGTGGTGACACACACCTGTAG TCCCAGCTATTCAGGAGGCTGAGGTGAGGGGATCACTTAAGGCTGGGAGGTTGAGGCTGCAGTG AGTCGTGGTTGCGCCACTGCACTCCAGCCTGGGCAACAGTGAGACCCTGTCTCAAAAGACAAAA AAAAAAAAAAAAAAAAAAGAACATATCCTGGTGTGGAGTAGGGGACGCTGCTCTGACAGAGGC TCGGGGGCCTGAGCTGGCTCTGTGAGCTGGGGAGGAGGCAGACAGCCAGGCCTTGTCTGCAAGC AGACCTGGCAGCATTGGGCTGGCCGCCCCCCAGGGCCTCCTCTTCATGCCCAGTGAATGACTCAC CTTGGCACAGACACAATGTTCGGGGTGGGCACAGTGCCTGCTTCCCGCCGCACCCCAGCCCCCCT |
| | | CAAATGCCTTCCGAGAAGCCCATTGAGCAGGGGGCTTGCATTGCACCCCAGCCTGACAGCCTGG CATCTTGGGATAAAAGCAGCACAGCCCCCTAGGGGCTGCCCTTGCTGTGTGGCGCCACCGGCGG TGGAGAACAAGGCTCTATTCAGCCTGTGCCCAGGAAAGGGGATCAGGGGATGCCCAGGCATGG ACAGTGGGTGGCAGGGGGGGAGAGGAGGGCTGTCTGCTTCCCAGAAGTCCAAGGACACAAATG GGTGAGGGGACTGGGCAGGGTTCTGACCCTGTGGGACCAGAGTGGAGGGCGTAGATGGACCTG AAGTCTCCAGGGACAACAGGGCCCAGGTCTCAGGCTCCTAGTTGGGCCCAGTGGCTCCAGCGTT TCCAAACCCATCCATCCCCAGAGGTTCTTCCCATCTCTCCAGGCTGATGTGTGGGAACTCGAGGA AATAAATCTCCAGTGGGAGACGGAGGGGTGGCCAGGGAAACGGGGCGCTGCAGGAATAAAGAC GAGCCAGCACAGCCAGCTCATGTGTAACGGCTTTGTGGAGCTGTCAAGGCCTGGTCTCTGGGAG AGAGGCACAGGGAGGCCAGACAAGGAAGGGGTGACCTGGAGGGACAGATCCAGGGGCTAAAG TCCTGATAAGGCAAGAGAGTGCCGGCCCCCTCTTGCCCTATCAGGACCTCCACTGCCACATAGA GGCCATGATTGACCCTTAGACAAAGGGCTGGTGTCCAATCCCAGCCCCCAGCCCCAGAACTCCA GGGAATGAATGGGCAGAGAGCAGGAATGTGGGACATCTGTGTTCAAGGGAAGGACTCCAGGAG TCTGCTGGGAATGAGGCCTAGTAGGAAATGAGGTGGCCCTTGAGGGTACAGAACAGGTTCATTC TTCGCCAAATTCCCAGCACCTTGCAGGCACTTACAGCTGAGTGAGATAATGCCTGGGTTATGAAA TCAAAAAGTTGGAAAGCAGGTCAGAGGTCATCTGGTACAGCCCTTCCTTCCCTTTTTTTTTTTTT TTTTTGTGAGACAAGGTCTCTCTCTGTTGCCCAGGCTGGAGTGGCGCAAACACAGCTCACTGCAGC CTCAACCTACTGGGCTCAAGCAATCCTCCAGCCTCAGCCTCCCAAAGTGCTGGGATTACAAGCAT GAGCCACCCCACTCAGCCCTTTCCTTCCTTTTTAATTGATGCATAATAATTGTAAGTATTCATCAT GGTCCAACCAACCCTTTCTTGACCCACCTTCCTAGAGAGAGGGTCCTCTTGCTTCAGCGGTCAGG GCCCCAGACCCATGGTCTGGCTCCAGGTACCACCTGCCTCATGCAGGAGTTGGCGTGCCCAGGA AGCTCTGCCTCTGGGCACAGTGACCTCAGTGGGGTGAGGGGAGCTCTCCCCATAGCTGGGCTGC GGCCCAACCCCACCCCCTCAGGCTATGCCAGGGGGTGTTGCCAGGGGCACCCGGGCATCGCCAG TCTAGCCCACTCCTTCATAAAGCCCTCGCATCCCAGGAGCGAGCAGAGCCAGAGCAGGTTGGAG AGGAGACGCATCACCTCCGCTGCTCGCGG |
| 66 | GFAP Promoter 2 | AACATATCCTGGTGTGGAGTAGGGGACGCTGCTCTGACAGAGGCTCGGGGGCCTGAGCTGGCTC TGTGAGCTGGGGAGGAGGCAGACAGCCAGGCCTTGTCTGCAAGCAGACCTGGCAGCATTGGGCT GGCCGCCCCCCAGGGCCTCCTCTTCATGCCCAGTGAATGACTCACCTTGGCACAGACACAATGTT CGGGGTGGGCACAGTGCCTGCTTCCCGCCGCACCCCAGCCCCCCTCAAATGCCTTCCGAGAAGC CCATTGAGCAGGGGGCTTGCATTGCACCCCAGCCTGACAGCCTGGCATCTTGGGATAAAAGCAG CACAGCCCCCTAGGGGCTGCCCTTGCTGTGTGGCGCCACCGGCGGTGGAGAACAAGGCTCTATT CAGCCTGTGCCCAGGAAAGGGGATCAGGGGATGCCCAGGCATGGACAGTGGGTGGCAGGGGGG GAGAGGAGGGCTGTCTGCTTCCCAGAAGTCCAAGGACACAAATGGGTGAGGGGAGAGCTCTCCC CATAGCTGGGCTGCGGCCCAaccccaccccctcaggctatgccagggggtgttgccaggggcacccgggcatcgccagtctagcccactc cttcataaagccctcgcatcccaggagcgagcagagccagagcaggttggagaggagacgcatcacctccgctgctcgc |
| 67 | RZFD-V8 amino acid sequence | MRADALDDFDLDMLGSDALDDFDLDMLGSDALDDFDLDMLGSDALDDFDLDMLYIDGSGSGGSGS GSGGSGSGGSGSGKRTADGSEFESPKKKRKVGSGKTKPFRCKPCQYEAESEEQFVHHIRVHSAKKFF VEESAEKQAKARESGSSTAEEGDFSKGPIRCDRCGYNTNRYDHYTAHLKHHTRAGDNERVYKCIICT YTTVSEYHWRKHLRNHFPRKVYTCGKCNYFSDRKNNYVQHVRTHTGERPYKCELCPYSSSQKTHLT RHMRTHSGEKPFKCDQCSYVASNQHEVTRHARQVHNGPKPLNCPHCDYKTADRSNFKKHVELHVN PRQFNCPVCDYAASKKCNLQYHFKSKHPTCPNKTMGSKRTADGSEFESPKKKRKVSGGSSGGSSGSE TPGTSESATPESSGGSSGGSSRADALDDFDLDMLGSDALDDFDLDMLGSDALDDFDLDMLGSDALD DFDLDMLYIDGSGSGSVLPQAPAPAPAPAMVSALAQAPAPVPVLAPGPPQAVAPPAPKPTQAGEGTL SEALLQLQFDDEDLGALLGNSTDPAVFTDLASVDNSEFQQLLNQGIPVAPHTTEPMLMEYPEAITRLV TGAQRPPDPAPAPLGAPGLPNGLLSGDEDFSSIADMDFSALLSGGGSGGSGSDLSHPPPRGHLDELTT TLESMTEDLNLDSPLTPELNEILDTFLNDECLLHAMHISTGLSIFDTSLF |
| 68 | RZFD-V8 nucleic acid sequence | ATGAGAGCCGACGCCCTGGATGACTTCGACCTGGACATGCTCGGCAGCGACGCCCTGGACGACT TCGACCTGGATATGCTGGGCTCCGATGCTCTGGACGACTTCGACCTGGACATGCTGGGCTCTGAT GCCCTGGATGATTTTGATCTGGACATGCTGTACATCGACGGCAGCGGCAGCGGCGGCAGCGGCA GCGGCAGCGGCGGAAGCGGATCTGGAGGCAGCGGAAGCGGCAAACGGACAGCCGATGGCAGCG AGTTCGAGAGCCCCAAGAAGAAGAGAAAGGTGGGCAGCGGCAAGACCAAGCCCTTCAGGTGTA AACCCTGCCAGTACGAGGCCGAGTCTGAGGAACAGTTTGTCCACCACATCAGAGTGCACAGCGC TAAGAAATTCTTCGTTGAGGAATCTGCCGAGAAGCAGGCCAAGGCCCGGGAAAGCGGCTCCAGT ACAGCCGAAGAGGGCGACTTCAGCAAGGGCCCTATCCGGTGCGACAGATGTGGCTATAATACCA ACAGATACGACCACTACACAGCGCACCTGAAGCACCACACCCGGGCCGGCGACAACGAGAGAG TGTACAAGTGCATCATCTGCACCTACACCACAGTGTCCGAGTACCACTGGCGGAAACATCTCAG AAACCACTTTCCAAGAAAGGTGTACACCTGCGGCAAGTGCAACTACTTCAGCGACCGGAAGAAC AACTACGTGCAGCACGTGCGGACACACACCGGCGAGAGACCTTATAAGTGTGAACTGTGCCCTT ACAGCAGCAGCCAGAAAACTCACCTGACCCGCCACATGAGAACCCATAGCGGAGAAAAGCCAT TTAAGTGCGACCAGTGCAGCTACGTGGCCAGCAATCAACACGAGGTGACCAGACACGCCAGACA GGTGCACAACGGCCCTAAGCCTCTGAACTGTCCCCACTGCGACTACAAAACCGCCGATCGGAGC AACTTCAAGAAACACGTGGAACTGCACGTGAATCCTAGACAGTTCAACTGCCCCGTGTGCGATT ACGCCGCCTCCAAGAAGTGTAACCTGCAATACCACTTCAAGAGCAAGCATCCTACATGCCCTAA CAAGACCATGGGCAGCAAGCGGACCGCCGATGGCTCTGAGTTCGAGAGCCCCAAGAAGAAAAG AAAGGTGTCCGGCGGCAGCAGCGGAGGCAGCAGCGGCAGCGAGACACCTGGCACCAGCGAATC CGCCACCCCTGAGAGCAGCGGCGGCTCTTCTGGCGGAAGCTCTAGAGCCGACGCTCTGGACGAC TTCGACCTGGATATGCTGGGCTCCGATGCTCTGGATGACTTCGACCTGGACATGCTGGGCAGCGA CGCCCTGGACGACTTCGACCTGGACATGCTGGGAAGCGACGCCCTGGATGATTTTGATCTGGAC ATGCTCTACATCGACGGCAGCggctctggatcagtgctgcctcaggctcctgctcctgcaccagctccagccatggtgtctgcactggctca ggcaccagcacccgtgcctgtgctggctcctggacctccacaggctgtggctccaccagcccctaaacctacacaggccggcgagggcacactgtctgaag ctctgctgcagctgcagttcgacgacgaggatctgggagccctgctgggaaacagcaccgatcctgccgtgttcaccgacctggccagcgtggacaacagcg agttccagcagctgctgaaccagggcatccctgtggcccctcacaccaccgagcccatgctgatggaataccccgaggccatcacccggctcgtgacaggcg ctcagaggcctcctgatccagctcctgcccctctgggagcaccaggcctgcctaatggactgctgtctggcgacgaggacttcagctctatcgccgatatggatt tctcagccttgctgtcaggcggtggtagtggtgggagcggtagtgacctttcccatccgcccccaaggggccatctggatgagctgacaaccacacttgagtcc atgaccgaggatctgaacctggactcacccctgaccccggaattgaacgagattctggataccttcctgaacgacgagtgcctcttgcatgccatgcatatcagc acaggactgtccatcttcgacacatctctgttt |
| 69 | P65 amino acid sequence | PSGQISNQALALAPSSAPVLAQTMVPSSAMVPLAQPPAPAPVLTPGPPQSLSAPVPKSTQAGEGTLSE ALLHLQFDADEDLGALLGNSTDPGVFTDLASVDNSEFQQLLNQGVSMSHSTAEPMLMEYPEAITRLV TGSQRPPDPAPTPLGTSGLPNGLSGDEDFSSIADMDFSALLSQISSSGQ |
| 70 | HSF1 amino acid sequence | GFSVDTSALLDLFSPSVTVPDMSLPDLDSSLASIQELLSPQEPPRPPEAENSSPDSGKQLVHYTAQPLFL LDPGSVDTGSNDLPVLFELGEGSYFSEGDGFAEDPTISLLTGSEPPKAKDPTVS |
| 71 | P65-RTA amino acid sequence | VLPQAPAPAPAPAMVSALAQAPAPVPVLAPGPPQAVAPPAPKPTQAGEGTLSEALLQLQFDDEDLGA LLGNSTDPAVFTDLASVDNSEFQQLLNQGIPVAPHTTEPMLMEYPEAITRLVTGAQRPPDPAPAPLGA PGLPNGLLSGDEDFSSIADMDFSALLSGGGSGGSGSDLSHPPPRGHLDELTTTLESMTEDLNLDSPLTP ELNEILDTFLNDECLLHAMHISTGLSIFDTSLF |
| 72 | VP64-P65-RTA amino acid sequence | DALDDFDLDMLGSDALDDFDLDMLGSDALDDFDLDMLGSDALDDFDLDMLYIDGSGSGSVLPQAP APAPAPAMVSALAQAPAPVPVLAPGPPQAVAPPAPKPTQAGEGTLSEALLQLQFDDEDLGALLGNST DPAVFTDLASVDNSEFQQLLNQGIPVAPHTTEPMLMEYPEAITRLVTGAQRPPDPAPAPLGAPGLPNG LLSGDEDFSSIADMDFSALLSGGGSGGSGSDLSHPPPRGHLDELTTTLESMTEDLNLDSPLTPELNEIL DTFLNDECLLHAMHISTGLSIFDTSLF |
| 73 | VP64-P65-HSF1 amino acid sequence | DALDDFDLDMLGSDALDDFDLDMLGSDALDDFDLDMLGSDALDDFDLDMLYIDGSGSGGGGSGGG GSPSGQISNQALALAPSSAPVLAQTMVPSSAMVPLAQPPAPAPVLTPGPPQSLSAPVPKSTQAGEGTLS EALLHLQFDADEDLGALLGNSTDPGVFTDLASVDNSEFQQLLNQGVSMSHSTAEPMLMEYPEAITRL VTGSQRPPDPAPTPLGTSGLPNGLSGDEDFSSIADMDFSALLSQISSSGQGGGGSGFSVDTSALLDLFSP SVTVPDMSLPDLDSSLASIQELLSPQEPPRPPEAENSSPDSGKQLVHYTAQPLFLLDPGSVDTGSNDLP VLFELGEGSYFSEGDGFAEDPTISLLTGSEPPKAKDPTVS |
| 74 | RZFD(209-412) amino acid sequence | GDFSKGPIRCDRCGYNTNRYDHYTAHLKHHTRAGDNERVYKCIICTYTTVSEYHWRKHLRNHFPRK VYTCGKCNYFSDRKNNYVQHVRTHTGERPYKCELCPYSSSQKTHLTRHMRTHSGEKPFKCDQCSYV ASNQHEVTRHARQVHNGPKPLNCPHCDYKTADRSNFKKHVELHVNPRQFNCPVCDYAASKKCNLQ YHFKSKH |
| 75 | RZFD(159-388) amino acid sequence | FRCKPCQYEAESEEQFVHHIRVHSAKKFFVEESAEKQAKARESGSSTAEEGDFSKGPIRCDRCGYNTN RYDHYTAHLKHHTRAGDNERVYKCIICTYTTVSEYHWRKHLRNHFPRKVYTCGKCNYFSDRKNNY VQHVRTHTGERPYKCELCPYSSSQKTHLTRHMRTHSGEKPFKCDQCSYVASNQHEVTRHARQVHNG PKPLNCPHCDYKTADRSNFKKHVELHVNPRQ |
| 76 | RZFD(209-388) amino acid sequence | GDFSKGPIRCDRCGYNTNRYDHYTAHLKHHTRAGDNERVYKCIICTYTTVSEYHWRKHLRNHFPRK VYTCGKCNYFSDRKNNYVQHVRTHTGERPYKCELCPYSSSQKTHLTRHMRTHSGEKPFKCDQCSYV ASNQHEVTRHARQVHNGPKPLNCPHCDYKTADRSNFKKHVELHVNPRQ |
| 77 | NLS-RZFD(159-412) amino acid sequence | FRCKPCQYEAESEEQFVHHIRVHSAKKFFVEESAEKQAKARESGSSTAEEGDFSKGPIRCDRCGYNTN RYDHYTAHLKHHTRAGDNERVYKCIICTYTTVSEYHWRKHLRNHFPRKVYTCGKCNYFSDRKNNY VQHVRTHTGERPYKCELCPYSSSQKTHLTRHMRTHSGEKPFKCDQCSYVASNQHEVTRHARQVHNG PKPLNCPHCDYKTADRSNFKKHVELHVNPRQFNCPVCDYAASKKCNLQYHFKSKH |
| 78 | NLS-RZFD(209-412) amino acid sequence | MKRTADGSEFESPKKKRKVGSGGDFSKGPIRCDRCGYNTNRYDHYTAHLKHHTRAGDNERVYKCII CTYTTVSEYHWRKHLRNHFPRKVYTCGKCNYFSDRKNNYVQHVRTHTGERPYKCELCPYSSSQKTH LTRHMRTHSGEKPFKCDQCSYVASNQHEVTRHARQVHNGPKPLNCPHCDYKTADRSNFKKHVELH VNPRQFNCPVCDYAASKKCNLQYHFKSKH |
| 79 | NLS-RZFD(159-388) amino acid sequence | MKRTADGSEFESPKKKRKVGSGFRCKPCQYEAESEEQFVHHIRVHSAKKFFVEESAEKQAKARESGS STAEEGDFSKGPIRCDRCGYNTNRYDHYTAHLKHHTRAGDNERVYKCIICTYTTVSEYHWRKHLRN HFPRKVYTCGKCNYFSDRKNNYVQHVRTHTGERPYKCELCPYSSSQKTHLTRHMRTHSGEKPFKCD QCSYVASNQHEVTRHARQVHNGPKPLNCPHCDYKTADRSNFKKHVELHVNPRQ |
| 80 | NLS-RZFD(209-388) amino acid sequence | MKRTADGSEFESPKKKRKVGSGGDFSKGPIRCDRCGYNTNRYDHYTAHLKHHTRAGDNERVYKCII CTYTTVSEYHWRKHLRNHFPRKVYTCGKCNYFSDRKNNYVQHVRTHTGERPYKCELCPYSSSQKTH LTRHMRTHSGEKPFKCDQCSYVASNQHEVTRHARQVHNGPKPLNCPHCDYKTADRSNFKKHVELH VNPRQ |
| 81 | NLS-RZFD(155-419)-VPH | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA ASKKCNLQYHFKSKHPTCPNKTMGSKRTADGSEFESPKKKRKVSGGSSGGSSGSETPGTSESATPESS GGSSGGSSRADALDDFDLDMLGSDALDDFDLDMLGSDALDDFDLDMLGSDALDDFDLDMLYIDGS GSGGGGSGGGGSPSGQISNQALALAPSSAPVLAQTMVPSSAMVPLAQPPAPAPVLTPGPPQSLSAPVP KSTQAGEGTLSEALLHLQFDADEDLGALLGNSTDPGVFTDLASVDNSEFQQLLNQGVSMSHSTAEP MLMEYPEAITRLVTGSQRPPDPAPTPLGTSGLPNGLSGDEDFSSIADMDFSALLSQISSSGQGGGGSGF SVDTSALLDLFSPSVTVPDMSLPDLDSSLASIQELLSPQEPPRPPEAENSSPDSGKQLVHYTAQPLFLLD PGSVDTGSNDLPVLFELGEGSYFSEGDGFAEDPTISLLTGSEPPKAKDPTVS |
| 82 | NLS-RZFD(155-419)-VPR | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA ASKKCNLQYHFKSKHPTCPNKTMGSKRTADGSEFESPKKKRKVSGGSSGGSSGSETPGTSESATPESS GGSSGGSSRADALDDFDLDMLGSDALDDFDLDMLGSDALDDFDLDMLGSDALDDFDLDMLYIDGS GSGSVLPQAPAPAPAPAMVSALAQAPAPVPVLAPGPPQAVAPPAPKPTQAGEGTLSEALLQLQFDDE DLGALLGNSTDPAVFTDLASVDNSEFQQLLNQGIPVAPHTTEPMLMEYPEAITRLVTGAQRPPDPAPA PLGAPGLPNGLLSGDEDFSSIADMDFSALLSGGGSGGSGSDLSHPPPRGHLDELTTTLESMTEDLNLD SPLTPELNEILDTFLNDECLLHAMHISTGLSIFDTSLF |
| 83 | gRNA targeting PTBP1 | ttctggaagttcttggagcccggcttcttg |
| 84 | RZFD-V9 amino acid sequence | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA ASKKCNLQYHFKSKHPTCPNKTMGSKRTADGSEFESPKKKRKVSGGSSGGSSGSETPGTSESATPESS GGSSGGSSRADALDDFDLDMLGSDALDDFDLDMLGSDALDDFDLDMLGSDALDDFDLDMLYIDGS GSGGGGSGGGGSPSGQISNQALALAPSSAPVLAQTMVPSSAMVPLAQPPAPAPVLTPGPPQSLSAPVP KSTQAGEGTLSEALLHLQFDADEDLGALLGNSTDPGVFTDLASVDNSEFQQLLNQGVSMSHSTAEP MLMEYPEAITRLVTGSQRPPDPAPTPLGTSGLPNGLSGDEDFSSIADMDFSALLSQISSSGQGGGGSGF SVDTSALLDLFSPSVTVPDMSLPDLDSSLASIQELLSPQEPPRPPEAENSSPDSGKQLVHYTAQPLFLLD PGSVDTGSNDLPVLFELGEGSYFSEGDGFAEDPTISLLTGSEPPKAKDPTVS |
| 85 | RZFD-V9 nucleic acid sequence | ATGAAACGGACAGCCGATGGCAGCGAGTTCGAGAGCCCCAAGAAGAAGAGAAAGGTGGGCAGC GGCAAGACCAAGCCCTTCAGGTGTAAACCCTGCCAGTACGAGGCCGAGTCTGAGGAACAGTTTG TCCACCACATCAGAGTGCACAGCGCTAAGAAATTCTTCGTTGAGGAATCTGCCGAGAAGCAGGC CAAGGCCCGGGAAAGCGGCTCCAGTACAGCCGAAGAGGGCGACTTCAGCAAGGGCCCTATCCG GTGCGACAGATGTGGCTATAATACCAACAGATACGACCACTACACAGCGCACCTGAAGCACCAC ACCCGGGCCGGCGACAACGAGAGAGTGTACAAGTGCATCATCTGCACCTACACCACAGTGTCCG AGTACCACTGGCGGAAACATCTCAGAAACCACTTTCCAAGAAAGGTGTACACCTGCGGCAAGTG CAACTACTTCAGCGACCGGAAGAACAACTACGTGCAGCACGTGCGGACACACACCGGCGAGAG ACCTTATAAGTGTGAACTGTGCCCTTACAGCAGCAGCCAGAAAACTCACCTGACCCGCCACATG AGAACCCATAGCGGAGAAAAGCCATTTAAGTGCGACCAGTGCAGCTACGTGGCCAGCAATCAAC ACGAGGTGACCAGACACGCCAGACAGGTGCACAACGGCCCTAAGCCTCTGAACTGTCCCCACTG CGACTACAAAACCGCCGATCGGAGCAACTTCAAGAAACACGTGGAACTGCACGTGAATCCTAGA CAGTTCAACTGCCCCGTGTGCGATTACGCCGCCTCCAAGAAGTGTAACCTGCAATACCACTTCAA GAGCAAGCATCCTACATGCCCTAACAAGACCATGGGCAGCAAGCGGACCGCCGATGGCTCTGAG TTCGAGAGCCCCAAGAAGAAAAGAAAGGTGTCCGGCGGCAGCAGCGGAGGCAGCAGCGGCAGC GAGACACCTGGCACCAGCGAATCCGCCACCCCTGAGAGCAGCGGCGGCTCTTCTGGCGGAAGCT CTAGAGCCGACGCTCTGGACGACTTCGACCTGGATATGCTGGGCTCCGATGCTCTGGATGACTTC GACCTGGACATGCTGGGCAGCGACGCCCTGGACGACTTCGACCTGGACATGCTGGGAAGCGACG CCCTGGATGATTTTGATCTGGACATGCTCTACATCGACGGCAGCGGTAGTGGCGGTGGTGGTTCA GGAGGCGGCGGAAGCCCTTCAGGGCAGATCAGCAACCAGGCCCTGGCTCTGGCCCCTAGCTCCG CTCCAGTGCTGGCCCAGACTATGGTGCCCTCTAGTGCTATGGTGCCTCTGGCCCAGCCACCTGCT CCAGCCCCTGTGCTGACCCCAGGACCACCCCAGTCACTGAGCGCTCCAGTGCCCAAGTCTACAC AGGCCGGCGAGGGGACTCTGAGTGAAGCTCTGCTGCACCTGCAGTTCGACGCTGATGAGGACCT GGGAGCTCTGCTGGGGAACAGCACCGATCCCGGAGTGTTCACAGATCTGGCCTCCGTGGACAAC TCTGAGTTTCAGCAGCTGCTGAATCAGGGCGTGTCCATGTCTCATAGTACAGCCGAACCAATGCT GATGGAGTACCCCGAAGCCATTACCCGGCTGGTGACCGGCAGCCAGCGGCCCCCCGACCCCGCT CCAACTCCCCTGGGAACCAGCGGCCTGCCTAATGGGCTGTCCGGAGATGAAGACTTCTCAAGCA TCGCTGATATGGACTTTAGTGCCCTGCTGTCACAGATTTCCTCTAGTGGGCAGGGAGGAGGTGGA AGCGGCTTCAGCGTGGACACCAGTGCCCTGCTGGACCTGTTCAGCCCCTCGGTGACCGTGCCCGA CATGAGCCTGCCTGACCTTGACAGCAGCCTGGCCAGTATCCAAGAGCTCCTGTCTCCCCAGGAGC CCCCCAGGCCTCCCGAGGCAGAGAACAGCAGCCCGGATTCAGGGAAGCAGCTGGTGCACTACAC AGCGCAGCCGCTGTTCCTGCTGGACCCCGGCTCCGTGGACACCGGGAGCAACGACCTGCCGGTG CTGTTTGAGCTGGGAGAGGGCTCCTACTTCTCCGAAGGGGACGGCTTCGCCGAGGACCCCACCA TCTCCCTGCTGACAGGCTCGGAGCCTCCCAAAGCCAAGGACCCCACTGTCTCC |
| 86 | RZFD-V10 amino acid sequence | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA ASKKCNLQYHFKSKHPTCPNKTMGSKRTADGSEFESPKKKRKVSGGSSGGSSGSETPGTSESATPESS GGSSGGSSRADALDDFDLDMLGSDALDDFDLDMLGSDALDDFDLDMLGSDALDDFDLDMLYIDGS GSGSVLPQAPAPAPAPAMVSALAQAPAPVPVLAPGPPQAVAPPAPKPTQAGEGTLSEALLQLQFDDE DLGALLGNSTDPAVFTDLASVDNSEFQQLLNQGIPVAPHTTEPMLMEYPEAITRLVTGAQRPPDPAPA PLGAPGLPNGLLSGDEDFSSIADMDFSALLSGGGSGGSGSDLSHPPPRGHLDELTTTLESMTEDLNLD SPLTPELNEILDTFLNDECLLHAMHISTGLSIFDTSLF |
| 87 | RZFD-V10 nucleic acid sequence | ATGAAACGGACAGCCGATGGCAGCGAGTTCGAGAGCCCCAAGAAGAAGAGAAAGGTGGGCAGC GGCAAGACCAAGCCCTTCAGGTGTAAACCCTGCCAGTACGAGGCCGAGTCTGAGGAACAGTTTG TCCACCACATCAGAGTGCACAGCGCTAAGAAATTCTTCGTTGAGGAATCTGCCGAGAAGCAGGC CAAGGCCCGGGAAAGCGGCTCCAGTACAGCCGAAGAGGGCGACTTCAGCAAGGGCCCTATCCG GTGCGACAGATGTGGCTATAATACCAACAGATACGACCACTACACAGCGCACCTGAAGCACCAC ACCCGGGCCGGCGACAACGAGAGAGTGTACAAGTGCATCATCTGCACCTACACCACAGTGTCCG AGTACCACTGGCGGAAACATCTCAGAAACCACTTTCCAAGAAAGGTGTACACCTGCGGCAAGTG CAACTACTTCAGCGACCGGAAGAACAACTACGTGCAGCACGTGCGGACACACACCGGCGAGAG ACCTTATAAGTGTGAACTGTGCCCTTACAGCAGCAGCCAGAAAACTCACCTGACCCGCCACATG AGAACCCATAGCGGAGAAAAGCCATTTAAGTGCGACCAGTGCAGCTACGTGGCCAGCAATCAAC ACGAGGTGACCAGACACGCCAGACAGGTGCACAACGGCCCTAAGCCTCTGAACTGTCCCCACTG CGACTACAAAACCGCCGATCGGAGCAACTTCAAGAAACACGTGGAACTGCACGTGAATCCTAGA CAGTTCAACTGCCCCGTGTGCGATTACGCCGCCTCCAAGAAGTGTAACCTGCAATACCACTTCAA GAGCAAGCATCCTACATGCCCTAACAAGACCATGGGCAGCAAGCGGACCGCCGATGGCTCTGAG TTCGAGAGCCCCAAGAAGAAAAGAAAGGTGTCCGGCGGCAGCAGCGGAGGCAGCAGCGGCAGC GAGACACCTGGCACCAGCGAATCCGCCACCCCTGAGAGCAGCGGCGGCTCTTCTGGCGGAAGCT CTAGAGCCGACGCTCTGGACGACTTCGACCTGGATATGCTGGGCTCCGATGCTCTGGATGACTTC GACCTGGACATGCTGGGCAGCGACGCCCTGGACGACTTCGACCTGGACATGCTGGGAAGCGACG CCCTGGATGATTTTGATCTGGACATGCTCTACATCGACGGCAGCggctctggatcagtgctgcctcaggctcctgctc ctgcaccagctccagccatggtgtctgcactggctcaggcaccagcacccgtgcctgtgctggctcctggacctccacaggctgtggctccaccagcccctaa acctacacaggccggcgagggcacactgtctgaagctctgctgcagctgcagttcgacgacgaggatctgggagccctgctgggaaacagcaccgatcctg ccgtgttcaccgacctggccagcgtggacaacagcgagttccagcagctgctgaaccagggcatccctgtggcccctcacaccaccgagcccatgctgatgg aataccccgaggccatcacccggctcgtgacaggcgctcagaggcctcctgatccagctcctgcccctctgggagcaccaggcctgcctaatggactgctgtc tggcgacgaggacttcagctctatcgccgatatggatttctcagccttgctgtcaggcggtggtagtggtgggagcggtagtgacctttcccatccgcccccaag gggccatctggatgagctgacaaccacacttgagtccatgaccgaggatctgaacctggactcacccctgaccccggaattgaacgagattctggataccttcc tgaacgacgagtgcctcttgcatgccatgcatatcagcacaggactgtccatcttcgacacatctctgttt |
| 88 | dRT-VI amino acid sequence | MFSDSEEGEGLEESADIKGEPHGLENMELRSLELSVVEPQPVFEASGAPDIYSSNKDLPPETPGAEDKG KSSKTKPFRCKPCQYEAESEEQFVHHIRVHSAKKFFVEESAEKQAKARESGSSTAEEGDFSKGPIRCD RCGYNTNRYDHYTAHLKHHTRAGDNERVYKCIICTYTTVSEYHWRKHLRNHFPRKVYTCGKCNYF SDRKNNYVQHVRTHTGERPYKCELCPYSSSQKTHLTRHMRTHSGEKPFKCDQCSYVASNQHEVTRH ARQVHNGPKPLNCPHCDYKTADRSNFKKHVELHVNPRQFNCPVCDYAASKKCNLQYHFKSKHPTC PNKTMDVSKVKLKKTKKREADLPDNITNEKTEIEQTKIKGDVAGKKNEKSVKAEKRDVSKEKKPSN NVSVIQVTTRTRKSVTEVKEMDVHTGSNSEKFSKTKKSKRKLEVDSHSLHGPVNDEESSTKKKKKVE SKSKNNSQEVPKGDSKVEENKKQNTCMKKSTKKKTLKNKSSKKSSKPPQKEPVEKGSAQMDPPQM GPAPTEAVQKGPVQVEPPPPMEHAQMEGAQIRPAPDEPVQMEVVQEGPAQKELLPPVEPAQMVGAQ IVLAHMELPPPMETAQTEVAQMGPAPMEPAQMEVAQVESAPMQVVQKEPVQMELSPPMEVVQKEP |
| | | VQIELSPPMEVVQKEPVKIELSPPIEVVQKEPVQMELSPPMGVVQKEPAQREPPPPREPPLHMEPISKK PPLRKDKKEKSNMQSERARKEQVLIEVGLVPVKDSWLLKESVSTEDLSPPSPPLPKENLREEASGDQK LLNTGEGNKEAPLQKVGAEEADESLPGLAANINESTHISSSGQNLNTPEGETLNGKHQTDSIVCEMK MDTDQNTRENLTGINSTVEEPVSPMLPPSAVEEREAVSKTALASPPATMAANESQEIDEGSGGGGSG GGGSRADALDDFDLDMLGSDALDDFDLDMLGSDALDDFDLDMLGSDALDDFDLDMLYID |
| 89 | dRT-V2 amino acid sequence | MAELMPVGDNNFSDSEEGEGLEESADIKGEPHGLENMELRSLELSVVEPQPVFEASGAPDIYSSNKDL PPETPGAEDKGKSSKTKPFRCKPCQYEAESEEQFVHHIRVHSAKKFFVEESAEKQAKARESGSSTAEE GDFSKGPIRCDRCGYNTNRYDHYTAHLKHHTRAGDNERVYKCIICTYTTVSEYHWRKHLRNHFPRK VYTCGKCNYFSDRKNNYVQHVRTHTGERPYKCELCPYSSSQKTHLTRHMRTHSGEKPFKCDQCSYV ASNQHEVTRHARQVHNGPKPLNCPHCDYKTADRSNFKKHVELHVNPRQFNCPVCDYAASKKCNLQ YHFKSKHPTCPNKTMDVSKVKLKKTKKREADLPDNITNEKTEIEQTKIKGDVAGKKNEKSVKAEKR DVSKEKKPSNNVSVIQVTTRTRKSVTEVKEMDVHTGSGSDALDDFDLDML |
| 90 | RZFD-V11 amino acid sequence | MKRTADGSEFESPKKKRKVGSGKTKPFRCKPCQYEAESEEQFVHHIRVHSAKKFFVEESAEKQAKAR ESGSSTAEEGDFSKGPIRCDRCGYNTNRYDHYTAHLKHHTRAGDNERVYKCIICTYTTVSEYHWRKH LRNHFPRKVYTCGKCNYFSDRKNNYVQHVRTHTGERPYKCELCPYSSSQKTHLTRHMRTHSGEKPF KCDQCSYVASNQHEVTRHARQVHNGPKPLNCPHCDYKTADRSNFKKHVELHVNPRQFNCPVCDYA ASKKCNLQYHFKSKHPTCPNKTMGSKRTADGSEFESPKKKRKVSGGSSGGSSGSETPGTSESATPESS GGSSGGSSRADALDDFDLDMLGSDALDDFDLDMLGSDALDDFDLDMLGSDALDDFDLDMLYIDGS |
| 91 | RZFD-V11 nucleic acid sequence | ATGAAACGGACAGCCGATGGCAGCGAGTTCGAGAGCCCCAAGAAGAAGAGAAAGGTGGGCAGC GGCAAGACCAAGCCCTTCAGGTGTAAACCCTGCCAGTACGAGGCCGAGTCTGAGGAACAGTTTG TCCACCACATCAGAGTGCACAGCGCTAAGAAATTCTTCGTTGAGGAATCTGCCGAGAAGCAGGC CAAGGCCCGGGAAAGCGGCTCCAGTACAGCCGAAGAGGGCGACTTCAGCAAGGGCCCTATCCG GTGCGACAGATGTGGCTATAATACCAACAGATACGACCACTACACAGCGCACCTGAAGCACCAC ACCCGGGCCGGCGACAACGAGAGAGTGTACAAGTGCATCATCTGCACCTACACCACAGTGTCCG AGTACCACTGGCGGAAACATCTCAGAAACCACTTTCCAAGAAAGGTGTACACCTGCGGCAAGTG CAACTACTTCAGCGACCGGAAGAACAACTACGTGCAGCACGTGCGGACACACACCGGCGAGAG ACCTTATAAGTGTGAACTGTGCCCTTACAGCAGCAGCCAGAAAACTCACCTGACCCGCCACATG AGAACCCATAGCGGAGAAAAGCCATTTAAGTGCGACCAGTGCAGCTACGTGGCCAGCAATCAAC ACGAGGTGACCAGACACGCCAGACAGGTGCACAACGGCCCTAAGCCTCTGAACTGTCCCCACTG CGACTACAAAACCGCCGATCGGAGCAACTTCAAGAAACACGTGGAACTGCACGTGAATCCTAGA CAGTTCAACTGCCCCGTGTGCGATTACGCCGCCTCCAAGAAGTGTAACCTGCAATACCACTTCAA GAGCAAGCATCCTACATGCCCTAACAAGACCATGGGCAGCAAGCGGACCGCCGATGGCTCTGAG TTCGAGAGCCCCAAGAAGAAAAGAAAGGTGTCCGGCGGCAGCAGCGGAGGCAGCAGCGGCAGC GAGACACCTGGCACCAGCGAATCCGCCACCCCTGAGAGCAGCGGCGGCTCTTCTGGCGGAAGCT CTAGAGCCGACGCTCTGGACGACTTCGACCTGGATATGCTGGGCTCCGATGCTCTGGATGACTTC GACCTGGACATGCTGGGCAGCGACGCCCTGGACGACTTCGACCTGGACATGCTGGGAAGCGACG CCCTGGATGATTTTGATCTGGACATGCTCTACATCGACGGCAGC |
| 92 | RZFD-V12 amino acid sequence | MKRTADGSEFESPKKKRKVGSGDALDDFDLDMLGSDALDDFDLDMLGSDALDDFDLDMLGSDALD DFDLDMLYIDGSSGGSSGGSSGSETPGTSESATPESSGGSSGGSSKTKPFRCKPCQYEAESEEQFVHHI RVHSAKKFFVEESAEKQAKARESGSSTAEEGDFSKGPIRCDRCGYNTNRYDHYTAHLKHHTRAGDN ERVYKCIICTYTTVSEYHWRKHLRNHFPRKVYTCGKCNYFSDRKNNYVQHVRTHTGERPYKCELCP YSSSQKTHLTRHMRTHSGEKPFKCDQCSYVASNQHEVTRHARQVHNGPKPLNCPHCDYKTADRSNF KKHVELHVNPRQFNCPVCDYAASKKCNLQYHFKSKHPTCPNKTMSGGSSGGSSGSETPGTSESATPE SSGGSSGGSSRADALDDFDLDMLGSDALDDFDLDMLGSDALDDFDLDMLGSDALDDFDLDMLYID GSGSKRTADGSEFESPKKKRKV |
| 93 | RZFD-V12 nucleic acid sequence | ATGAAACGGACAGCCGATGGCAGCGAGTTCGAGAGCCCCAAGAAGAAGAGAAAGGTGGGCAGC GGCGACGCTCTGGACGACTTCGACCTGGATATGCTGGGCTCCGATGCTCTGGATGACTTCGACCT GGACATGCTGGGCAGCGACGCCCTGGACGACTTCGACCTGGACATGCTGGGAAGCGACGCCCTG GATGATTTTGATCTGGACATGCTCTACATCGACGGCAGCTCCGGCGGCAGCAGCGGAGGCAGCA GCGGCAGCGAGACACCTGGCACCAGCGAATCCGCCACCCCTGAGAGCAGCGGCGGCTCTTCTGG CGGAAGCTCTAAGACCAAGCCCTTCAGGTGTAAACCCTGCCAGTACGAGGCCGAGTCTGAGGAA CAGTTTGTCCACCACATCAGAGTGCACAGCGCTAAGAAATTCTTCGTTGAGGAATCTGCCGAGA AGCAGGCCAAGGCCCGGGAAAGCGGCTCCAGTACAGCCGAAGAGGGCGACTTCAGCAAGGGCC CTATCCGGTGCGACAGATGTGGCTATAATACCAACAGATACGACCACTACACAGCGCACCTGAA GCACCACACCCGGGCCGGCGACAACGAGAGAGTGTACAAGTGCATCATCTGCACCTACACCACA GTGTCCGAGTACCACTGGCGGAAACATCTCAGAAACCACTTTCCAAGAAAGGTGTACACCTGCG GCAAGTGCAACTACTTCAGCGACCGGAAGAACAACTACGTGCAGCACGTGCGGACACACACCG GCGAGAGACCTTATAAGTGTGAACTGTGCCCTTACAGCAGCAGCCAGAAAACTCACCTGACCCG CCACATGAGAACCCATAGCGGAGAAAAGCCATTTAAGTGCGACCAGTGCAGCTACGTGGCCAGC AATCAACACGAGGTGACCAGACACGCCAGACAGGTGCACAACGGCCCTAAGCCTCTGAACTGTC CCCACTGCGACTACAAAACCGCCGATCGGAGCAACTTCAAGAAACACGTGGAACTGCACGTGAA TCCTAGACAGTTCAACTGCCCCGTGTGCGATTACGCCGCCTCCAAGAAGTGTAACCTGCAATACC ACTTCAAGAGCAAGCATCCTACATGCCCTAACAAGACCATGTCCGGCGGCAGCAGCGGAGGCAG CAGCGGCAGCGAGACACCTGGCACCAGCGAATCCGCCACCCCTGAGAGCAGCGGCGGCTCTTCT GGCGGAAGCTCTAGAGCCGACGCTCTGGACGACTTCGACCTGGATATGCTGGGCTCCGATGCTC TGGATGACTTCGACCTGGACATGCTGGGCAGCGACGCCCTGGACGACTTCGACCTGGACATGCT GGGAAGCGACGCCCTGGATGATTTTGATCTGGACATGCTCTACATCGACGGCAGCGGCAGCAAG CGGACCGCCGATGGCTCTGAGTTCGAGAGCCCCAAGAAGAAAAGAAAGGTG |
| 94 | dRT-VI nucleic acid sequence | atgttttcagatagtgaagaaggagaaggacttgaagagtctgctgatataaaaggtgaacctcatggactggaaaacatggaactgagaagtttggaactcagc gtcgtagaacctcagcctgtatttgaggcatcaggtgctccagatatttacagttcaaataaagatcttccccctgaaacacctggagcggaggacaaaggcaag agctcgaagaccaaaccctttcgctgtaagccatgccaatatgaagcagaatctgaagaacagtttgtgcatcacatcagagttcacagtgctaagaaattttttgt ggaagagagtgcagagaagcaggcaaaagccagggaatctggctcttccactgcagaagagggagatttctccaagggccccattcgctgtgaccgctgcg gctacaatactaatcgatatgatcactatacagcacacctgaaacaccacaccagagctggggataatgagcgagtctacaagtgtatcatttgcacatacacaa cagtgagcgagtatcactggaggaaacatttaagaaaccattttccaaggaaagtatacacatgtggaaaatgcaactatttttcagacagaaaaaacaattatgtt cagcatgttagaactcatacaggagaacgcccatataaatgtgaactttgtccttactcaagttctcagaagactcatctaactagacatatgcgtactcattcaggt gagaagccatttaaatgtgatcagtgcagttatgtggcctctaatcaacatgaagtaacccgccatgcaagacaggttcacaatgggcctaaacctcttaattgcc cacactgtgattacaaaacagcagatagaagcaacttcaaaaaacatgtagagctacatgtgaacccacggcagttcaattgccctgtatgtgactatgcagcttc caagaagtgtaatctacagtatcacttcaaatctaagcatcctacttgtcctaataaaacaatggatgtctcaaaagtgaaactaaagaaaaccaaaaaacgagag gctgacttgcctgataatattaccaatgaaaaaacagaaatagaacaaacaaaaataaaaggggatgtggctggaaagaaaaatgaaaagtccgtcaaagcag agaaaagagatgtctcaaaagagaaaaagccttctaataatgtgtcagtgatccaggtgactaccagaactcgaaaatcagtaacagaggtgaaagagatggat gtgcatacaggaagcaattcagaaaaattcagtaaaactaagaaaagcaaaaggaagctggaagttgacagccattctttacatggtcctgtgaatgatgagga atcttcaacaaaaaagaaaaagaaggtagaaagcaaatccaaaaataatagtcaggaagtgccaaagggtgacagcaaagtggaggagaataaaaagcaaa atacttgcatgaaaaaaagtacaaagaagaaaactctgaaaaataaatcaagtaagaaaagcagtaagcctcctcagaaggaacctgttgagaagggatctgct cagatggaccctcctcagatggggcctgctcccacagaggcggttcagaaggggcccgttcaggtggagccgccacctcccatggagcatgctcagatgga gggtgcccagatacggcctgctcctgacgagcctgttcagatggaggtggttcaggaggggcctgctcagaaggagctgctgcctcccgtggagcctgctca gatggtgggtgcccaaattgtacttgctcacatggagctgcctcctcccatggagactgctcagacggaggttgcccaaatggggcctgctcccatggaacctg ctcagatggaggttgcccaggtagaatctgctcccatgcaggtggtccagaaggagcctgttcagatggagctgtctcctcccatggaggtggtccagaagga gcctgttcagatagagctgtctcctcccatggaggtggtccagaaggaacctgttaagatagagctgtctcctcccatagaggtggtccagaaggagcctgttca gatggagttgtctcctcccatgggggtggttcagaaggagcctgctcagagggagccacctcctcccagagagcctccccttcacatggagccaatttccaaaa agcctcctctccgaaaagataaaaaggaaaagtctaacatgcagagtgaaagggcacggaaggagcaagtccttattgaagttggcttagtgcctgttaaagat agctggcttctaaaggaaagtgtaagcacagaggatctctcaccaccatcaccaccactgccaaaggaaaatttaagagaagaggcatcaggagaccaaaaat tactcaacacaggtgaaggaaataaagaagcccctcttcagaaagtaggagcagaagaggcagatgagagcctacctggtcttgctgctaatatcaacgaatct acccatatttcatcctctggacaaaacttgaatacgccagagggtgaaactttaaatggtaaacatcagactgacagtatagtttgtgaaatgaaaatggacactga tcagaacacaagagagaatctcactggtataaattcaacagttgaagaaccagtttcaccaatgcttcccccttcagcagtagaagaacgtgaagcagtgtccaa aactgcactggcatcacctcctgctacaatggcagcaaatgagtctcaggaaattgatgaaGGTAGTGGCGGTGGTGGTTCAGGAGGC GGCGGAAGCCGCGCCGACGCGCTGGACGATTTCGATCTCGACATGCTGGGTTCTGATGCCCTCG ATGACTTTGACCTGGATATGTTGGGAAGCGACGCATTGGATGACTTTGATCTGGACATGCTCGGC TCCGATGCTCTGGACGATTTCGATCTCGATATGTTATATATCGAT |
| 95 | dRT-V2 nucleic acid sequence | atggcagaactgatgccggttggggataacaacttttcagatagtgaagaaggagaaggacttgaagagtctgctgatataaaaggtgaacctcatggactgga aaacatggaactgagaagtttggaactcagcgtcgtagaacctcagcctgtatttgaggcatcaggtgctccagatatttacagttcaaataaagatcttccccctg aaacacctggagcggaggacaaaggcaagagctcgaagaccaaaccctttcgctgtaagccatgccaatatgaagcagaatctgaagaacagtttgtgcatca catcagagttcacagtgctaagaaattttttgtggaagagagtgcagagaagcaggcaaaagccagggaatctggctcttccactgcagaagagggagatttct ccaagggccccattcgctgtgaccgctgcggctacaatactaatcgatatgatcactatacagcacacctgaaacaccacaccagagctggggataatgagcg agtctacaagtgtatcatttgcacatacacaacagtgagcgagtatcactggaggaaacatttaagaaaccattttccaaggaaagtatacacatgtggaaaatgc aactatttttcagacagaaaaaacaattatgttcagcatgttagaactcatacaggagaacgcccatataaatgtgaactttgtccttactcaagttctcagaagactc atctaactagacatatgcgtactcattcaggtgagaagccatttaaatgtgatcagtgcagttatgtggcctctaatcaacatgaagtaacccgccatgcaagacag gttcacaatgggcctaaacctcttaattgcccacactgtgattacaaaacagcagatagaagcaacttcaaaaaacatgtagagctacatgtgaacccacggcag ttcaattgccctgtatgtgactatgcagcttccaagaagtgtaatctacagtatcacttcaaatctaagcatcctacttgtcctaataaaacaatggatgtctcaaaagt gaaactaaagaaaaccaaaaaacgagaggctgacttgcctgataatattaccaatgaaaaaacagaaatagaacaaacaaaaataaaaggggatgtggctgg aaagaaaaatgaaaagtccgtcaaagcagagaaaagagatgtctcaaaagagaaaaagccttctaataatgtgtcagtgatccaggtgactaccagaactcga aaatcagtaacagaggtgaaagagatggatgtgcatacaggaagcGGCTCCGATGCTCTGGACGACTTCGACCTGGACATG CTG |

## Claims

1. An expression vector, comprising:
(a) a nucleic acid sequence encoding an artificial transcription factor capable of binding to RE1, said artificial transcription factor comprises an RZFD core domain;
(b) a regulatory element regulating the expression of the nucleic acid of said artificial transcription factor;
wherein the amino acid sequence of said RZFD core domain comprises any 5-8 sequences of SEQ ID NOs: 3-10, and does not comprise the sequences shown in SEQ ID NO: 12 and 13;
preferably, the amino acid sequence of said RZFD core domain comprises any 6-8 sequences of SEQ ID NOs: 3-10.

2. The expression vector according to claim 1, wherein,
the amino acid sequence of said RZFD core domain comprises the sequence shown in SEQ ID NO: 76, or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:76; or
the amino acid sequence of said RZFD core domain comprises the sequence shown in SEQ ID NO: 74 or 75, or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:74 or 75; or
the amino acid sequence of said RZFD core domain comprises the sequence shown in SEQ ID NO: 2, or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 2.

3. The expression vector according to claim 2, wherein the amino acid sequence of said RZFD core domain comprises the sequence shown in SEQ ID NO: 14, or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:14.

4. The expression vector according to claim 2, wherein the nucleic acid sequence encoding said RZFD core domain comprises the sequence shown in SEQ ID NO: 15 or 64, or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:15 or 64.

5. The expression vector according to any one of claims 1-4, wherein said regulatory element comprises a promoter regulating the expression of the nucleic acid of the artificial transcription factor; preferably, said promoter comprises a glial cell-specific promoter.

6. The expression vector according to claim 5, wherein said glial cell-specific promoter is an astrocyte-specific promoter, or a Müller cell-specific promoter, or a cochlear glial cell-specific promoter.

7. The expression vector according to claim 6, wherein said glial cell-specific promoter comprises GFAP promoter, ALDH1L1 promoter, EAAT1/GLAST promoter, glutamine synthetase promoter, S100β promoter, EAAT2/GLT-1 promoter, Plp1 promoter, or Rlbp1 promoter.

8. The expression vector according to claim 5, wherein said regulatory element further comprises an enhancer for increasing transcription of the artificial transcription factor;
preferably, said enhancer comprises CMV enhancer, SV40 intronic enhancer, MVM intronic enhancer, β-globin intronic enhancer, or synthetic intronic enhancer, or any combination thereof.

9. The expression vector according to claim 8, wherein the nucleic acid sequence of said enhancer comprises the sequence shown in any one of SEQ ID NOs: 16-22 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NOs: 16-22.

10. The expression vector according to any one of claims 5-9, wherein said regulatory element further comprises Woodchuck Hepatitis Virus Post-transcriptional Regulatory Element (WPRE).

11. The expression vector according to any one of claims 1-10, wherein the artificial transcription factor further comprises at least one nuclear localization sequence (NLS);
preferably, said NLS is selected from the NLS of SV40, the NLS of nucleoplasmin, the NLS of c-myc, the NLS of hRNPA1 M9, the NLS of importin α , the NLS of p53, the NLS of Influenza virus NS1, the NLS of hepatitis delta antigen, or the bpNLS.

12. The expression vector according to claim 11, wherein the NLS is located at the N-terminus and/or C-terminus of the RZFD core domain.

13. The expression vector according to claim 11, wherein the amino acid sequence of the NLS comprises the sequence shown in SEQ ID NO: 24 or comprises an amino acid sequence having at least 70%, at least 80%, at least 85%, at least 90%, at least 91%, at least 92%, at least 93%, at least 94%, or at least 95% identity to the sequence shown in SEQ ID NO: 24.

14. The expression vector according to claim 13, wherein the nucleic acid sequence encoding the NLS comprises the sequence shown in SEQ ID NO: 25 or comprises a sequence having at least 70%, at least 80%, at least 85%, at least 90%, at least 91%, at least 92%, at least 93%, at least 94%, or at least 95% identity to the sequence shown in SEQ ID NO:25.

15. The expression vector according to any one of claims 1-10, wherein the artificial transcription factor further comprises a gene activation domain; preferably, said gene activation domain is located at the C-terminus and/or N-terminus of said RZFD core domain.

16. The expression vector according to claim 15, wherein the gene activation domain comprises the activation domain of VP64, P65, HSF1, VP16, RTA, Suntag, P300, or CBP, or comprises any combination of these activation domains;
preferably, said gene activation domain comprises the activation domain of VP64, the activation domain of P65, the activation domain of RTA, or the activation domain of HSF1, or any combination thereof.

17. The expression vector according to claim 16, wherein the activation domain is located at the C-terminus and/or N-terminus of the RZFD core domain.

18. The expression vector according to claim 16 or 17, wherein,
the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 26;
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:69;
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 11;
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 70.

19. The expression vector according to claim 18, wherein the nucleic acid sequence encoding the VP64 activation domain comprises the sequence shown in SEQ ID NO: 27 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 27.

20. The expression vector according to claim 16, wherein said gene activation domain comprises the activation domain of VP64 and the activation domain of P65; preferably, said gene activation domain further comprises the activation domain of HSF1 or the activation domain of RTA.

21. The expression vector according to claim 20, wherein said gene activation domain is located at the N-terminus and/or C-terminus of the RZFD core domain.

22. The expression vector according to claim 20 or 21, wherein said gene activation domain comprises the activation domain of VP64, the activation domain of P65, and the activation domain of HSF1,
preferably, the amino acid sequence of said activation domain comprises the sequence shown in SEQ ID NO: 73 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 73.

23. The expression vector according to claim 20 or 21, wherein said gene activation domain comprises the activation domain of VP64, the activation domain of P65, and the activation domain of RTA,
preferably, the amino acid sequence of said activation domain comprises the sequence shown in SEQ ID NO: 72 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 72.

24. The expression vector according to claim 16, wherein the amino acid sequence of said gene activation domain comprises the sequence shown in any one of SEQ ID NO: 28, 69, 70, or 71 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NO:28, 69, 70, or 71.

25. The expression vector according to any one of claims 1-10, wherein the artificial transcription factor further comprises nuclear localization sequence (NLS) and gene activation domain; preferably, the NLS and/or gene activation domain is located at the N-terminus and/or C-terminus of the RZFD core domain; more preferably, the connection type of artificial transcription factor from the N-terminus to the C-terminus is in the following manner:
RZFD core domain - NLS - gene activation domain;
RZFD core domain - gene activation domain - NLS;
NLS - gene activation domain - RZFD core domain;
gene activation domain - NLS - RZFD core domain;
NLS - RZFD core domain - gene activation domain;
gene activation domain - RZFD core domain - NLS;
gene activation domain - NLS - RZFD core domain - NLS - gene activation domain; or
NLS - gene activation domain - RZFD core domain - gene activation domain - NLS.

26. The expression vector according to claim 25, wherein said gene activation domain comprises an activation domain of VP64, P65, HSF1, VP16, RTA, Suntag, P300, or CBP, or any combination of these activation domains;
preferably, said gene activation domain comprises the activation domain of VP64, the activation domain of P65, the activation domain of RTA, or the activation domain of HSF1, or any combination thereof.

27. The expression vector according to claim 26, wherein,
the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 26;
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 69;
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 11;
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 70.

28. The expression vector according to claim 25, wherein the amino acid sequence of said NLS comprises the sequence shown in SEQ ID NO: 24 or comprises an amino acid sequence having at least 70%, at least 80%, at least 85%, at least 90%, at least 91%, at least 92%, at least 93%, at least 94%, or at least 95% identity to the sequence shown in SEQ ID NO: 24.

29. The expression vector according to any one of claims 25-28, wherein said RZFD core domain is connected to said NLS via a linker; or
said RZFD core domain is connected to said gene activation domain via a linker; or
said NLS is connected to said gene activation domain via a linker.

30. The expression vector according to any one of claims 1-29, wherein said artificial transcription factor comprises the sequence shown in any one of SEQ ID NOs: 2, 14, 30, 32, 34, 57, 59, 61, 63, 67, 77, 78, 79, 80, 81, 82, 84, 86, 90, or 92, or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to any one of the sequence shown in SEQ ID NOs: 2, 14, 30, 32, 34, 57, 59, 61, 63, 67, 77, 78, 79, 80, 81, 82, 84, 86, 90, or 92.

31. The expression vector according to any one of claims 1-30, wherein the nucleic acid sequence encoding said artificial transcription factor comprises the sequence shown in any one of SEQ ID NOs: 15, 31, 33, 35, 56, 58, 60, 64, 68, 85, 87, 91, or 93; or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to any one of the sequence shown in SEQ ID NOs: 15, 31, 33, 35, 56, 58, 60, 64, 68, 85, 87, 91, or 93.

32. The expression vector according to claim 1, wherein said expression vector comprises the sequence shown in any one of SEQ ID NOs: 36, 37, or 62; or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to any sequence shown in SEQ ID NOs: 36, 37, or 62.

33. A composition, comprising:
(a) an artificial transcription factor capable of binding to RE1, comprising: an RZFD core domain, a first NLS, a first gene activation domain; or
(b) a nucleic acid encoding the artificial transcription factor of (a),
wherein said RZFD core domain comprises any 5-8 sequences of SEQ ID NOs: 3-10, and does not comprise the sequences shown in SEQ ID NO: 12 and 13;
preferably, the amino acid sequence of said RZFD core domain comprises any 6-8 sequences of SEQ ID NOs: 3-10.

34. The composition according to claim 33, wherein,
the amino acid sequence of said RZFD core domain comprises the sequence shown in SEQ ID NO: 76 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 76; or
the amino acid sequence of said RZFD core domain comprises the sequence shown in SEQ ID NO: 74 or 75 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 74 or 75; or
the amino acid sequence of said RZFD core domain comprises the sequence shown in SEQ ID NO: 2 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 2.

35. The composition according to claim 33, wherein the amino acid sequence of said RZFD core domain comprises the sequence shown in SEQ ID NO: 14 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 14; or
the nucleic acid sequence encoding said RZFD core domain comprises the sequence shown in SEQ ID NO: 15 or 64 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 15 or 64.

36. The composition according to claim 33, wherein the connection type from the N-terminus to the C-terminus of the RZFD core domain, the first NLS, and the first gene activation domain is selected from:
1) RZFD core domain-first NLS-first gene activation domain;
2) first gene activation domain-first NLS-RZFD core domain;
3) RZFD core domain-first gene activation domain-first NLS;
4) first NLS-first gene activation domain-RZFD core domain;
5) first NLS-RZFD core domain-first gene activation domain;
6) first gene activation domain-RZFD core domain-first NLS;

37. The composition according to claim 33, wherein said artificial transcription factor further comprises a second NLS.

38. The composition according to claim 37, wherein the first NLS is located at the N-terminus of the RZFD core domain, and the second NLS is located at the C-terminus of the RZFD core domain.

39. The composition according to any one of claims 33-38, wherein said artificial transcription factor further comprises a second gene activation domain.

40. The composition according to claim 39, wherein the second gene activation domain and the first gene activation domain are located at opposite ends of the RZFD core domain, respectively.

41. The composition according to any one of claims 33-40, wherein said first NLS and/or second NLS comprises the sequence shown in SEQ ID NO: 24 or comprises an amino acid sequence having at least 70%, at least 80%, at least 85%, at least 90%, at least 91%, at least 92%, at least 93%, at least 94%, or at least 95% identity to the sequence shown in SEQ ID NO: 24.

42. The composition according to claim 41, wherein the nucleic acid sequence encoding said first NLS and/or second NLS comprises the sequence shown in SEQ ID NO: 25 or comprises a nucleotide sequence having at least 70%, at least 80%, at least 85%, at least 90%, at least 91%, at least 92%, at least 93%, at least 94%, or at least 95% identity to the sequence shown in SEQ ID NO: 25.

43. The composition according to any one of claims 33-42, wherein the first gene activation domain comprises an activation domain of VP64, P65, HSF1, VP16, RTA, Suntag, P300, or CBP, or any combination of these activation domains;
preferably, said first gene activation domain comprises the activation domain of VP64, the activation domain of P65, the activation domain of RTA, or the activation domain of HSF1, or any combination thereof.

44. The composition according to claim 43, wherein,
the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 26;
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 69;
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 11;
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 70.

45. The composition according to claim 44, wherein the nucleic acid sequence encoding the VP64 activation domain comprises the sequence shown in SEQ ID NO: 27 or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 27.

46. The composition according to claim 43, wherein said first gene activation domain comprises the activation domain of VP64 and the activation domain of P65;
preferably, said first gene activation domain further comprises the activation domain of HSF1 or the activation domain of RTA.

47. The composition according to claim 46, wherein said first gene activation domain comprises the activation domain of VP64, the activation domain of P65, and the activation domain of HSF1,
preferably, the amino acid sequence of said first activation domain comprises the sequence shown in SEQ ID NO: 73 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 73.

48. The composition according to claim 46, wherein said first gene activation domain comprises the activation domain of VP64, the activation domain of P65, and the activation domain of RTA,
preferably, the amino acid sequence of said first activation domain comprises the sequence shown in SEQ ID NO: 72 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 72.

49. The composition according to claim 43, wherein the amino acid sequence of said first gene activation domain comprises the sequence shown in any one of SEQ ID NO: 28, 69, 70, or 71, or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NO: 28, 69, 70, or 71.

50. The composition according to claim 39, wherein the second gene activation domain comprises an activation domain of VP64, P65, HSF1, VP16, RTA, Suntag, P300, or CBP, or any combination of these activation domains;
preferably, said second gene activation domain comprises the activation domain of VP64;
more preferably, said second gene activation domain comprises the activation domain of VP64 and the activation domain of P65; more preferably, said second gene activation domain further comprises the activation domain of HSF1 or the activation domain of RTA.

51. The composition according to claim 50, wherein the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:26;
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:69;
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:11;
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:70.

52. The composition according to claim 51, wherein the nucleic acid sequence encoding the VP64 activation domain comprises the sequence shown in SEQ ID NO: 27 or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:27.

53. The composition according to claim 39, wherein said second gene activation domain comprises the activation domain of VP64 and the activation domain of P65; preferably, said second gene activation domain further comprises the activation domain of HSF1 or the activation domain of RTA.

54. The composition according to claim 53, wherein said second gene activation domain comprises the activation domain of VP64, the activation domain of P65, and the activation domain of HSF1,
preferably, the amino acid sequence of said second activation domain comprises the sequence shown in SEQ ID NO: 73 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:73.

55. The composition according to claim 53, wherein said second gene activation domain comprises the activation domain of VP64, the activation domain of P65, and the activation domain of RTA,
preferably, the amino acid sequence of said second activation domain comprises the sequence shown in SEQ ID NO: 72 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:72.

56. The composition according to claim 39, wherein the amino acid sequence of said second gene activation domain comprises the sequence shown in any one of SEQ ID NO: 28, 69, 70, or 71 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NO:28, 69, 70, or 71.

57. The composition according to any one of claims 33-56, wherein said artificial transcription factor comprises the sequence shown in any one of SEQ ID NOs: 2, 14, 30, 32, 34, 57, 59, 61, 63, 67, 77, 78, 79, 80, 81, 82, 84, 86, 90, or 92; or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to any sequence shown in SEQ ID NOs: 2, 14, 30, 32, 34, 57, 59, 61, 63, 67, 77, 78, 79, 80, 81, 82, 84, 86, 90, or 92.

58. The composition according to claim 57, wherein the nucleic acid sequence encoding said artificial transcription factor comprises the sequence shown in any one of SEQ ID NOs: 15, 31, 33, 35, 56, 58, 60, 64, 68, 85, 87, 91, or 93; or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NOs: 15, 31, 33, 35, 56, 58, 60, 64, 68, 85, 87, 91, or 93.

59. The composition according to any one of claims 33-58, wherein said composition further comprises a regulatory element for regulating the nucleic acid expression of the artificial transcription factor; preferably, said regulatory element comprises a promoter for regulating the nucleic acid expression of the artificial transcription factor; more preferably, said promoter comprises glial cell-specific promoter.

60. The composition according to claim 59, wherein said glial cell-specific promoter comprises an astrocyte-specific promoter, a Müller cell-specific promoter, or a cochlear glial cell-specific promoter;
preferably, said glial cell-specific promoter comprises GFAP promoter, ALDH1L1 promoter, EAAT1/GLAST promoter, glutamine synthetase promoter, S100β promoter, EAAT2/GLT-1 promoter, Plp1 promoter, or Rlbp1 promoter.

61. The composition according to claim 59, wherein said regulatory element further comprises an enhancer that increases transcription of said artificial transcription factor;
preferably, said enhancer comprises CMV enhancer, SV40 intronic enhancer, MVM intronic enhancer, β-globin intronic enhancer, or a synthetic intronic enhancer, or any combination thereof.

62. The composition according to claim 59, wherein said regulatory element further comprises Woodchuck Hepatitis Virus Post-transcriptional Regulatory Element (WPRE).

63. The composition according to claim 59, wherein it comprises the sequence shown in any one of SEQ ID NOs: 36, 37, or 62; or comprises a nucleic acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NOs: 36, 37, or 62.

64. A method for transdifferentiating non-neuronal cells of a subject into functional neurons, comprising contacting the expression vector according to any one of claims 1-32, or the composition according to any one of claims 33-63, with the non-neuronal cells;
preferably, said contacting is performed in vitro or in vivo.

65. The method according to claim 64, wherein said functional neurons comprise dopamine neurons, retinal ganglion cells, photoreceptor cells, cochlear spiral ganglion cells, GABA neurons, 5-HT neurons, glutamatergic neurons, ChAT neurons, NE neurons, motor neurons, spinal cord neurons, spinal motor neurons, spinal sensory neurons, bipolar cells, amacrine cells, pyramidal cells, interneurons, medium spiny neurons (MSN), Purkinje cells, granule cells, olfactory sensory neurons, periglomerular cells, or any combination thereof.

66. The method according to claim 65, wherein said dopamine neurons express one or more of the following markers: NeuN, TH, FoxA2, Nurr1, Pitx3, Vmat2, or DAT;
said retinal ganglion neurons express one or more of the following markers: RBPMS, Pax6, Brn3a, Brn3b, Brn3c, and Map2;
said photoreceptor cells express one or more of the following markers: rhodopsin, mCAR, m-opsin, S-opsin;
said cochlear spiral ganglion cells express one or more of the following markers: NeuN, Prox1, Tuj-1, and Map2.

67. The method according to any one of claims 64-66, wherein said functional neurons comprise axons.

68. The method according to any one of claims 64-66, wherein said non-neuronal cells comprise glial cells, fibroblasts, stem cells, neural precursor cells, or neural stem cells;
preferably, said glial cells comprise astrocytes, oligodendrocytes, ependymal cells, Schwann cells, NG2 cells, satellite cells, Müller cells, inner ear glial cells.

69. The method according to any one of claims 64-68, wherein the efficiency of transdifferentiation of non-neuronal cells into functional neurons is at least 1%.

70. A method for treating a disease in a subject in need, comprising administering a therapeutically effective amount of the expression vector according to any one of claims 1-32, or administering a therapeutically effective amount of the composition according to any one of claims 33-63;
preferably, said disease comprises Parkinson's disease, Alzheimer's disease, stroke, schizophrenia, Huntington's disease, depression, motor neuron disease, ALS, spinal muscular atrophy, epilepsy, ataxia, visual impairment caused by RGC cell death, glaucoma, age-related RGC injury, optic nerve injury, retinal ischemia or hemorrhage, Leber's hereditary optic neuropathy, photoreceptor cell degeneration or death due to trauma or degeneration, macular degeneration, retinitis pigmentosa, diabetes-related blindness,

71. An artificial transcription factor, comprising an RZFD core domain; wherein the amino acid sequence of said RZFD core domain comprises any 5-8 sequences of SEQ ID NOs: 3-10, and does not comprise the sequences shown in SEQ ID NO: 12 and 13; preferably, the amino acid sequence of said RZFD core domain comprises any 6-8 sequences of SEQ ID NOs: 3-10.

72. The artificial transcription factor according to claim 71, wherein,
the amino acid sequence of said RZFD core domain comprises the sequence shown in SEQ ID NO: 76 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:76;
preferably, the amino acid sequence of said RZFD core domain comprises the sequence shown in SEQ ID NO: 74 or 75 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:74 or 75;
more preferably, the amino acid sequence of said RZFD core domain comprises the sequence shown in SEQ ID NO: 2 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:2.

73. The artificial transcription factor according to claim 72, wherein the amino acid sequence of said RZFD core domain comprises the sequence shown in SEQ ID NO: 14 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:14.

74. The artificial transcription factor according to claim 72, wherein the nucleic acid sequence encoding said R ZFD core domain comprises the sequence shown in SEQ ID NO: 15 or 64; or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO: 15 or 64.

75. The artificial transcription factor according to any one of claims 71-74, wherein the artificial transcription factor further comprises at least one nuclear localization sequence (NLS);
preferably, said NLS is selected from the NLS of SV40, the NLS of nucleoplasmin, the NLS of c-myc, the NLS of hRNPA1 M9, the NLS of importin α , the NLS of p53, the NLS of Influenza virus NS1, the NLS of Hepatitis Delta Antigen, or bpNLS.

76. The artificial transcription factor according to claim 75, wherein the NLS is located at the N-terminus and/or C-terminus of the RZFD core domain;
preferably, the amino acid sequence of said NLS comprises the sequence shown in SEQ ID NO: 24 or comprises an amino acid sequence having at least 70%, at least 80%, at least 85%, at least 90%, at least 91%, at least 92%, at least 93%, at least 94%, or at least 95% identity to the sequence shown in SEQ ID NO:24.
more preferably, the nucleic acid sequence encoding said NLS comprises the sequence shown in SEQ ID NO: 25 or comprises a sequence having at least 70%, at least 80%, at least 85%, at least 90%, at least 91%, at least 92%, at least 93%, at least 94%, or at least 95% identity to the sequence shown in SEQ ID NO:25.

77. The artificial transcription factor according to any one of claims 71-76, wherein the artificial transcription factor further comprises gene activation domain;
preferably, said gene activation domain is located at the C-terminus and/or N-terminus of said RZFD core domain.

78. The artificial transcription factor according to claim 77, wherein the gene activation domain comprises the activation domain of VP64, P65, HSF1, VP16, RTA, Suntag, P300, or CBP, or any combination of these activation domains;
preferably, the gene activation domain comprises the activation domain of VP64, the activation domain of P65, the activation domain of RTA, or the activation domain of HSF1, or any combination thereof.

79. The artificial transcription factor according to claim 78, wherein the activation domain is located at the C-terminus and/or N-terminus of the RZFD core domain.

80. The artificial transcription factor according to claim 78, wherein,
the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:26;
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:69;
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:11;
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:70.

81. The artificial transcription factor according to claim 80, wherein the nucleic acid sequence encoding the VP64 activation domain comprises the sequence shown in SEQ ID NO: 27 or comprises a sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:27.

82. The artificial transcription factor according to claim 78, wherein said gene activation domain comprises the activation domain of VP64 and the activation domain of P65; preferably, said gene activation domain further comprises the activation domain of HSF1 or the activation domain of RTA.

83. The artificial transcription factor according to any one of claims 77-82, wherein said gene activation domain comprises the activation domain of VP64, the activation domain of P65, and the activation domain of HSF1,
preferably, the amino acid sequence of said activation domain comprises the sequence shown in SEQ ID NO: 73 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:73.

84. The artificial transcription factor according to any one of claims 77-82, wherein said gene activation domain comprises the activation domain of VP64, the activation domain of P65, and the activation domain of RTA,
preferably, the amino acid sequence of said activation domain comprises the sequence shown in SEQ ID NO: 72 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:72.

85. The artificial transcription factor according to claim 78, wherein the amino acid sequence of said gene activation domain comprises the sequence shown in any one of SEQ ID NOs: 28, 69, 70, or 71; or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in any one of SEQ ID NOs: 28, 69, 70, or 71.

86. The artificial transcription factor according to any one of claims 71-74, wherein the artificial transcription factor further comprises nuclear localization sequence (NLS) and gene activation domain;
preferably, the NLS and/or gene activation domain is located at the N-terminus and/or C-terminus of the RZFD core domain;
more preferably, the connection type of said artificial transcription factor from the N-terminus to the C-terminus is:
RZFD core domain - NLS - gene activation domain;
RZFD core domain - gene activation domain - NLS;
NLS - gene activation domain - RZFD core domain;
gene activation domain - NLS - RZFD core domain;
NLS - RZFD core domain - gene activation domain;
gene activation domain - RZFD core domain - NLS;
gene activation domain - NLS - RZFD core domain - NLS - gene activation domain; or
NLS - gene activation domain - RZFD core domain - gene activation domain - NLS.

87. The artificial transcription factor according to claim 86, wherein said gene activation domain comprises an activation domain of VP64, P65, HSF1, VP16, RTA, Suntag, P300, or CBP, or any combination of activation domains;
preferably, said gene activation domain comprises the activation domain of VP64, the activation domain of P65, the activation domain of RTA, or the activation domain of HSF1, or any combination thereof;
more preferably, the amino acid sequence of the VP64 activation domain comprises the sequence shown in SEQ ID NO: 26 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:26; or
the amino acid sequence of the P65 activation domain comprises the sequence shown in SEQ ID NO: 69 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:69; or
the amino acid sequence of the RTA activation domain comprises the sequence shown in SEQ ID NO: 11 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:11; or
the amino acid sequence of the HSF1 activation domain comprises the sequence shown in SEQ ID NO: 70 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to the sequence shown in SEQ ID NO:70.

88. The artificial transcription factor according to claim 86, wherein the amino acid sequence of said NLS comprises the sequence shown in SEQ ID NO: 24 or comprises an amino acid sequence having at least 70%, at least 80%, at least 85%, at least 90%, at least 91%, at least 92%, at least 93%, at least 94%, or at least 95% identity to the sequence shown in SEQ ID NO:24.

89. The artificial transcription factor according to any one of claims 86-88, wherein said RZFD core domain is connected to said NLS via a linker; or said RZFD core domain is connected to said gene activation domain via a linker; or said NLS is connected to said gene activation domain via a linker.

90. The artificial transcription factor according to any one of claims 71-89, wherein said artificial transcription factor comprises the sequence shown in any one of SEQ ID NOs: 2, 14, 30, 32, 34, 57, 59, 61, 63, 67, 77, 78, 79, 80, 81, 82, 84, 86, 90, or 92 or comprises an amino acid sequence having at least 70%, at least 80%, at least 85%, at least 90%, at least 91%, at least 92%, at least 93%, at least 94%, at least 95%, at least 96%, at least 97%, at least 98%, or at least 99% identity to any sequence shown in SEQ ID NOs: 2, 14, 30, 32, 34, 57, 59, 61, 63, 67, 77, 78, 79, 80, 81, 82, 84, 86, 90, or 92.

91. The artificial transcription factor according to any one of claims 71-90, wherein the nucleic acid sequence encoding said artificial transcription factor comprises the sequence shown in any one of SEQ ID NOs: 15, 31, 33, 35, 56, 58, 60, 64, 68, 85, 87, 91, or 93 or comprises a nucleotide sequence having at least 70%, at least 80%, at least 85%, at least 90%, at least 91%, at least 92%, at least 93%, at least 94%, at least 95%, at least 96%, at least 97%, at least 98%, or at least 99% identity to any sequence shown in SEQ ID NOs: 15, 31, 33, 35, 56, 58, 60, 64, 68, 85, 87, 91, or 93.
